# EUROPEAN PATENT SPECIFICATION

(11) **EP 3 234 117 B1**
(45) Date of publication and mention of the grant of the patent: **03.03.2021**
(21) Application number: 15816605.8
(22) Date of filing: 02.12.2015
(51) Int. Cl.: C12N 9/22, C12N 15/10, C12N 15/70

(54) **COMPOSITIONS AND METHODS FOR EFFICIENT GENE EDITING IN E. COLI USING GUIDE RNA/CAS ENDONUCLEASE SYSTEMS IN COMBINATION WITH CIRCULAR POLYNUCLEOTIDE MODIFICATION TEMPLATES**
ZUSAMMENSETZUNGEN UND VERFAHREN ZUR EFFIZIENTEN GENEDITIERUNG IN E. COLI UNTER VERWENDUNG VON RNA/CAS-ENDONUKLEASE-FÜHRUNGSSYSTEMEN IN KOMBINATION MIT KREISFÖRMIGEN SCHABLONEN ZUR POLYNUKLEOTIDMODIFIKATION
COMPOSITIONS ET PROCÉDÉS POUR L'ÉDITION DE GÈNES EFFICACE DANS E COLI AU MOYEN DE SYSTÈMES D'ARN GUIDE/ENDONUCLÉASE CAS EN COMBINAISON AVEC DES MATRICES DE MODIFICATION DE POLYNUCLÉOTIDE CIRCULAIRE

(30) Priority: 17.12.2014 US 201462092914 P
(43) Date of publication of application: 25.10.2017
(73) Proprietor: DuPont US Holding, LLC, Wilmington, DE 19805 (US)
(72) Inventor: FRISCH, Ryan, L., Newark, Delaware 19702 (US); JACKSON, Ethel, Noland, Greenville, Delaware 19807 (US)
(74) Representative: Dehns
(86) International application number: PCT/US2015/063434
(87) International publication number: WO 2016/099887

(56) References cited:
- WO-A1-2012/164565
- WO-A2-2005/056786
- WENYAN JIANG ET AL: "RNA-guided editing of bacterial genomes using CRISPR-Cas systems", NATURE BIOTECHNOLOGY, vol. 31, no. 3, 29 January 2013 (2013-01-29), pages 233-239, XP055249123, US ISSN: 1087-0156, DOI: 10.1038/nbt.2508
- M. JINEK ET AL: "A Programmable Dual-RNA-Guided DNA Endonuclease in Adaptive Bacterial Immunity", SCIENCE, vol. 337, no. 6096, 17 August 2012 (2012-08-17), pages 816-821, XP055229606, US ISSN: 0036-8075, DOI: 10.1126/science.1225829

## Description

### FIELD OF INVENTION

The invention relates to the field of bacterial molecular biology, in particular, to compositions and methods for editing a nucleotide sequence in the genome of *Escherichia coli.*

### BACKGROUND

A way to understand the function of a gene within an organism is to inhibit its expression. Inhibition of gene expression can be accomplished, for example, by interrupting or deleting the DNA sequence of the gene, resulting in "knock-out" of the gene (Austin et al., Nat. Genetics 36:921-924). Gene knock-outs mostly have been carried out through homologous recombination (HR), a technique applicable across a wide array of organisms from bacteria to mammals. Another way for studying gene function can be through genetic "knock-in", which is also usually performed by HR. HR for gene targeting has been shown to be enhanced when the targeted DNA site contains a double-strand break (Rudin et al., Genetics 122:519-534; Smih et al., Nucl. Acids Res. 23:5012-5019). Strategies for introducing double-strand breaks to facilitate HR-mediated DNA targeting have therefore been developed. For example, zinc finger nucleases have been engineered to cleave specific DNA sites leading to enhanced levels of HR at the site when a polynucleotide modification template DNA was present (Bibikova et al., Science 300:764; Bibikova et al., Mol. Cell. Biol. 21:289-297). Similarly, artificial meganucleases (homing endonucleases) and transcription activator-like effector (TALE) nucleases have also been developed for use in HR-mediated DNA targeting (Epinat et al., Nucleic Acids Res. 31: 2952-2962; Miller et al., Nat. Biotech. 29:143-148).

Loci encoding CRISPR (clustered regularly interspaced short palindromic repeats) DNA cleavage systems have been found exclusively in about 40% of bacterial genomes and most archaeal genomes (Horvath and Barrangou, Science 327:167-170; Karginov and Hannon, Mol. Cell 37:7-19). In particular, the CRISPR-associated (Cas) RNA-guided endonuclease (RGEN), Cas9, of the type II CRIPSR system has been developed as a means for introducing site-specific DNA strand breaks that stimulate HR (U.S. Provisional Appl. No. 61/868,706, filed August 22, 2013). The sequence of the RNA component of Cas9 can be designed such that Cas9 recognizes and cleaves DNA containing (i) sequence complementary to a portion of the RNA component and (ii) a protospacer adjacent motif (PAM) sequence.

Native RNA/Cas9 complexes comprise two RNA sequences, a CRISPR RNA (crRNA) and a trans-activating CRISPR RNA (tracrRNA). A crRNA contains, in the 5'-to-3' direction, a unique sequence complementary to a target DNA site and a portion of a sequence encoded by a repeat region of the CRISPR locus from which the crRNA was derived. A tracrRNA contains, in the 5'-to-3' direction, a sequence that anneals with the repeat region of crRNA and a stem loop-containing portion. Recent work has led to the development of guide RNAs (gRNA), which are chimeric sequences containing, in the 5'-to-3' direction, a crRNA linked to a tracrRNA (U.S. Patent Appl. No. 14/463,687, filed August 20, 2014).

Recombinant DNA technology has made it possible to modify DNA sequences in the genome of an organism, thus, altering the organism's phenotype. Although several approaches have been developed to target a specific site for modification in the genome of an organism such as *E*. *coli,* there still remains a need for more efficient and effective methods for editing a nucleotide sequence in the genome of an *Escherichia coli* cell.

### SUMMARY

The present disclosure includes compositions and methods for genome modification of a target sequence in the genome of an *Escherichia coli cell.* The methods and compositions employ a guide RNA/Cas endonuclease system (also referred to as an RGEN) in combination with a circular polynucleotide modification template to provide an effective system for editing target sites within the genome of an *Escherichia coli cell.* The methods and compositions also employ a guide RNA/Cas endonuclease system in combination with a circular donor DNA to provide an effective system for gene knock-in an *Escherichia coli cell.*

In one embodiment of the disclosure, the method comprises a method for editing a target sequence in the genome of an *Escherichia coli* cell, the method comprising providing at least one recombinant DNA construct comprising a DNA sequence encoding a guide RNA and a circular polynucleotide modification template to an *E.coli* cell comprising a Cas9 endonuclease DNA sequence operably linked to an inducible promoter, wherein said Cas9 endonuclease DNA sequence encodes a Cas9 endonuclease, wherein the guide RNA and the Cas9 endonuclease can form a guide RNA/Cas9 endonuclease complex (RGEN) that is capable of mediating cleavage of the target sequence to allow for homologous recombination mediated gene editing at a target site in the genome of said *E*. *coli* cell, wherein said polynucleotide modification template comprises at least one nucleotide modification of said target sequence. The target sequence in the genome of an *E*. *coli* cell can be selected from the group consisting of a promoter sequence, a terminator sequence, a regulatory element sequence, a coding sequence, a prophage, a pseudogene, an exogenous gene, an endogenous gene. The recombinant DNA construct comprising a DNA sequence encoding a guide RNA can be provided via a circular plasmid. The recombinant DNA construct and the circular polynucleotide modification template can each be provided on separate plasmids. The recombinant DNA construct and the circular polynucleotide modification template can be provided on a single plasmid. The recombinant DNA construct and the circular polynucleotide template can be provided via one means selected from the group consisting of electroporation, heat-shock, phage delivery, mating, conjugation and transduction. The target site in the genome of the *E*. *coli* cell, can be flanked by a first genomic region and a second genomic region, wherein the circular polynucleotide template further comprises a first region of homology to said first genomic region and a second region of homology to said second genomic region.

In one embodiment, the *E*. *coli* cell does not express an exogenous recombinase protein, a RecET protein, a lambda-red protein, or a RecBCD inhibitor.

In one embodiment of the disclosure, the method comprises a method for producing a galK mutant E.coli cell, the method comprising: a) providing at least one circular recombinant DNA construct comprising a DNA sequence encoding a guide RNA and at least one circular polynucleotide modification template to an *E*. *coli* cell comprising a Cas9 endonuclease DNA sequence operably linked to an inducible promoter, wherein said Cas9 endonuclease DNA sequence encodes a Cas endonuclease that is capable of introducing a double-strand break at a target site within a *galK* genomic sequence in the *E*. *coli* genome, wherein said circular polynucleotide modification template comprises at least one nucleotide modification of said *galK* genomic sequence; b) growing progeny cells from the *E*. *coli* cell of (a); c) evaluating the progeny cells of (b) for the presence of said at least one nucleotide modification.

In one embodiment of the disclosure, the method comprises a method for editing a target sequence in the genome of an *Escherichia coli* cell, the method comprising providing at least a first recombinant DNA construct comprising a DNA sequence encoding a guide RNA, a circular polynucleotide modification template, and a second recombinant DNA construct comprising a DNA sequence encoding Cas9 endonuclease operably linked to an inducible promoter, to an *E.coli* cell, wherein the Cas9 endonuclease introduces a double-strand break at a target site in the genome of said *E*. *coli* cell, wherein said polynucleotide modification template comprises at least one nucleotide modification of said target sequence. The first recombinant DNA construct, the second recombinant DNA construct, and the circular polynucleotide modification template can each be provided on separate plasmids. The first recombinant DNA construct, the second recombinant DNA construct, and the circular polynucleotide modification template can be provided on a single plasmid

### BRIEF DESCRIPTION OF THE DRAWINGS AND SEQUENCES

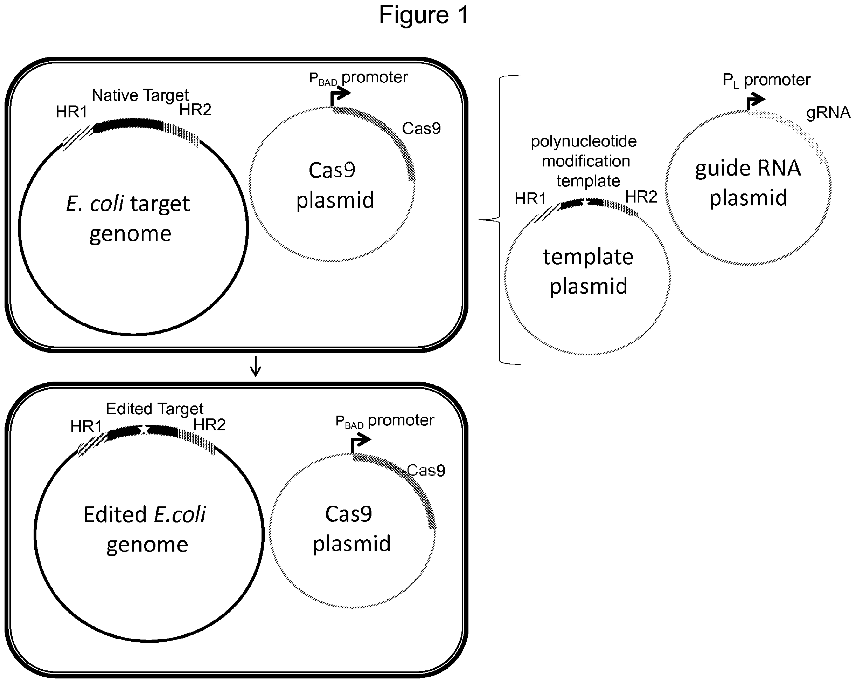

Figure 1. Use of a circular plasmid (template plasmid) comprising a polynucleotide modification template for gene editing of a native target in *E*. *coli* cells comprising a Cas9 plasmid. The schematic illustrates an *E*. *coli* cell containing a native target to be edited (located in the *E*. *coli* target genome) and a Cas9 plasmid comprising a Cas9 expression cassette driven by an inducible promoter (for example, Pbad). The polynucleotide modification template containing the desired edit (shown by a white star) to the native target sequence (shown as a black bar) flanked by two homologous regions (HR1 and HR2, allowing homologous recombination) is provided to *E*. *coli* cells (of which the Cas9 endonuclease expression was induced) via the template plasmid, together with a guide RNA plasmid comprising the guide RNA expression cassette capable of expressing a guide RNA (gRNA). The induced *E*. *coli* cell are capable of expressing the Cas9 endonuclease and form a guide RNA/Cas9 endonuclease complex (also referred to as RGEN) that is capable of mediating cleavage of the native target sequence allowing for homologous recombination mediated gene editing.

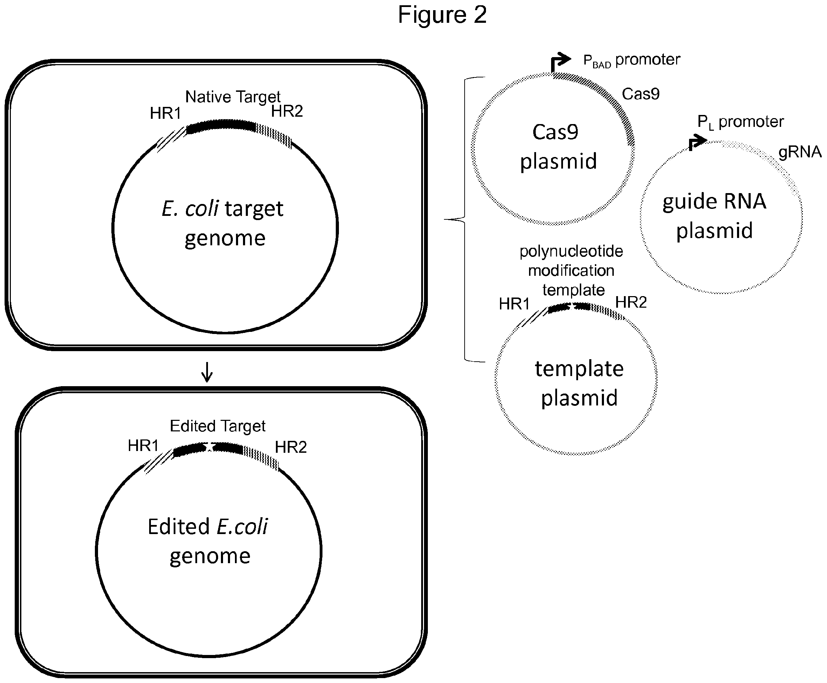

Figure 2. Use of a circular plasmid (template plasmid) comprising a polynucleotide modification template for gene editing of a native target in *E*. *coli* cells lacking a Cas9 plasmid. The schematic illustrates an *E. coli* cell containing a native target sequence to be edited (located in the E. *coli* target genome) promoter. The polynucleotide modification template containing the desired edit (shown by a white star) to the native target sequence (shown as a black bar) flanked by two homologous regions (HR1 and HR2, allowing homologous recombination) is provided to *E*. *coli* cells via the template plasmid, together with a guide RNA plasmid (comprising the guide RNA expression cassette) and a Cas9 plasmid (comprising an inducible Cas9 expression cassette driven by a Pbad). Once the *E. coli* cell are induced , the induced cells are capable of expressing the Cas9 endonuclease and form a guide RNA/Cas9 endonuclease complex (also referred to as RGEN) that is capable of mediating cleavage of the native target sequence allowing for homologous recombination mediated gene editing.

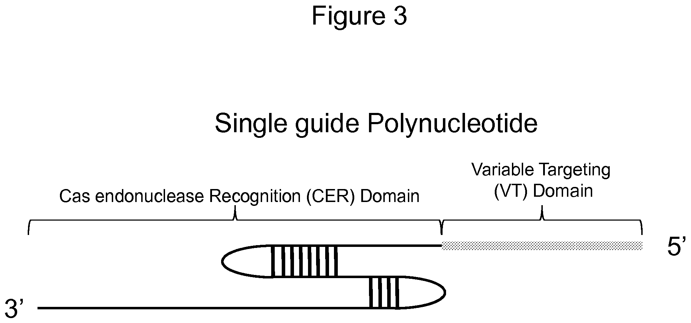

Figure 3 shows a single guide polynucleotide containing a Cas endonuclease recognition domain (CER) domain (black) linked to the variable targeting domain (VT) (grey).

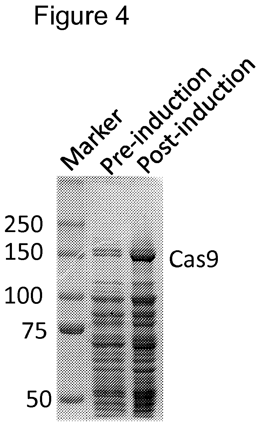

Figure 4 shows an SDS-PAGE gel of Cas9 expression from pRF48 in E. *coli* cells pre and post induction with arabinose. Marker weights are indicated in kilodaltons (kDa). The band corresponding to Cas9 in the gel is indicated (Cas9).

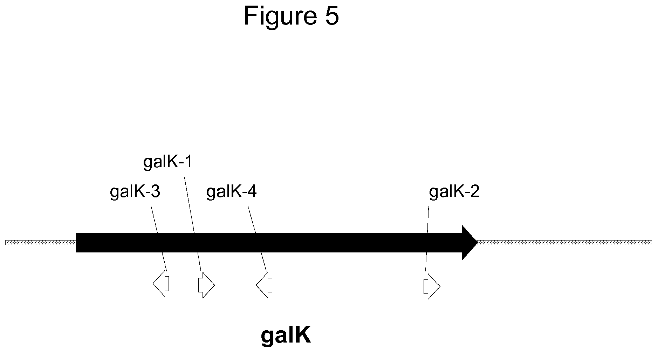

Figure 5 illustrates the *galK* gene of *E. coli* (black). The four native target sites in *galK* are indicated by arrows labelled with the target site name and the direction of the arrow indicating forward or reverse strand of the target DNA.

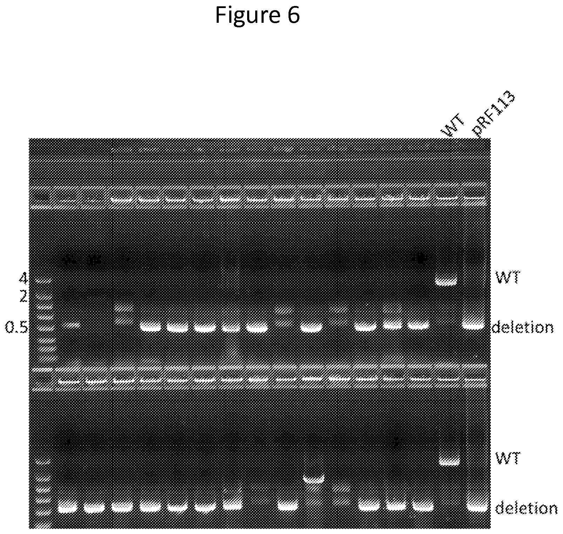

Figure 6 shows an agarose gel of DNA from a colony PCR of the *galK* locus of galactose resistant *E. coli* after gene editing with the guide RNA/Cas9 endonuclease complex (RGEN). Each lane corresponds to an individual galactose resistant colony. Marker weights are given in kilobases (kb). The size of the desired edit (deletion) is indicated next to the band. The size of the unedited allele is also indicated (WT). Two control reactions (WT and pRF113) are run on the gel to indicate the WT and edited allele respectively.

**Table 1**

| Summary ofNucleic Acid and Protein SEQ ID Numbers | | |
|---|---|---|
| Description | Nucleic acid SEQ ID NO. | Protein SEQ ID NO. |
| *Streptococcus pyogenes* Cas9 open reading frame | 1 (4107 bases) | |
| *Streptococcus pyogenes* Cas9 including C-terminal linker and SV40 NLS ("Cas9-NLS"); open reading frame codon-optimized for expression in *Y. lipolytica.* | 2 (4140 bases) | |
| *Simian virus40 NLS* | | 3 (9 aa) |
| *Yarrowia lipolytica* FBA1 promoter. | 4 (546 bases) | |
| Yarrowia optimized Cas9 expression cassette | 5 (4683 bases) | |
| pZUFCas9 plasmid. | 6 (10706 bases) | |
| Cas9-SV40 fusion | 7 (4144 bases) | |
| Cas9-NLS forward PCR primer. | 8 (35 bases) | |
| Cas9-NLS reverse PCR primer. | 9 (31 bases) | |
| EcoRI-Cas9-NLS-HinDIII PCR product | 10 (4166 bases) | |
| pBAD/HisB plasmid | 11 (4092 bases) | |
| pRF48 plasmid | 12 (8237 bases) | |
| GalK-1 target site | 13 (23 bases) | |
| GalK-2 target site | 14 (23 bases) | |
| GalK-3 target site | 15 (23 bases) | |
| GalK-4 target site | 16 (23 bases) | |
| Cas9 recognition domain (CER)25 | 17 (80 bases) | |
| GalK-1 sgRNA template DNA | 18 (100 bases) | |
| GalK-2 sgRNA template DNA | 19 (100 bases) | |
| GalK-3 sgRNA template DNA | 20 (100 bases) | |
| GalK-4sgRNA template DNA | 21 (100 bases) | |
| GalK-1 sgRNA | 22 (100 bases) | |
| GalK-2 sgRNA | 23 (100 bases) | |
| GalK-3 sgRNA | 24 (100 bases) | |
| GalK-4sgRNA | 25 (100 bases) | |
| Lambda PL promoter | 26 (52 bases) | |
| Lambda Terminator | 27 (43 bases) | |
| GalK-1 sgRNA expression cassette | 28 (212 bases) | |
| GalK-1 sgRNA expression cassette | 29 (212 bases) | |
| GalK-1 sgRNA expression cassette | 30 (212 bases) | |
| GalK-1 sgRNA expression cassette | 31 (212 bases) | |
| pACYC184 | 32 (4245 bases) | |
| pRF50 | 33 (4099 bases) | |
| pRF51 | 34 (4099 bases) | |
| pRF53 | 35 (4099 bases) | |
| pRF55 | 36 (4099 bases) | |
| 454bp 5' galK | 37 (454 bases) | |
| 5' forward primer | 38 (29 bases) | |
| 5' reverse primer | 39 (40 bases) | |
| upstream overlap extension product | 40 (483 bases) | |
| 376bp 3' galK | 41 (376 bases) | |
| 3' forward primer | 42 (40 bases) | |
| 3' reverse primer | 43 (30 bases) | |
| downstream overlap extension product | 44 (405 bases) | |
| galK deletion polynucleotide modification template | 45 (848 bases) | |
| pKD3 | 46 (2804 bases) | |
| pRF113; | 47 (2458 bases) | |
| galK locus | 48 (1717 bases) | |
| GalK forward primer | 49 (21 bases) | |
| GalK Reverse primer | 50 (21 bases) | |
| galK deletion locus (amplified from the edited strains) | 51 (1136 bases) | |
| Example of a Cas9 target site:PAM sequence | 52 (23 bases) | |

### DETAILED DESCRIPTION

As used herein, the term "disclosure" or "disclosed disclosure" is not meant to be limiting, but applies generally to any of the disclosures defined in the claims or described herein. These terms are used interchangeably herein.

Compositions and methods are provided for genome modification of a target sequence in the genome of an *Escherichia coli cell.* The methods and compositions employ a guide RNA/Cas endonuclease system in combination with a circular polynucleotide modification template to provide an effective system for editing target sites within the genome of an *Escherichia coli cell.*

The use of a circular plasmid (template plasmid) comprising a polynucleotide modification template for gene editing of a native target in *E. coli* cells comprising a Cas9 plasmid is illustrated in Figure 1 and described herein. The schematic illustrates an *E. coli* cell containing a native target to be edited (located in the *E. coli* target genome) and a Cas9 plasmid comprising a Cas9 expression cassette driven by an inducible promoter (for example, Pbad). The polynucleotide modification template containing the desired edit (shown by a white star) to the native target sequence (shown as a black bar) flanked by two homologous regions (HR1 and HR2, allowing homologous recombination) is provided to *E*. *coli* cells (of which the Cas9 endonuclease expression was induced) via the template plasmid, together with a guide RNA plasmid comprising the guide RNA expression cassette capable of expressing a guide RNA (gRNA). The induced *E. coli* cell are capable of expressing the Cas9 endonuclease and form a guide RNA/Cas9 endonuclease complex (also referred to as RGEN) that is capable of mediating cleavage of the native target sequence allowing for homologous recombination mediated gene editing.

The use of a circular plasmid (template plasmid) comprising a polynucleotide modification template for gene editing of a native target in *E. coli* cells lacking a Cas9 plasmid is illustrated in Figure 2 and described herein. The schematic illustrates an *E. coli* cell containing a native target sequence to be edited (located in the *E. coli* target genome). The polynucleotide modification template containing the desired edit (shown by a white star) to the native target sequence (shown as a black bar) flanked by two homologous regions (HR1 and HR2, allowing homologous recombination) is provided to *E. coli* cells via the template plasmid, together with a guide RNA plasmid (comprising the guide RNA expression cassette) and a Cas9 plasmid (comprising an inducible Cas9 expression cassette driven by a Pbad). Once the *E. coli* cell are induced , the induced cells are capable of expressing the Cas9 endonuclease and form a guide RNA/Cas9 endonuclease complex (also referred to as RGEN) that is capable of mediating cleavage of the native target sequence allowing for homologous recombination mediated gene editing.

A circular plasmid comprising a donor DNA comprising a polynucleotide of interest can also be used for a for gene knock-in *E. coli* as described herein.

The term "CRISPR" (clustered regularly interspaced short palindromic repeats) refers to certain genetic loci encoding factors of class I, II, or III DNA cleavage systems, for example, used by bacterial and archaeal cells to destroy foreign DNA (Horvath and Barrangou, Science 327:167-170). Components of CRISPR systems are taken advantage of herein in a heterologous manner for DNA targeting in cells.

The terms "type II CRISPR system" and "type II CRISPR-Cas system" are used interchangeably herein and refer to a DNA cleavage system utilizing a Cas9 endonuclease in complex with at least one RNA component. For example, a Cas9 can be in complex with a CRISPR RNA (crRNA) and a trans-activating CRISPR RNA (tracrRNA). In another example, a Cas9 can be in complex with a guide RNA. Thus, crRNA, tracrRNA, and guide RNA are non-limiting examples of RNA components herein.

The term CRISPR-associated ("Cas") endonuclease herein refers to a Cas protein encoded by a Cas gene. A Cas endonuclease, when in complex with a suitable RNA component, is capable of cleaving all or part of a specific DNA target sequence. For example, it is capable of introducing a double-strand break in a specific DNA target sequence; it can alternatively be characterized as being able to cleave one or both strands of a specific DNA target sequence. A Cas endonuclease can unwind the DNA duplex at the target sequence and cleaves at least one DNA strand, as mediated by recognition of the target sequence by a crRNA or guide RNA that is in complex with the Cas. Such recognition and cutting of a target sequence by a Cas endonuclease typically occurs if the correct protospacer-adjacent motif (PAM) is located at or adjacent to the 3' end of the DNA target sequence. Alternatively, a Cas protein herein may lack DNA cleavage or nicking activity, but can still specifically bind to a DNA target sequence when complexed with a suitable RNA component. A preferred Cas protein herein is Cas9.

"Cas9" (formerly referred to as Cas5, Csn1, or Csx12) herein refers to a Cas endonuclease of a type II CRISPR system that forms a complex with crRNA and tracrRNA, or with a guide RNA, for specifically recognizing and cleaving all or part of a DNA target sequence. Cas9 protein comprises an RuvC nuclease domain and an HNH (H-N-H) nuclease domain, each of which cleaves a single DNA strand at a target sequence (the concerted action of both domains leads to DNA double-strand cleavage, whereas activity of one domain leads to a nick). In general, the RuvC domain comprises subdomains I, II and III, where domain I is located near the N-terminus of Cas9 and subdomains II and III are located in the middle of the protein, flanking the HNH domain (Hsu et al, Cell 157:1262-1278). "Apo-Cas9" refers to Cas9 that is not complexed with an RNA component. Apo-Cas9 can bind DNA, but does so in a non-specific manner, and cannot cleave DNA (Sternberg et al., Nature 507:62-67).

The term "CRISPR RNA" (crRNA) herein refers to an RNA sequence that can form a complex with one or more Cas proteins (e.g., Cas9) and provides DNA binding specificity to the complex. A crRNA provides DNA binding specificity since it contains a "variable targeting domain" (VT) that is complementary to a strand of a DNA target sequence. A crRNA further comprises a "repeat sequence" ("tracr RNA mate sequence") encoded by a repeat region of the CRISPR locus from which the crRNA was derived. A repeat sequence of a crRNA can anneal to sequence at the 5'-end of a tracrRNA. crRNA in native CRISPR systems is derived from a "pre-crRNA" transcribed from a CRISPR locus. A pre-crRNA comprises spacer regions and repeat regions; spacer regions contain unique sequence complementary to a DNA target site sequence. Pre-crRNA in native systems is processed to multiple different crRNAs, each with a guide sequence along with a portion of repeat sequence. CRISPR systems utilize crRNA, for example, for DNA targeting specificity.

The term "trans-activating CRISPR RNA" (tracrRNA) herein refers to a non-coding RNA used in type II CRISPR systems, and contains, in the 5'-to-3' direction, (i) a sequence that anneals with the repeat region of CRISPR type II crRNA and (ii) a stem loop-containing portion (Deltcheva et al., Nature 471:602-607).

A "CRISPR DNA" (crDNA) can optionally be used instead of an RNA component. A crDNA has a DNA sequence corresponding to the sequence of a crRNA as disclosed herein. A crDNA can be used with a tracrRNA in a crDNA/tracrRNA complex, which in turn can be associated with an RGEN protein component. U.S. Appl. No. 61/953,090 discloses crDNA and the methods of its use in RGEN-mediated DNA targeting. It is contemplated that any disclosure herein regarding a crRNA can similarly apply to using a crDNA, accordingly. Thus, in embodiments herein incorporating a crDNA, an "RNA-guided endonuclease" (RGEN) could instead be referred to as a complex comprising at least one Cas protein and at least one crDNA.

As used herein, the term "guide polynucleotide", relates to a polynucleotide sequence that can form a complex with a Cas endonuclease and enables the Cas endonuclease to recognize and optionally cleave a DNA target site. The guide polynucleotide can be a single molecule or a double molecule. The guide polynucleotide sequence can be a RNA sequence, a DNA sequence, or a combination thereof (an RNA-DNA combination sequence). Optionally, the guide polynucleotide can comprise at least one nucleotide, phosphodiester bond or linkage modification such as, but not limited, to Locked Nucleic Acid (LNA), 5-methyl dC, 2,6-Diaminopurine, 2'-Fluoro A, 2'-Fluoro U, 2'-O-Methyl RNA, Phosphorothioate bond, linkage to a cholesterol molecule, linkage to a polyethylene glycol molecule, linkage to a spacer 18 (hexaethylene glycol chain) molecule, or 5' to 3' covalent linkage resulting in circularization.

A guide polynucleotide that solely comprises ribonucleic acids is also referred to as a "guide RNA". The guide RNA can form a complex with a Cas endonuclease referred to a guide RNA/Cas endonuclease complex (also referred to as an RGEN) The terms "guide RNA" (gRNA) and "single guide RNA" (sgRNA) are used interchangeably herein. A gRNA herein can refer to a chimeric sequence containing a crRNA operably linked to a tracrRNA. Alternatively, a gRNA can refer to a synthetic fusion of a crRNA and a tracrRNA, for example. A gRNA can also be characterized in terms of having a variable targeting domain followed by Cas endonuclease recognition (CER) domain. A CER domain can comprise a tracrRNA mate sequence followed by a tracrRNA sequence.

The guide polynucleotide can be a double molecule (also referred to as duplex guide polynucleotide) comprising a first nucleotide sequence domain (referred to as Variable Targeting domain or VT domain) that is complementary to a nucleotide sequence in a target DNA and a second nucleotide sequence domain (referred to as Cas endonuclease recognition domain or CER domain) that interacts with a Cas endonuclease polypeptide. The CER domain of the double molecule guide polynucleotide comprises two separate molecules that are hybridized along a region of complementarity. The two separate molecules can be RNA, DNA, and/or RNA-DNA- combination sequences. In some embodiments, the first molecule of the duplex guide polynucleotide comprising a VT domain linked to a CER domain ( "crNucleotide") is referred to as "crDNA" (when composed of a contiguous stretch of DNA nucleotides) or "crRNA" (when composed of a contiguous stretch of RNA nucleotides), or "crDNA-RNA" (when composed of a combination of DNA and RNA nucleotides). In some embodiments the second molecule of the duplex guide polynucleotide comprising a CER domain is referred to as "tracrRNA" (when composed of a contiguous stretch of RNA nucleotides) or "tracrDNA" (when composed of a contiguous stretch of DNA nucleotides) or "tracrDNA-RNA" (when composed of a combination of DNA and RNA nucleotides).

The guide polynucleotide can also be a single molecule comprising a first nucleotide sequence domain (referred to as Variable Targeting domain or VT domain, Figure 3) that is complementary to a nucleotide sequence in a target DNA and a second nucleotide domain (referred to as Cas endonuclease recognition domain or CER domain, Figure 3) that interacts with a Cas endonuclease polypeptide. By "domain" it is meant a contiguous stretch of nucleotides that can be RNA, DNA, and/or RNA-DNA-combination sequence. The VT domain and/or the CER domain of a single guide polynucleotide can comprise an RNA sequence, a DNA sequence, or a, RNA-DNA-combination sequence. In some embodiments the single guide polynucleotide comprises a crNucleotide (comprising a VT domain linked to a CER domain) linked to a tracrNucleotide (comprising a CER domain), wherein the linkage is a nucleotide sequence comprising an RNA sequence, a DNA sequence, or an RNA-DNA combination sequence. The single guide polynucleotide being comprised of sequences from the crNucleotide and tracrNucleotide may be referred to as "single guide RNA" (when composed of a contiguous stretch of RNA nucleotides) or "single guide DNA" (when composed of a contiguous stretch of DNA nucleotides) or "single guide RNA-DNA" (when composed of a combination of RNA and DNA nucleotides).

Thus, a guide polynucleotide and a type II Cas endonuclease in certain embodiments can form a complex with each other (referred to as a "guide polynucleotide/Cas endonuclease complex" or also referred to as "guide polynucleotide/Cas endonuclease system"), wherein the guide polynucleotide/Cas endonuclease complex can direct the Cas endonuclease to target a genomic target site in a cell (e.g., plant cell), optionally enabling the Cas endonuclease to introduce a single- or double-strand break into the genomic target site. A guide polynucleotide/Cas endonuclease complex can be linked to at least one CPP, wherein such complex is capable of binding to, and optionally creating a single- or double-strand break to, a target site of a cell (e.g., a plant cell).

The term "variable targeting domain" or "VT domain" is used interchangeably herein and refers to a nucleotide sequence that is complementary to one strand (nucleotide sequence) of a double strand DNA target site. The percent complementation between the first nucleotide sequence domain (VT domain) and the target sequence can be at least 50%, 51%, 52%, 53%, 54%, 55%, 56%, 57%, 58%, 59%, 60%, 61%, 62%, 63%, 63%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The variable target domain can be at least 12, 13, 14, 15, 16, 17, 18, 19, 20, 21, 22, 23, 24, 25, 26, 27, 28, 29 or 30 nucleotides in length. In some embodiments, the variable targeting domain comprises a contiguous stretch of 12 to 30 nucleotides. The variable targeting domain can be composed of a DNA sequence, an RNA sequence, a modified DNA sequence, a modified RNA sequence (see, e.g., modifications described herein), or any combination thereof.

The term "Cas endonuclease recognition domain" or "CER domain" of a guide polynucleotide is used interchangeably herein and relates to a nucleotide sequence (such as a second nucleotide sequence domain of a guide polynucleotide), that interacts with a Cas endonuclease polypeptide. A CER domain can be composed of a DNA sequence, an RNA sequence, a modified DNA sequence, a modified RNA sequence (see, e.g., modifications described herein), or any combination thereof.

The term "RNA-guided endonuclease", "RGEN", "guide RNA/Cas endonuclease complex", "guide RNA/Cas endonuclease system" can be used interchangeably herein and refers to a complex comprising at least one CRISPR (clustered regularly interspaced short palindromic repeats)-associated (Cas) protein and at least one RNA component. The terms "protein component of an RGEN" and "RGEN protein component" are used interchangeably herein and refer to a Cas protein, which is, or forms part of, the endonuclease component of an RGEN. A protein component in certain embodiments can be a complete endonuclease (e.g., Cas9); such a protein component can alternatively be referred to as "the endonuclease component" of an RGEN. An RGEN herein typically has specific DNA targeting activity, given its association with at least one RNA component.

The term "RNA component" herein refers to an RNA component of an RGEN containing a ribonucleic acid sequence that is complementary to a strand of a DNA target sequence. This complementary sequence is referred to herein as a "guide sequence" or "variable targeting domain" sequence (Figure 3). Examples of suitable RNA components herein include crRNA and guide RNA. RNA components in certain embodiments (e.g., guide RNA alone, crRNA + tracrRNA) can render an RGEN competent for specific DNA targeting.

Briefly, an RNA component of an RGEN contains sequence that is complementary to a DNA sequence in a target site sequence. Based on this complementarity, an RGEN can specifically recognize and cleave a particular DNA target site sequence. An RGEN herein can comprise Cas protein(s) and suitable RNA component(s) of any of the four known CRISPR systems (Horvath and Barrangou, Science 327:167-170) such as a type I, II, or III CRISPR system. An RGEN in preferred embodiments comprises a Cas9 endonuclease (CRISPR II system) and at least one RNA component (e.g., a crRNA and tracrRNA, or a gRNA).

An RGEN protein component can refer to a Cas protein such as Cas9. Examples of suitable Cas proteins include one or more Cas endonucleases of type I, II, or III CRISPR systems (Bhaya et al., Annu. Rev. Genet. 45:273-297). A type I CRISPR Cas protein can be a Cas3 or Cas4 protein, for example. A type II CRISPR Cas protein can be a Cas9 protein, for example. A type III CRISPR Cas protein can be a Cas10 protein, for example. A Cas9 protein is used in certain preferred embodiments. A Cas protein in certain embodiments may be a bacterial or archaeal protein. Type I-III CRISPR Cas proteins herein are typically prokaryotic in origin; type I and III Cas proteins can be derived from bacterial or archaeal species, whereas type II Cas proteins (i.e., a Cas9) can be derived from bacterial species, for example. In other embodiments, suitable Cas proteins include one or more of Cas1, Cas1B, Cas2, Cas3, Cas4, Cas5, Cas6, Cas7, Cas8, Cas9, Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof.

In other aspects of the disclosed disclosure, a Cas protein herein can be from any of the following genera: *Aeropyrum, Pyrobaculum, Sulfolobus, Archaeoglobus, Haloarcula, Methanobacteriumn, Methanococcus, Methanosarcina, Methanopyrus, Pyrococcus, Picrophilus, Thernioplasnia, Corynebacterium, Mycobacterium, Streptomyces, Aquifrx, Porphvromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myrococcus, Campylobacter, Wolinella, Acinetobacter, Erwinia, Escherichia, Legionella, Methylococcus, Pasteurella, Photobacterium, Salmonella, Xanthomonas, Yersinia, Streptococcus, Treponema, Francisella,* or *Thermotoga.* Alternatively, a Cas protein herein can be encoded, for example, by any of SEQ ID NOs:462-465, 467-472, 474-477, 479-487, 489-492, 494-497, 499-503, 505-508, 510-516, or 517-521 as disclosed in U.S. Appl. Publ. No. 2010/0093617.

An RGEN protein component can comprise a Cas9 amino acid sequence, for example. An RGEN comprising this type of protein component typically can be characterized as having Cas9 as the endonuclease component of the RGEN. The amino acid sequence of a Cas9 protein herein, as well as certain other Cas proteins herein, may be derived from a *Streptococcus* (e.g., S. *pyogenes, S. pneumoniae, S. thermophilus, S. agalactiae, S. parasanguinis, S. oralis, S. salivarius,* S. *macacae, S. dysgalactiae, S. anginosus, S. constellatus, S. pseudoporcinus, S. mutans), Listeria* (e.g., *L. innocua), Spiroplasma* (e.g., S. *apis, S. syrphidicola), Peptostreptococcaceae, Atopobium, Porphyromonas* (e.g., *P. catoniae), Prevotella* (e.g., *P. intermedia), Veillonella, Treponema* (e.g., *T. socranskii, T. denticola), Capnocytophaga, Finegoldia* (e.g., *F. magna), Coriobacteriaceae* (e.g., C. *bacterium), Olsenella* (e.g., O. *profusa), Haemophilus* (e.g., *H. sputorum, H. pittmaniae), Pasteurella* (e.g., *P. bettyae), Olivibacter* (e.g., *O. sitiensis), Epilithonimonas* (e.g., *E. tenax), Mesonia* (e.g., *M. mobilis), Lactobacillus, Bacillus* (e.g., *B*. *cereus), Aquimarina* (e.g., *A. muelleri), Chryseobacterium* (e.g., C. *palustre), Bacteroides* (e.g., *B*. *graminisolvens), Neisseria* (e.g., *N. meningitidis), Francisella* (e.g., *F. novicida),* or *Flavobacterium* (e.g., *F. frigidarium, F. soli)* species, for example. An S. *pyogenes* Cas9 is preferred in certain aspects herein. As another example, a Cas9 protein can be any of the Cas9 proteins disclosed in Chylinski et al. (RNA Biology 10:726-737).

Accordingly, the sequence of a Cas9 protein herein can comprise, for example, any of the Cas9 amino acid sequences disclosed in GenBank Accession Nos. G3ECR1 (S. *thermophilus),* WP_026709422, WP_027202655, WP_027318179, WP_027347504, WP_027376815, WP_027414302, WP_027821588, WP_027886314, WP_027963583, WP_028123848, WP_028298935, Q03JI6 (S. *thermophilus),* EGP66723, EGS38969, EGV05092, EHI65578 (S. *pseudoporcinus),* EIC75614 (S. *oralis),* EID22027 (S. *constellatus),* EIJ69711, EJP22331 (S. *oralis),* EJP26004 (S. *anginosus),* EJP30321, EPZ44001 *(S. pyogenes),* EPZ46028 (S. *pyogenes),* EQL78043 (S. *pyogenes),* EQL78548 (S. *pyogenes),* ERL10511, ERL12345, ERL19088 (S. *pyogenes),* ESA57807 (S. *pyogenes),* ESA59254 (S. *pyogenes),* ESU85303 (S. *pyogenes),* ETS96804, UC75522, EGR87316 (S. *dysgalactiae),* EGS33732, EGV01468 (S. *oralis),* EHJ52063 (S. *macacae),* EID26207 (S. *oralis),* EID33364, EIG27013 (S. *parasanguinis),* EJF37476, EJO19166 *(Streptococcus* sp. BS35b), EJU16049, EJU32481, YP_006298249, ERF61304, ERK04546, ETJ95568 (S. *agalactiae),* TS89875, ETS90967 *(Streptococcus* sp. SR4), ETS92439, EUB27844 *(Streptococcus* sp. BS21), AFJ08616, EUC82735 *(Streptococcus* sp. CM6), EWC92088, EWC94390, EJP25691, YP_008027038, YP_008868573, AGM26527, AHK22391, AHB36273, Q927P4, G3ECR1, or Q99ZW2 (S. *pyogenes).* A variant of any of these Cas9 protein sequences may be used, but should have specific binding activity, and optionally cleavage or nicking activity, toward DNA when associated with an RNA component herein. Such a variant may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to the amino acid sequence of the reference Cas9.

Alternatively, a Cas9 protein herein can be encoded by the SEQ ID NOs: 1-2, for example. Alternatively still, a Cas9 protein may comprise an amino acid sequence that is at least about 80%, 81%, 82%, 83%, 84%, 85%, 86%, 87%, 88%, 89%, 90%, 91%, 92%, 93%, 94%, 95%, 96%, 97%, 98%, or 99% identical to any of the foregoing amino acid sequences, for example. Such a variant Cas9 protein should have specific binding activity, and optionally cleavage or nicking activity, toward DNA when associated with an RNA component herein.

The origin of a Cas protein used herein (e.g., Cas9) may be from the same species from which the RNA component(s) is derived, or it can be from a different species. For example, an RGEN comprising a Cas9 protein derived from a *Streptococcus* species (e.g., S. *pyogenes* or S. *thermophilus)* may be complexed with at least one RNA component having a sequence (e.g., crRNA repeat sequence, tracrRNA sequence) derived from the same *Streptococcus* species. Alternatively, the origin of a Cas protein used herein (e.g., Cas9) may be from a different species from which the RNA component(s) is derived (the Cas protein and RNA component(s) may be heterologous to each other); such heterologous Cas/RNA component RGENs should have DNA targeting activity.

Determining binding activity and/or endonucleolytic activity of a Cas protein herein toward a specific target DNA sequence may be assessed by any suitable assay known in the art, such as disclosed in U.S. Patent No. 8697359. A determination can be made, for example, by expressing a Cas protein and suitable RNA component in a cell, and then examining the predicted DNA target site for the presence of an indel (a Cas protein in this particular assay would typically have complete endonucleolytic activity [double-strand cleaving activity]). Examining for the presence of an alteration/modification (e.g., indel) at the predicted target site could be done via a DNA sequencing method or by inferring alteration/modification formation by assaying for loss of function of the target sequence, for example.

In still another example, Cas protein activity can be determined using an *in vitro* assay in which a Cas protein and suitable RNA component are mixed together along with a DNA polynucleotide containing a suitable target sequence. This assay can be used to detect binding (e.g., gel-shift) by Cas proteins lacking cleavage activity, or cleavage by Cas proteins that are endonucleolytically competent.

A Cas protein herein such as a Cas9 can further comprise a heterologous nuclear localization sequence (NLS) in certain aspects. A heterologous NLS amino acid sequence herein may be of sufficient strength to drive accumulation of a Cas protein, or Cas protein-CPP complex, in a detectable amount in the nucleus of a cell herein, for example. An NLS may comprise one (monopartite) or more (e.g., bipartite) short sequences (e.g., 2 to 20 residues) of basic, positively charged residues (e.g., lysine and/or arginine), and can be located anywhere in a Cas amino acid sequence but such that it is exposed on the protein surface. An NLS may be operably linked to the N-terminus or C-terminus of a Cas protein herein, for example. Two or more NLS sequences can be linked to a Cas protein, for example, such as on both the N- and C-termini of a Cas protein. Non-limiting examples of suitable NLS sequences herein include those disclosed in U.S. Patent Nos. 6660830 and 7309576 (e.g., Table 1 therein). A Cas protein as disclosed herein can be fused with a CPP (an example of a Cas protein covalently linked to a CPP), for example. It would be understood that such a Cas-CPP fusion protein can also comprise an NLS as described above. It would also be understood that, in embodiments in which a Cas protein is fused with an amino acid sequence targeting a different organelle (e.g., mitochondria), such a Cas protein typically would not contain an NLS.

A Cas protein can be part of a fusion protein comprising one or more heterologous protein domains (e.g., 1, 2, 3, or more domains in addition to the Cas protein). For example, a Cas protein can be covalently linked to a CPP and/or one or more additional heterologous amino acid sequences (see US provisional patent application NO. 62/036652, filed August 13, 2014). A Cas protein can also be covalently linked to one or more additional heterologous amino acid sequences not including a CPP, for example (a CPP would be non-covalently linked to a Cas fusion protein in such embodiments). A fusion protein comprising a Cas protein may comprise any additional protein sequence, and optionally a linker sequence between any two domains, such as between Cas and a first heterologous domain. Examples of protein domains that may be fused to a Cas protein herein include, without limitation, epitope tags (e.g., histidine [His, poly-histidine], V5, FLAG, influenza hemagglutinin [HA], myc, VSV-G, thioredoxin [Trx]), reporters (e.g., glutathione-5-transferase [GST], horseradish peroxidase [HRP], chloramphenicol acetyltransferase [CAT], beta-galactosidase, beta-glucuronidase [GUS], luciferase, green fluorescent protein [GFP], HcRed, DsRed, cyan fluorescent protein [CFP], yellow fluorescent protein [YFP], blue fluorescent protein [BFP]), and domains having one or more of the following activities: methylase activity, demethylase activity, transcription activation activity (e.g., VP16 or VP64), transcription repression activity, transcription release factor activity, histone modification activity, RNA cleavage activity and nucleic acid binding activity. A Cas protein in other embodiments may be in fusion with a protein that binds DNA molecules or other molecules, such as maltose binding protein (MBP), S-tag, Lex A DNA binding domain (DBD), GAL4A DNA binding domain, and herpes simplex virus (HSV) VP16. Additional domains that may be part of a fusion protein comprising a Cas protein herein are disclosed in U.S. Patent Appl. Publ. No. 2011/0059502. In certain embodiments in which a Cas protein is fused to a heterologous protein (e.g., a transcription factor), the Cas protein has DNA recognition and binding activity (when in complex with a suitable RNA component herein), but no DNA nicking or cleavage activity.

Other examples of heterologous domains that can be linked to a Cas protein herein include amino acid sequences targeting the protein to a particular organelle (i.e., localization signal). Examples of organelles that can be targeted include mitochondria and chloroplasts. Typically, such targeting domains are used instead of an NLS when targeting extra-nuclear DNA sites. A mitochondrial targeting sequence (MTS) can be situated at or near the N-terminus of a Cas protein, for example. MTS examples are disclosed in U.S. Patent Appl. Publ. Nos. 2007/0011759 and 2014/0135275. A chloroplast targeting sequence can be as disclosed in U.S. Patent Appl. Publ. No. 2010/0192262 or 2012/0042412, for example.

The protein component of an RGEN can be associated with at least one RNA component (thereby constituting a complete RGEN) that comprises a sequence complementary to a target site sequence on a chromosome or episome in a cell, for example. The RGEN in such embodiments can bind to the target site sequence, and cleave one or both DNA strands at the target site sequence. An RGEN can cleave one or both strands of a DNA target sequence, for example. An RGEN can cleave both strands of a DNA target sequence in another example. It would be understood that in all these embodiments, an RGEN protein component can be covalently or non-covalently linked to at least one CPP in an RGEN protein-CPP complex. The association of an RGEN protein-CPP complex with an RNA component herein can be characterized as forming an RGEN-CPP complex. Any disclosure herein regarding an RGEN can likewise apply to the RGEN component of an RGEN-CPP complex, unless otherwise noted.

An RGEN herein that can cleave both strands of a DNA target sequence typically comprises a Cas protein that has all of its endonuclease domains in a functional state (e.g., wild type endonuclease domains or variants thereof retaining some or all activity in each endonuclease domain). Thus, a wild type Cas protein (e.g., a Cas9 protein disclosed herein), or a variant thereof retaining some or all activity in each endonuclease domain of the Cas protein, is a suitable example of an RGEN that can cleave both strands of a DNA target sequence. A Cas9 protein comprising functional RuvC and HNH nuclease domains is an example of a Cas protein that can cleave both strands of a DNA target sequence. An RGEN herein that can cleave both strands of a DNA target sequence typically cuts both strands at the same position such that blunt-ends (i.e., no nucleotide overhangs) are formed at the cut site.

An RGEN herein that can cleave one strand of a DNA target sequence can be characterized herein as having nickase activity (e.g., partial cleaving capability). A Cas nickase (e.g., Cas9 nickase) herein typically comprises one functional endonuclease domain that allows the Cas to cleave only one strand (i.e., make a nick) of a DNA target sequence. For example, a Cas9 nickase may comprise (i) a mutant, dysfunctional RuvC domain and (ii) a functional HNH domain (e.g., wild type HNH domain). As another example, a Cas9 nickase may comprise (i) a functional RuvC domain (e.g., wild type RuvC domain) and (ii) a mutant, dysfunctional HNH domain.

Non-limiting examples of Cas9 nickases suitable for use herein are disclosed by Gasiunas et al. (Proc. Natl. Acad. Sci. U.S.A. 109:E2579-E2586), Jinek et al. (Science 337:816-821), Sapranauskas et al. (Nucleic Acids Res. 39:9275-9282) and in U.S. Patent Appl. Publ. No. 2014/0189896. For example, a Cas9 nickase herein can comprise an *S. thermophilus* Cas9 having an Asp-31 substitution (e.g., Asp-31-Ala) (an example of a mutant RuvC domain), or a His-865 substitution (e.g., His-865-Ala), Asn-882 substitution (e.g., Asn-882-Ala), or Asn-891 substitution (e.g., Asn-891-Ala) (examples of mutant HNH domains). Also for example, a Cas9 nickase herein can comprise an *S. pyogenes* Cas9 having an Asp-10 substitution (e.g., Asp-10-Ala), Glu-762 substitution (e.g., Glu-762-Ala), or Asp-986 substitution (e.g., Asp-986-Ala) (examples of mutant RuvC domains), or a His-840 substitution (e.g., His-840-Ala), Asn-854 substitution (e.g., Asn-854-Ala), or Asn-863 substitution (e.g., Asn-863-Ala) (examples of mutant HNH domains). Regarding *S. pyogenes* Cas9, the three RuvC subdomains are generally located at amino acid residues 1-59, 718-769 and 909-1098, respectively, and the HNH domain is located at amino acid residues 775-908 (Nishimasu et al., Cell 156:935-949).

A Cas9 nickase herein can be used for various purposes in cells, if desired. For example, a Cas9 nickase can be used to stimulate HR at or near a DNA target site sequence with a suitable polynucleotide modification template. Since nicked DNA is not a substrate for NHEJ processes, but is recognized by HR processes, nicking DNA at a specific target site should render the site more receptive to HR with a suitable polynucleotide modification template.

As another example, a pair of Cas9 nickases can be used to increase the specificity of DNA targeting. In general, this can be done by providing two Cas9 nickases that, by virtue of being associated with RNA components with different guide sequences, target and nick nearby DNA sequences on opposite strands in the region for desired targeting. Such nearby cleavage of each DNA strand creates a DSB (i.e., a DSB with single-stranded overhangs), which is then recognized as a substrate for NHEJ (leading to indel formation) or HR (leading to recombination with a suitable polynucleotide modification template, if provided). Each nick in these embodiments can be at least about 5, 10, 15, 20, 30, 40, 50, 60, 70, 80, 90, or 100 (or any integer between 5 and 100) bases apart from each other, for example. One or two Cas9 nickase proteins herein can be used in a Cas9 nickase pair as described above. For example, a Cas9 nickase with a mutant RuvC domain, but functioning HNH domain (i.e., Cas9 HNH⁺/RuvC⁻), could be used (e.g., *S. pyogenes* Cas9 HNH⁺/RuvC⁻). Each Cas9 nickase (e.g., Cas9 HNH⁺/RuvC⁻) would be directed to specific DNA sites nearby each other (up to 100 base pairs apart) by using suitable RNA components herein with guide RNA sequences targeting each nickase to each specific DNA site.

An RGEN herein can bind to a target site sequence, and cleave one or both strands of the target site sequence, in a chromosome, episome, or any other DNA molecule in the genome of a cell. This recognition and binding of a target sequence is specific, given that an RNA component of the RGEN comprises a sequence (guide sequence) that is complementary to a strand of the target sequence.

The terms "target site", "target sequence", "target DNA", "DNA target sequence", "target locus", "protospacer" and the like are used interchangeably herein. A target site sequence refers to a polynucleotide sequence on a chromosome, episome, or any other DNA molecule in the genome of a cell to which an RGEN herein can recognize, bind to, and optionally nick or cleave. A target site can be (i) an endogenous/native site in the cell, (ii) heterologous to the cell and therefore not be naturally occurring in the genome, or (iii) found in a heterologous genomic location compared to where it natively occurs.

A target site sequence herein is at least 13 nucleotides in length and has a strand with sufficient complementarity to a variable targeting domain (of a crRNA or gRNA) to be capable of hybridizing with the guide sequence and direct sequence-specific binding of a Cas protein or Cas protein complex to the target sequence (if a suitable PAM is adjacent to the target sequence in certain embodiments). A cleavage/nick site (applicable with a endonucleolytic or nicking Cas) can be within the target sequence (e.g., using a Cas9) or a cleavage/nick site could be outside of the target sequence (e.g., using a Cas9 fused to a heterologous endonuclease domain such as one derived from a Fokl enzyme). It is also possible for a target site sequence to be bound by an RGEN lacking cleavage or nicking activity.

An "artificial target site" or "artificial target sequence" herein refers to a target sequence that has been introduced into the genome of a cell. An artificial target sequence in some embodiments can be identical in sequence to a native target sequence in the genome of the cell, but be located at a different position (a heterologous position) in the genome, or it can different from the native target sequence if located at the same position in the genome of the cell.

The length of a target sequence herein can be at least 13, 14, 15, 16, 17, 18, 19, 20, 21, 22, 23, 24, 25, 26, 27, 28, 29, or 30 nucleotides; between 13-30 nucleotides; between 17-25 nucleotides; or between 17-20 nucleotides, for example. This length can include or exclude a PAM (protospacer-adjacent motif) sequence. Also, a strand of a target sequence herein has sufficient complementarity with a variable targeting domain (of a crRNA or gRNA) to hybridize with the guide sequence and direct sequence-specific binding of a Cas protein or Cas protein complex to the target sequence (if a suitable PAM is adjacent to the target sequence, see below). The degree of complementarity between a guide sequence and a strand of its corresponding DNA target sequence is at least about 90%, 91%, 92%, 93%, 94%, 95%, 96%, 97%, 98%, 99%, or 100%, for example. A target site herein may be located in a sequence encoding a gene product (e.g., a protein or an RNA) or a non-coding sequence (e.g., a regulatory sequence or a "junk" sequence), for example.

A "protospacer adjacent motif" (PAM) herein refers to a short sequence that is recognized by an RGEN herein. The sequence and length of a PAM herein can differ depending on the Cas protein or Cas protein complex used, but are typically 2, 3, 4, 5, 6, 7, or 8 nucleotides long, for example.

A PAM (protospacer-adjacent motif) sequence may be adjacent to the target site sequence. A PAM sequence is a short DNA sequence recognized by an RGEN herein. The associated PAM and first 11 nucleotides of a DNA target sequence are likely important to Cas9/gRNA targeting and cleavage (Jiang et al., Nat. Biotech. 31:233-239). The length of a PAM sequence herein can vary depending on the Cas protein or Cas protein complex used, but is typically 2, 3, 4, 5, 6, 7, or 8 nucleotides long, for example. A PAM sequence is immediately downstream from, or within 2, or 3 nucleotides downstream of, a target site sequence that is complementary to the strand in the target site that is in turn complementary to an RNA component guide sequence, for example. In embodiments herein in which an RGEN is an endonucleolytically active Cas9 protein complexed with an RNA component, Cas9 binds to the target sequence as directed by the RNA component and cleaves both strands immediately 5' of the third nucleotide position upstream of the PAM sequence. Consider the following example of a target site:PAM sequence:
5'-NNNNNNNNNNNNNNNNNNNNXGG-3' (SEQ ID NO:52). N can be A, C, T, or G, and X can be A, C, T, or G in this example sequence (X can also be referred to as N_{PAM}). The PAM sequence in this example is XGG (underlined). A suitable Cas9/RNA component complex would cleave this target immediately 5' of the double-underlined N. The string of N's in SEQ ID NO:52 represents target sequence that is at least about 90%, 91%, 92%, 93%, 94%, 95%, 96%, 97%, 98%, 99%, or 100% identical, for example, with a guide sequence in an RNA component herein (where any T's of the DNA target sequence would align with any U's of the RNA guide sequence). A guide sequence of an RNA component of a Cas9 complex, in recognizing and binding at this target sequence (which is representive of target sites herein), would anneal with the complement sequence of the string of N's; the percent complementarity between a guide sequence and the target site complement is at least about 90%, 91%, 92%, 93%, 94%, 95%, 96%, 97%, 98%, 99%, or 100%, for example. If a Cas9 nickase is used to target SEQ ID NO:52 in a genome, the nickase would nick immediately 5' of the double-underlined N or at the same position of the complementary strand, depending on which endonuclease domain in the nickase is dysfunctional. If a Cas9 having no nucleolytic activity (both RuvC and HNH domains dysfuntional) is used to target SEQ ID NO:52 in a genome, it would recognize and bind the target sequence, but not make any cuts to the sequence.

A PAM herein is typically selected in view of the type of RGEN being employed. A PAM sequence herein may be one recognized by an RGEN comprising a Cas, such as Cas9, derived from any of the species disclosed herein from which a Cas can be derived, for example. In certain embodiments, the PAM sequence may be one recognized by an RGEN comprising a Cas9 derived from S. *pyogenes, S. thermophilus, S. agalactiae, N. meningitidis, T. denticola,* or *F*. *novicida.* For example, a suitable Cas9 derived from S. *pyogenes* could be used to target genomic sequences having a PAM sequence of NGG (N can be A, C, T, or G). As other examples, a suitable Cas9 could be derived from any of the following species when targeting DNA sequences having the following PAM sequences: S. *thermophilus* (NNAGAA ) *S. agalactiae* (NGG, NNAGAAW [W is A or T], NGGNG, *N. meningitidis* (NNNNGATT), *T. denticola* (NAAAAC), or *F. novicida* (NG) (where N's in all these particular PAM sequences are A, C, T, or G). Other examples of Cas9/PAMs useful herein include those disclosed in Shah et al. (RNA Biology 10:891-899) and Esvelt et al. (Nature Methods 10:1116-1121). Examples of target sequences herein follow SEQ ID NO:43, but with the 'XGG' PAM replaced by any one of the foregoing PAMs.

An RNA component herein can comprise a sequence complementary to a target site sequence in a chromosome or episome in a cell. An RGEN can specifically bind to a target site sequence, and optionally cleave one or both strands of the target site sequence, based on this sequence complementary. Thus, the complementary sequence of an RNA component in certain embodiments of the disclosed disclosure can also be referred to as a guide sequence or variable targeting domain.

The guide sequence of an RNA component (e.g., crRNA or gRNA) herein can be at least 13, 14, 15, 16, 17, 18, 19, 20, 21, 22, 23, 24, 25, 26, 27, 28, 29, or 30 ribonucleotides in length; between 13-30 ribonucleotides in length; between 17-25 ribonucleotides in length; or between 17-20 ribonucleotides in length, for example. In general, a guide sequence herein has sufficient complementarity with a strand of a target DNA sequence to hybridize with the target sequence and direct sequence-specific binding of a Cas protein or Cas protein complex to the target sequence (if a suitable PAM is adjacent to the target sequence). The degree of complementarity between a guide sequence and its corresponding DNA target sequence is at least about 90%, 91%, 92%, 93%, 94%, 95%, 96%, 97%, 98%, 99%, or 100%, for example. The guide sequence can be engineered accordingly to target an RGEN to a DNA target sequence in a cell.

An RNA component herein can comprise a crRNA, for example, which comprises a guide sequence and a repeat (tracrRNA mate) sequence. The guide sequence is typically located at or near (within 1, 2, 3, 4, 5, 6, 7, 8, 9, 10 or more bases) the 5' end of the crRNA. Downstream the guide sequence of a crRNA is a "repeat" or "tracrRNA mate" sequence that is complementary to, and can hybridize with, sequence at the 5' end of a tracrRNA. Guide and tracrRNA mate sequences can be immediately adjacent, or separated by 1, 2, 3, 4 or more bases, for example. A tracrRNA mate sequence has, for example, at least 50%, 60%, 70%, 80%, 90%, 95%, 96%, 97%, 98%, 99%, or 100% sequence complementarity to the 5' end of a tracrRNA. In general, degree of complementarity can be with reference to the optimal alignment of the tracrRNA mate sequence and 5' end of the tracrRNA sequence, along the length of the shorter of the two sequences. The length of a tracrRNA mate sequence herein can be at least 8, 9, 10, 11, 12, 13, 14, 15, 16, 17, or 18 ribonucleotides in length, for example, and hybridizes with sequence of the same or similar length (e.g., plus or minus 1, 2, 3, 4, or 5 bases) at the 5' end of a tracrRNA. The length of a crRNA herein can be at least about 18, 20, 22, 24, 26, 28, 30, 32, 34, 36, 38, 40, 42, 44, 46, or 48 ribonucleotides; or about 18-48 ribonucleotides; or about 25-50 ribonucleotides, for example.

A tracrRNA can be included along with a crRNA in embodiments in which a Cas9 protein of a type II CRISPR system is comprised in the RGEN. A tracrRNA herein comprises in 5'-to-3' direction (i) a sequence that anneals with the repeat region (tracrRNA mate sequence) of crRNA and (ii) a stem loop-containing portion. The length of a sequence of (i) can be the same as, or similar with (e.g., plus or minus 1, 2, 3, 4, or 5 bases), any of the tracrRNA mate sequence lengths disclosed above, for example. The total length of a tracrRNA herein (i.e., sequence components [i] and [ii]) can be at least about 30, 35, 40, 45, 50, 55, 60, 65, 70, 75, 80, 85, or 90 (or any integer between 30 and 90) ribonucleotides, for example. A tracrRNA may further include 1, 2, 3, 4, 5, or more uracil residues at the 3'-end, which may be present by virtue of expressing the tracrRNA with a transcription terminator sequence.

A tracrRNA herein can be derived from bacterial species, such as but not limited to *Streptococcus* species (e.g., *S. pyogenes, S. thermophilus)* or can include those disclosed in U.S. Patent No. 8697359 and Chylinski et al. (RNA Biology 10:726-737).

The terms "ribozyme", "ribonucleic acid enzyme" and "self-cleaving ribozyme" are used interchangeably herein. A ribozyme refers to one or more RNA sequences that form secondary, tertiary, and/or quaternary structure(s) that can cleave RNA at a specific site, particularly at a *cis*-site relative to the ribozyme sequence (i.e., autocatalytic, or self-cleaving). The general nature of ribozyme nucleolytic activity has been described (e.g., Lilley, Biochem. Soc. Trans. 39:641-646). A "hammerhead ribozyme" (HHR) herein may comprise a small catalytic RNA motif made up of three base-paired stems and a core of highly conserved, non-complementary nucleotides that are involved in catalysis. Pley et al. (Nature 372:68-74) and Hammann et al. (RNA 18:871-885), disclose hammerhead ribozyme structure and activity. A hammerhead ribozyme herein may comprise a "minimal hammerhead" sequence as disclosed by Scott et al. (Cell 81:991-1002), for example.

The terms "targeting", "gene targeting", "DNA targeting", "editing", "gene editing" and "DNA editing" are used interchangeably herein. DNA targeting herein may be the specific introduction of an indel, knock-out, or knock-in at a particular DNA sequence, such as in a chromosome or episome of a cell. In general, DNA targeting can be performed herein by cleaving one or both strands at a specific DNA sequence in a cell with a Cas protein associated with a suitable RNA component. Such DNA cleavage, if a double-strand break (DSB), can prompt NHEJ processes which can lead to indel formation at the target site. Also, regardless of whether the cleavage is a single-strand break (SSB) or DSB, HR processes can be prompted if a suitable polynucleotide modification template or donor DNA is provided at the DNA nick or cleavage site. Such an HR process can be used to introduce a knock-out or knock-in at the target site, depending on the sequence of the polynucleotide modification template. Alternatively, DNA targeting herein can refer to specific association of a Cas/RNA component complex herein to a target DNA sequence, where the Cas protein does or does not cut a DNA strand (depending on the status of the Cas protein's endonucleolytic domains).

The term "indel" herein refers to an insertion or deletion of a nucleotide base or bases in a target DNA sequence in a chromosome or episome. Such an insertion or deletion may be of 1, 2, 3, 4, 5, 6, 7, 8, 9, 10, or more bases, for example. An indel in certain embodiments can be even larger, at least about 20, 30, 40, 50, 60, 70, 80, 90, or 100 bases. If an indel is introduced within an open reading frame (ORF) of a gene, oftentimes the indel disrupts wild type expression of protein encoded by the ORF by creating a frameshift mutation.

The terms "knock-out", "gene knock-out" and "genetic knock-out" are used interchangeably herein. A knock-out represents a DNA sequence of a cell herein that has been rendered partially or completely inoperative by targeting with a Cas protein; such a DNA sequence prior to knock-out could have encoded an amino acid sequence, or could have had a regulatory function (e.g., promoter), for example. A knock-out may be produced by an indel (by NHEJ, prompted by Cas-mediated cleavage), or by specific removal of sequence (by HR, prompted by Cas-mediated cleavage or nicking, when a suitable polynucleotide modification template is also used), that reduces or completely destroys the function of sequence at, adjoining, or near the targeting site. A knocked out DNA polynucleotide sequence herein can alternatively be characterized as being partially or totally disrupted or downregulated, for example.

The terms "knock-in", "gene knock-in" and "genetic knock-in" are used interchangeably herein. A knock-in represents the replacement or insertion of a DNA sequence at a specific DNA sequence in a cell by targeting with a Cas protein (by HR, prompted by Cas-mediated cleavage or nicking, when a suitable donor DNA is also used). Examples of knock-ins are a specific insertion of a polynucleotide of interest, a heterologous amino acid coding sequence in a coding region of a gene, or a specific insertion of a transcriptional regulatory element in a genetic locus.

The terms "recombinant DNA molecule", "recombinant construct", "expression construct", " construct", "construct", and "recombinant DNA construct" are used interchangeably herein. A recombinant construct comprises an artificial combination of nucleic acid fragments, e.g., regulatory and coding sequences that are not all found together in nature. For example, a construct may comprise regulatory sequences and coding sequences that are derived from different sources, or regulatory sequences and coding sequences derived from the same source, but arranged in a manner different than that found in nature. Such a construct may be used by itself or may be used in conjunction with a vector or plasmid. The skilled artisan will also recognize that different independent gene editing events may result in different levels and patterns of expression (Jones et al., (1985) EMBO J 4:2411-2418; De Almeida et al., (1989) Mol Gen Genetics 218:78-86), and thus that multiple events are typically screened in order to obtain lines displaying the desired expression level and pattern. Such screening may be accomplished standard molecular biological, biochemical, and other assays including Southern analysis of DNA, Northern analysis of mRNA expression, PCR, real time quantitative PCR (qPCR), reverse transcription PCR (RT-PCR), immunoblotting analysis of protein expression, enzyme or activity assays, and/or phenotypic analysis.

The term "expression", as used herein, refers to the production of a functional end-product (e.g., an mRNA, guide RNA, or a protein) in either precursor or mature form.

The term "providing" herein refers to providing (introducing) a nucleic acid (e.g., expression construct, plasmid) or protein into a cell. Providing includes reference to the incorporation of a nucleic acid into a eukaryotic or prokaryotic cell where the nucleic acid may be incorporated into the genome of the cell, and includes reference to the transient provision of a nucleic acid or protein to the cell. Providing includes reference to electroporation (Green MR, Sambrook J. 2012. Molecular Cloning: A Laboratory Manual, Fourth Edition ed. Cold Spring Harbor Laboratory Press, Cold Spring Harbor, NY), heat-shock treatments (Green MR, Sambrook J. 2012. Molecular Cloning: A Laboratory Manual, Fourth Edition ed. Cold Spring Harbor Laboratory Press, Cold Spring Harbor, NY), chemical treatments (Green MR, Sambrook J. 2012. Molecular Cloning: A Laboratory Manual, Fourth Edition ed. Cold Spring Harbor Laboratory Press, Cold Spring Harbor, NY), phage delivery (Tyler BM, Goldberg RB. 1976. Transduction of chromosomal genes between enteric bacteria by bacteriophage P1. Journal of bacteriology 125:1105-1111), mating, conjugation and transduction (Methods for General and Molecular Bacteriology. 1994. ASM Press, Washington D.C.). Providing in the context of inserting a nucleic acid fragment (e.g., a recombinant DNA construct/expression construct) into a cell, includes "transfection" or "transformation" or "transduction" and includes reference to the incorporation of a nucleic acid fragment into a prokaryotic cell where the nucleic acid fragment may be incorporated into the genome of the cell (e.g., large circular genome, plasmid), converted into an autonomous replicon, or transiently expressed.

A nucleic acid molecule that has been provided into an organism/cell may be one that replicates autonomously in the organism/cell, or that integrates into the genome of the organism/cell, or that exists transiently in the cell without replicating or integrating. Non-limiting examples of nucleic acid molecules that can be provided to a cell are disclosed herein, such as plasmids and linear DNA molecules.

As described herein, the guide RNA/Cas endonuclease system can be used in combination with a co-delivered polynucleotide modification template to allow for editing of a genomic nucleotide sequence of interest. Also, as described herein, for each embodiment that uses a guide RNA/Cas endonuclease system, a similar guide *polynucleotide*/*Cas* endonuclease system can be deployed where the guide polynucleotide does not solely comprise ribonucleic acids but wherein the guide polynucleotide comprises a combination of RNA-DNA molecules or solely comprise DNA molecules.

A "modified nucleotide" or "edited nucleotide" refers to a nucleotide sequence of interest that comprises at least one alteration when compared to its non-modified nucleotide sequence. Such "alterations" include, for example: (i) replacement of at least one nucleotide, (ii) a deletion of at least one nucleotide, (iii) an insertion of at least one nucleotide, or (iv) any combination of (i) - (iii).

The term "polynucleotide modification template" refers to a polynucleotide that comprises at least one nucleotide modification when compared to the nucleotide sequence to be edited. A nucleotide modification can include, for example: (i) a replacement of at least one nucleotide, (ii) a deletion of at least one nucleotide, (iii) an insertion of at least one nucleotide, or (iv) any combination of (i) - (iii). Optionally, the polynucleotide modification template can further comprise homologous nucleotide sequences flanking the at least one nucleotide modification, wherein the flanking homologous nucleotide sequences provide sufficient homology to the desired nucleotide sequence to be edited.

As used herein, "donor DNA" is a DNA construct that comprises a polynucleotide of Interest to be inserted into the target site of a Cas endonuclease. The donor DNA construct can further comprise a first and a second region of homology that flank the polynucleotide of Interest. The first and second regions of homology of the donor DNA share homology to a first and a second genomic region, respectively, present in or flanking the target site of the plant genome.

A polynucleotide modification template or donor DNA can be able to undergo homologous recombination (HR) with a DNA target site. A "homologous sequence" within a polynucleotide modification template or donor DNA herein can, for example, comprise or consist of a sequence of at least about 25 nucleotides, for example, having 100% identity with a sequence at or near a target site, or at least about 90%, 91%, 92%, 93%, 94%, 95%, 96%, 97%, 98%, or 99% identity with a sequence at or near a target site.

A polynucleotide modification template or donor DNA can have two homologous sequences separated by a sequence (or base pair) that is heterologous to sequence at a target site. These two homologous sequences of such a polynucleotide modification template or donor DNA can be referred to as "homology arms", which flank the heterologous sequence. HR between a target site and a polynucleotide modification template or donor DNA with two homology arms typically results in the editing of a sequence at the target site.

A region of homology can be of any length that is sufficient to promote homologous recombination at the cleaved target site. For example, the region of homology can comprise at least 5-10, 5-15, 5-20, 5-25, 5-30, 5-35, 5-40, 5-45, 5-50, 5-55, 5-60, 5-65, 5- 70, 5-75, 5-80, 5-85, 5-90, 5-95, 5-100, 5-200, 5-300, 5-400, 5-500, 5-600, 5-700, 5-800, 5-900, 5-1000, 5-1100, 5-1200, 5-1300, 5-1400, 5-1500, 5-1600, 5-1700, 5-1800, 5-1900, 5-2000, 5-2100, 5-2200, 5-2300, 5-2400, 5-2500, 5-2600, 5-2700, 5-2800, 5-2900, 5-3000, 5-3100 or more bases in length such that the region of homology has sufficient homology to undergo homologous recombination with the corresponding genomic region. "Sufficient homology" indicates that two polynucleotide sequences have sufficient structural similarity to act as substrates for a homologous recombination reaction. The structural similarity includes overall length of each polynucleotide fragment, as well as the sequence similarity of the polynucleotides. Sequence similarity can be described by the percent sequence identity over the whole length of the sequences, and/or by conserved regions comprising localized similarities such as contiguous nucleotides having 100% sequence identity, and percent sequence identity over a portion of the length of the sequences.

The amount of homology or sequence identity shared by a target and a polynucleotide modification template or a Donor DNA can vary and includes total lengths and/or regions having unit integral values in the ranges of about 1-20 bp, 20-50 bp, 50-100 bp, 75-150 bp, 100-250 bp, 150-300 bp, 200-400 bp, 250-500 bp, 300-600 bp, 350-750 bp, 400-800 bp, 450-900 bp, 500-1000 bp, 600-1250 bp, 700-1500 bp, 800-1750 bp, 900-2000 bp, 1-2.5 kb, 1.5-3 kb, 2-4 kb, 2.5-5 kb, 3-6 kb, 3.5-7 kb, 4-8 kb, 5-10 kb, or up to and including the total length of the target site. These ranges include every integer within the range, for example, the range of 1-20 bp includes 1, 2, 3, 4, 5, 6, 7, 8, 9, 10, 11, 12, 13, 14, 15, 16, 17, 18, 19 and 20 bp. The amount of homology can also described by percent sequence identity over the full aligned length of the two polynucleotides which includes percent sequence identity of about at least 50%, 55%, 60%, 65%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. Sufficient homology includes any combination of polynucleotide length, global percent sequence identity, and optionally conserved regions of contiguous nucleotides or local percent sequence identity, for example sufficient homology can be described as a region of 75-150 bp having at least 80% sequence identity to a region of the target locus. Sufficient homology can also be described by the predicted ability of two polynucleotides to specifically hybridize under high stringency conditions, see, for example, Sambrook et al., (1989) Molecular Cloning: A Laboratory Manual, (Cold Spring Harbor Laboratory Press, NY); Current Protocols in Molecular Biology, Ausubel et al., Eds (1994) Current Protocols, (Greene Publishing Associates, Inc. and John Wiley & Sons, Inc.); and, Tijssen (1993) Laboratory Techniques in Biochemistry and Molecular Biology--Hybridization with Nucleic Acid Probes, (Elsevier, New York).

In one embodiment, the disclosure describes a method for editing a target sequence in the genome of an *Escherichia coli* cell, the method comprising providing at least one recombinant DNA construct comprising a DNA sequence encoding a guide RNA and a circular polynucleotide modification template to an E.coli cell comprising a Cas9 endonuclease DNA sequence operably linked to an inducible promoter, wherein said Cas9 endonuclease DNA sequence encodes a Cas9 endonuclease wherein the guide RNA and the Cas9 endonuclease can form a guide RNA/Cas9 endonuclease complex (RGEN) that is capable of mediating cleavage of the target sequence to allow for homologous recombination mediated gene editing at a target site in the genome of said E. coli cell, wherein said polynucleotide modification template comprises at least one nucleotide modification of said target sequence. The nucleotide sequence in the genome of an E. *coli* cell can be selected from the group consisting of a promoter sequence, a terminator sequence, a regulatory element sequence, a coding sequence, a prophage, a pseudogene, an exogenous gene, an endogenous gene. The recombinant DNA construct comprising a DNA sequence encoding a guide RNA can be provided via a circular plasmid. The recombinant DNA construct and the circular polynucleotide modification template can be provided on separate plasmids or they can be provided on a single plasmid. The recombinant DNA construct and the circular polynucleotide template can be provided via one mean selected from the group consisting of electroporation, heat-shock, phage delivery, mating, conjugation and transduction, or any one combination thereof.

The nucleotide sequence to be edited can be a sequence that is endogenous, artificial, pre-existing, or transgenic to the cell that is being edited. For example, the nucleotide sequence in the genome of a cell can be a native gene, a mutated gene, a non-native gene, a foreign gene, or a transgene that is stably incorporated into the genome of a cell. Editing of such nucleotide may result in a further desired phenotype or genotype.

In one embodiment, the disclosure describes a method for editing a target sequence in the genome of an Escherichia coli cell, the method comprising providing at least a first recombinant DNA construct comprising a DNA sequence encoding a guide RNA, a circular polynucleotide modification template, and a second recombinant DNA construct comprising a DNA sequence encoding Cas9 endonuclease operably linked to an inducible promoter, to an E.coli cell, wherein the Cas9 endonuclease introduces a double-strand break at a target site in the genome of said E. coli cell, wherein said polynucleotide modification template comprises at least one nucleotide modification of said target sequence.

In one embodiment of the disclosure , the method comprises a method for inserting a polynucleotide sequence of interest in the genome of an *Escherichia coli* cell, the method comprising providing at least one recombinant DNA construct comprising a DNA sequence encoding a guide RNA and a circular donor DNA to an *E.coli* cell comprising a Cas9 endonuclease DNA sequence operably linked to an inducible promoter, wherein said Cas9 endonuclease DNA sequence encodes a Cas9 endonuclease that is capable of introducing a double-strand break at a target site in the genome of said *E. coli* cell, wherein said donor DNA comprises a polynucleotide .

Example of target sites in *E. coli* include sugar utilization genes (e.g. galactokinase, galK), metabolic genes (e.g. isocitrate dehydrogenase, icd, (Kabir MM, Shimizu K. 2004.. Applied microbiology and biotechnology 65:84-96), biosynthetic genes (e.g. thymidylate synthase, thyA (Belfort M, Maley G, Pedersen-Lane J, Maley F..PNAS. 1983. 80(16):4914-18), transcriptional regulators (e.g. the general stress response regulator, rpoS (Notley-McRobb L, King T, Ferenci T (2002) J Bacteriol 184(3);806-11. PMID: 11790751), signaling proteins (e.g. sensor for anoxic redox control, arcB(luchi S, Matsuda Z, Fujiwara T, Lin EC (1990). Mol Microbiol 1990;4(5);715-27. PMID: 2201868), tRNAs (e.g. tRNA alanine, alaU (Siekevitz P, Zamecnik PC (1981). Cell Biol 91(3 Pt 2);53s-65s. PMID: 7033244)), stress-response proteins (e.g. phage shock protein A, pspA (Adams H, Teertstra W, Demmers J, Boesten R, Tommassen J (2003). J Bacteriol 2003; 185(4); 1174-80. PMID: 12562786)), ribosomal components (e.g. S12 ribosoml protein,rpsL,(Funatsu G, Yaguchi M, Wittmann-Liebold B(1977). "Primary stucture of protein S12 from the small Escherichia coli ribosomal subunit." FEBS Lett 73(1);12-7. PMID: 320034) and 23s ribosomal RNA, rrlD(Arkov AL, Hedenstierna KO, Murgola EJ (2002). "Mutational evidence for a functional connection between two domains of 23S rRNA in translation termination." J Bacteriol 184(18);5052-7. PMID: 12193621)), DNA replication (e.g. DNA polymerase II, polB(Chen H, Bryan SK, Moses RE (1989). "Cloning the polB gene of Escherichia coli and identification of its product." J Biol Chem 264(34);20591-5. PMID: 2684981)), Transcriptional machinery (e.g. the β' subunit of RNA polymerase, rpoC(Squires C, Krainer A, Barry G, Shen WF, Squires CL (1981). "Nucleotide sequence at the end of the gene for the RNA polymerase beta' subunit (rpoC)." Nucleic Acids Res 1981;9(24);6827-40. PMID: 6278450), transporters (eg. lactose permease, lacY(Buchel DE, Gronenborn B, Muller-Hill B (1980). "Sequence of the lactose permease gene." Nature 1980;283(5747);541-5. PMID: 6444453)), phage attachment sites (eg. λ attachment site, attB (Landy A, Ross W (1977). "Viral integration and excision: structure of the lambda att sites." Science 197(4309); 1147-60. PMID: 331474)), prophage genes (eg. rac prophoage inhibitor of cell division, kilR(Conter A, Bouche JP, Dassain M (1996). "Identification of a new inhibitor of essential division gene ftsZ as the kil gene of defective prophage Rac." J Bacteriol 178(17);5100-4. PMID: 8752325)),or cell division (eg.cell division ring, ftsZ (Robinson AC, Kenan DJ, Hatfull GF, Sullivan NF, Spiegelberg R, Donachie WD (1984). "DNA sequence and transcriptional organization of essential cell division genes ftsQ and ftsA of Escherichia coli: evidence for overlapping transcriptional units." J Bacteriol 160(2);546-55. PMID: 6094474),). Additional genes suitable for target sites have been defined (Karp PD, Weaver D, Paley S, Fulcher C, Kubo A, Kothari A, Krummenacker M, Subhraveti P, Weerasinghe D, Gama-Castro S, Huerta AM, Muniz-Rascado L, Bonavides-Martinez C, Weiss V, Peralta-Gil M, Santos-Zavaleta A, Schroder I, Mackie A, Gunsalus R, Collado-Vides J, Keseler IM, Paulsen I. 2014. The EcoCyc Database. EcoSal Plus 2014; Keseler IM, Collado-Vides J, Santos-Zavaleta A, Peralta-Gil M, Gama-Castro S, Muniz-Rascado L, Bonavides-Martinez C, Paley S, Krummenacker M, Altman T, Kaipa P, Spaulding A, Pacheco J, Latendresse M, Fulcher C, Sarker M, Shearer AG, Mackie A, Paulsen I, Gunsalus RP, Karp PD. 2011. EcoCyc: a comprehensive database of Escherichia coli biology. Nucleic acids research 39:D583-590.; Keseler IM, Bonavides-Martinez C, Collado-Vides J, Gama-Castro S, Gunsalus RP, Johnson DA, Krummenacker M, Nolan LM, Paley S, Paulsen IT, Peralta-Gil M, Santos-Zavaleta A, Shearer AG, Karp PD. 2009. EcoCyc: a comprehensive view of Escherichia coli biology. Nucleic acids research 37:D464-470;. Escherichia coli and Salmonella typhimurium: Cellular and Molecular Biology, 1987 First ed. American Society of Microbiology, Washington, DC.

The terms "cell-penetrating peptide" (CPP) and "protein transduction domain" (PTD) are used interchangeably herein. A CPP refers to a peptide , typically of about 5-60 amino acid residues in length, that can facilitate cellular uptake of protein cargo, particularly one or more RGEN protein components described herein (e.g., Cas9 protein). Such protein cargo can be associated with one or more CPPs through covalent or non-covalent linkage. A CPP can also be characterized in certain embodiments as being able to facilitate the movement or traversal of protein cargo across/through one or more of a lipid bilayer, micelle, cell membrane, organelle membrane, vesicle membrane, or cell wall. A CPP herein can be cationic, amphipathic, or hydrophobic in certain embodiments. (see for example US provisional patent application NO. 62/036652, filed August 13, 2014).

The terms "percent by volume", "volume percent", "vol %" and "v/v %" are used interchangeably herein. The percent by volume of a solute in a solution can be determined using the formula: [(volume of solute)/(volume of solution)] x 100%.

The terms "percent by weight", "weight percentage (wt %)" and "weight-weight percentage (% w/w)" are used interchangeably herein. Percent by weight refers to the percentage of a material on a mass basis as it is comprised in a composition, mixture, or solution.

The terms "polynucleotide", "polynucleotide sequence", and "nucleic acid sequence" are used interchangeably herein. These terms encompass nucleotide sequences and the like. A polynucleotide may be a polymer of DNA or RNA that is single- or double-stranded, that optionally contains synthetic, non-natural or altered nucleotide bases. A polynucleotide may be comprised of one or more segments of cDNA, genomic DNA, synthetic DNA, or mixtures thereof. Nucleotides (ribonucleotides or deoxyribonucleotides) can be referred to by a single letter designation as follows: "A" for adenylate or deoxyadenylate (for RNA or DNA, respectively), "C" for cytidylate or deoxycytidylate (for RNA or DNA, respectively), "G" for guanylate or deoxyguanylate (for RNA or DNA, respectively), "U" for uridylate (for RNA), "T" for deoxythymidylate (for DNA), "R" for purines (A or G), "Y" for pyrimidines (C or T), "K" for G or T, "H" for A or C or T, "I" for inosine, "W" for A or T, and "N" for any nucleotide (e.g., N can be A, C, T, or G, if referring to a DNA sequence; N can be A, C, U, or G, if referring to an RNA sequence). Any RNA sequence (e.g., crRNA, tracrRNA, gRNA) disclosed herein may be encoded by a suitable DNA sequence.

The term "isolated" refers to a polynucleotide or polypeptide molecule that has been completely or partially purified from its native source. In some instances, the isolated polynucleotide or polypeptide molecule is part of a greater composition, buffer system or reagent mix. For example, the isolated polynucleotide or polypeptide molecule can be comprised within a cell or organism in a heterologous manner.

The term "gene" refers to a DNA polynucleotide sequence that expresses an RNA (RNA is transcribed from the DNA polynucleotide sequence) from a coding region, which RNA can be a messenger RNA (encoding a protein) or a non-protein-coding RNA (e.g., a crRNA, tracrRNA, or gRNA herein). A gene may refer to the coding region alone, or may include regulatory sequences upstream and/or downstream to the coding region (e.g., promoters, 5'-untranslated regions, 3'-transcription terminator regions). A coding region encoding a protein can alternatively be referred to herein as an "open reading frame" (ORF). A gene that is "native" or "endogenous" refers to a gene as found in nature with its own regulatory sequences; such a gene is located in its natural location in the genome of a host cell. A "chimeric" gene refers to any gene that is not a native gene, comprising regulatory and coding sequences that are not found together in nature (i.e., the regulatory and coding regions are heterologous with each other). Accordingly, a chimeric gene may comprise regulatory sequences and coding sequences that are derived from different sources, or regulatory sequences and coding sequences derived from the same source, but arranged in a manner different than that found in nature. A "foreign" or "heterologous" gene refers to a gene that is introduced into the host organism by gene transfer. Foreign/heterologous genes can comprise native genes inserted into a non-native organism, native genes introduced into a new location within the native host, or chimeric genes. The polynucleotide sequences in certain embodiments disclosed herein are heterologous. A "codon-optimized" open reading frame has its frequency of codon usage designed to mimic the frequency of preferred codon usage of the host cell.

A "modified gene" or "edited gene" refers to a gene of interest that comprises at least one alteration when compared to its non-modified gene sequence. Such "alterations" include, for example: (i) replacement of at least one nucleotide, (ii) a deletion of at least one nucleotide, (iii) an insertion of at least one nucleotide, or (iv) any combination of (i) - (iii).

"Regulatory sequences" as used herein refer to nucleotide sequences located upstream of a gene's transcription start site (e.g., promoter), 5' untranslated regions, and 3' non-coding regions, and which may influence the transcription, processing or stability, or translation of an RNA transcribed from the gene. Regulatory sequences herein may include promoters, enhancers, silencers, 5' untranslated leader sequences, introns, polyadenylation recognition sequences, RNA processing sites, effector binding sites, stem-loop structures, and other elements involved in regulation of gene expression. One or more regulatory elements herein may be heterologous to a coding region herein.

A "promoter" as used herein refers to a DNA sequence capable of controlling the transcription of RNA from a gene. In general, a promoter sequence is upstream of the transcription start site of a gene. Promoters may be derived in their entirety from a native gene, or be composed of different elements derived from different promoters found in nature, or even comprise synthetic DNA segments. Promoters that cause a gene to be expressed in a cell at most times under all circumstances are commonly referred to as "constitutive promoters". One or more promoters herein may be heterologous to a coding region herein.

A "strong promoter" as used herein refers to a promoter that can direct a relatively large number of productive initiations per unit time, and/or is a promoter driving a higher level of gene transcription than the average transcription level of the genes in a cell.

Constitutive *E*. *coli* promoters are well known in the art and include promoters that lack regulation by a transcription factor and are recognized by RNA polymerase alone (Shimada T, Yamazaki Y, Tanaka K, Ishihama A. The whole set of constitutive promoters recognized by RNA polymerase RpoD holoenzyme of Escherichia coli. PLoS One. 2014. Mar 6; 9(3):e90447; Science 2002, Stochastic Gene Expression in a Single Cell Vol. 297 no. 5584 pp. 1183-1186).

The terms "3' non-coding sequence", "transcription terminator" and "terminator" as used herein refer to DNA sequences located downstream of a coding sequence. This includes polyadenylation recognition sequences and other sequences encoding regulatory signals capable of affecting mRNA processing or gene expression.

The term "cassette" as used herein refers to a promoter operably linked to a DNA sequence encoding a protein-coding RNA or non-protein-coding RNA. A cassette may optionally be operably linked to a 3' non-coding sequence.

The terms "upstream" and "downstream" as used herein with respect to polynucleotides refer to "5' of" and "3' of", respectively.

The term "expression" as used herein refers to (i) transcription of RNA (e.g., mRNA or a non-protein coding RNA such as crRNA, tracrRNA, or gRNA) from a coding region, or (ii) translation of a polypeptide from mRNA.

When used to describe the expression of a gene or polynucleotide sequence, the terms "down-regulation", "disruption", "inhibition", "inactivation", and "silencing" are used interchangeably herein to refer to instances when the transcription of the polynucleotide sequence is reduced or eliminated. This results in the reduction or elimination of RNA transcripts from the polynucleotide sequence, which results in a reduction or elimination of protein expression derived from the polynucleotide sequence (if the gene comprised an ORF). Alternatively, down-regulation can refer to instances where protein translation from transcripts produced by the polynucleotide sequence is reduced or eliminated. Alternatively still, down-regulation can refer to instances where a protein expressed by the polynucleotide sequence has reduced activity. The reduction in any of the above processes (transcription, translation, protein activity) in a cell can be by about 20%, 30%, 40%, 50%, 60%, 70%, 80%, 90%, 95%, or 100% relative to the transcription, translation, or protein activity of a suitable control cell. Down-regulation can be the result of a targeting event as disclosed herein (e.g., indel, knock-out), for example.

The terms "control cell" and "suitable control cell" are used interchangeably herein and may be referenced with respect to a cell in which a particular modification (e.g., over-expression of a polynucleotide, down-regulation of a polynucleotide) has been made (i.e., an "experimental cell"). A control cell may be any cell that does not have or does not express the particular modification of the experimental cell. For example, a control cell may be a direct parent of the experimental cell, which direct parent cell does not have the particular modification that is in the experimental cell. Alternatively, a control cell may be a parent of the experimental cell that is removed by one or more generations. Alternatively still, a control cell may be a sibling of the experimental cell, which sibling does not comprise the particular modification that is present in the experimental cell.

The term "increased" as used herein may refer to a quantity or activity that is at least about 1%, 2%, 3%, 4%, 5%, 6%, 7%, 8%, 9%, 10%, 11%, 12%, 13%, 14%, 15%, 16%, 17%, 18%, 19%, 20%, 50%, 100%, or 200% more than the quantity or activity for which the increased quantity or activity is being compared. The terms "increased", "elevated", "enhanced", "greater than", and "improved" are used interchangeably herein. The term "increased" can be used to characterize the expression of a polynucleotide encoding a protein, for example, where "increased expression" can also mean "over-expression".

The term "operably linked" as used herein refers to the association of two or more nucleic acid sequences such that that the function of one is affected by the other. For example, a promoter is operably linked with a coding sequence when it is capable of affecting the expression of that coding sequence. That is, the coding sequence is under the transcriptional control of the promoter. Coding sequences can be operably linked to regulatory sequences, for example. Also, for example, a crRNA can be operably linked (fused to) a tracrRNA herein such that the tracrRNA mate sequence of the crRNA anneals with 5' sequence of the tracrRNA.

The term "recombinant" as used herein refers to an artificial combination of two otherwise separated segments of sequence, e.g., by chemical synthesis or by the manipulation of isolated segments of nucleic acids by genetic engineering techniques.

Methods for preparing recombinant constructs/vectors herein (e.g., a DNA polynucleotide encoding an RNA component cassette herein, or a DNA polynucleotide encoding a Cas protein or Cas-CPP fusion protein herein) can follow standard recombinant DNA and molecular cloning techniques as described by J. Sambrook and D. Russell (Molecular Cloning: A Laboratory Manual, 3rd Edition, Cold Spring Harbor Laboratory Press, Cold Spring Harbor, NY, 2001); T.J. Silhavy et al. (Experiments with Gene Fusions, Cold Spring Harbor Laboratory Press, Cold Spring Harbor, NY, 1984); and F.M. Ausubel et al. (Short Protocols in Molecular Biology, 5th Ed. Current Protocols, John Wiley and Sons, Inc., NY, 2002), for example.

A "phenotypic marker" is a screenable or selectable marker that includes visual markers and selectable markers whether it is a positive or negative selectable marker. Any phenotypic marker can be used. Specifically, a selectable or screenable marker comprises a DNA segment that allows one to identify, or select for or against a molecule or a cell that contains it, often under particular conditions. These markers can encode an activity, such as, but not limited to, production of RNA, peptide, or protein, or can provide a binding site for RNA, peptides, proteins, inorganic and organic compounds or compositions and the like.

Examples of selectable markers for *E. coli* include resistance to antibiotics (Ampicillin, Carbenicillin, Penicillin, Chloramphenicol, Kanamycin, Tetracycline, Eythromycin, spectinomycin, streptomycin) and auxotrophic markers (amino acid biosynthesis, sugar utilization, and vitamin biosynthesis) (Methods for General and Molecular Bacteriology. 1994. ASM Press, Washington D.C).

Screenable markers in E. coli include fluorescent proteins (GFP, RFP, CFP, YFP), sugar utilization (lactose, ribose, glucose, sucrose, galactose, glycerol) (Methods for General and Molecular Bacteriology. 1994. ASM Press, Washington D.C).and the generation of unique primer binding sites.

The terms "sequence identity" or "identity" as used herein with respect to polynucleotide or polypeptide sequences refer to the nucleic acid residues or amino acid residues in two sequences that are the same when aligned for maximum correspondence over a specified comparison window. Thus, "percentage of sequence identity" or "percent identity" refers to the value determined by comparing two optimally aligned sequences over a comparison window, wherein the portion of the polynucleotide or polypeptide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the results by 100 to yield the percentage of sequence identity. It would be understood that, when calculating sequence identity between a DNA sequence and an RNA sequence, T residues of the DNA sequence align with, and can be considered "identical" with, U residues of the RNA sequence. For purposes of determining percent complementarity of first and second polynucleotides, one can obtain this by determining (i) the percent identity between the first polynucleotide and the complement sequence of the second polynucleotide (or vice versa), for example, and/or (ii) the percentage of bases between the first and second polynucleotides that would create canonical Watson and Crick base pairs.

The Basic Local Alignment Search Tool (BLAST) algorithm, which is available online at the National Center for Biotechnology Information (NCBI) website, may be used, for example, to measure percent identity between or among two or more of the polynucleotide sequences (BLASTN algorithm) or polypeptide sequences (BLASTP algorithm) disclosed herein. Alternatively, percent identity between sequences may be performed using a Clustal algorithm (e.g., ClustalW or ClustalV). For multiple alignments using a Clustal method of alignment, the default values may correspond to GAP PENALTY=10 and GAP LENGTH PENALTY=10. Default parameters for pairwise alignments and calculation of percent identity of protein sequences using a Clustal method may be KTUPLE=1, GAP PENALTY=3, WINDOW=5 and DIAGONALS SAVED=5. For nucleic acids, these parameters may be KTUPLE=2, GAP PENALTY=5, WINDOW=4 and DIAGONALS SAVED=4. Alternatively still, percent identity between sequences may be performed using an EMBOSS algorithm (e.g., needle) with parameters such as GAP OPEN=10, GAP EXTEND=0.5, END GAP PENALTY=false, END GAP OPEN=10, END GAP EXTEND=0.5 using a BLOSUM matrix (e.g., BLOSUM62).

Herein, a first sequence that is "complementary" to a second sequence can alternatively be referred to as being in the "antisense" orientation with the second sequence.

Various polypeptide amino acid sequences and polynucleotide sequences are disclosed herein as features of certain embodiments of the disclosed disclosure. Variants of these sequences that are at least about 70-85%, 85-90%, or 90%-95% identical to the sequences disclosed herein can be used. Alternatively, a variant amino acid sequence or polynucleotide sequence can have at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity with a sequence disclosed herein. The variant amino acid sequence or polynucleotide sequence has the same function/activity of the disclosed sequence, or at least about 80%, 81%, 82%, 83%, 84%, 85%, 86%, 87%, 88%, 89%, 90%, 91%, 92%, 93%, 94%, 95%, 96%, 97%, 98%, or 99% of the function/activity of the disclosed sequence.

All the amino acid residues disclosed herein at each amino acid position of Cas9 proteins herein are examples. Given that certain amino acids share similar structural and/or charge features with each other (i.e., conserved), the amino acid at each position in a Cas9 can be as provided in the disclosed sequences or substituted with a conserved amino acid residue ("conservative amino acid substitution") as follows:
1. The following small aliphatic, nonpolar or slightly polar residues can substitute for each other: Ala (A), Ser (S), Thr (T), Pro (P), Gly (G);
2. The following polar, negatively charged residues and their amides can substitute for each other: Asp (D), Asn (N), Glu (E), Gln (Q);
3. The following polar, positively charged residues can substitute for each other: His (H), Arg (R), Lys (K);
4. The following aliphatic, nonpolar residues can substitute for each other: Ala (A), Leu (L), Ile (I), Val (V), Cys (C), Met (M); and
5. The following large aromatic residues can substitute for each other: Phe (F), Tyr (Y), Trp (W).

A genome of a bacterial cell, such as an *E. coli* cell, herein refers to a DNA molecule that can exist in a cell autonomously (can replicate and pass on to daughter cells). Genomic DNA can be either native or heterologous to a cell. Examples of genomic DNA in E. Coli include DNA located on a large circular DNA molecule as well as plasmid DNA

The term "cell" herein refers to any type of cell such as a prokaryotic or eukaryotic cell. A eukaryotic cell has a nucleus and other membrane-enclosed structures (organelles), whereas a prokaryotic cell lacks a nucleus. A cell in certain embodiments can be a mammalian cell or non-mammalian cell. Non-mammalian cells can be eukaryotic or prokaryotic. For example, a non-mammalian cell herein can refer to a microbial cell or cell of a non-mammalian multicellular organism such as a plant, insect, nematode, avian species, amphibian, reptile, or fish. A microbial cell herein can refer to a fungal cell (e.g., yeast cell), prokaryotic cell, protist cell (e.g., algal cell), euglenoid cell, stramenopile cell, or oomycete cell, for example. A prokaryotic cell herein can refer to a bacterial cell or archaeal cell, for example.

A bacterial cell can be those in the form of cocci, bacilli, spirochetes, spheroplasts, protoplasts, etc. Other non-limiting examples of bacteria include those that are Gram-negative and Gram-positive. Still other non-limiting examples of bacteria include those of the genera *Salmonella* (e.g., *S. typhi, S. enteritidis), Shigella* (e.g., *S. dysenteriae), Escherichia* (e.g., *E. coli*)*, Enterobacter, Serratia, Proteus, Yersinia, Citrobacter, Edwardsiella, Providencia, Klebsiella, Hafnia, Ewingella, Kluyvera, Morganella, Planococcus, Stomatococcus, Micrococcus, Staphylococcus* (e.g., *S. aureus, S. epidermidis), Vibrio* (e.g., *V. cholerae), Aeromonas, Plessiomonas, Haemophilus* (e.g., *H. influenzae), Actinobacillus, Pasteurella, Mycoplasma* (e.g., *M. pneumonia), Ureaplasma, Rickettsia, Coxiella, Rochalimaea, Ehrlichia, Streptococcus* (e.g., *S. pyogenes, S. mutans, S. pneumoniae), Enterococcus* (e.g., *E. faecalis), Aerococcus, Gemella, Lactococcus* (e.g., *L. lactis), Leuconostoc* (e.g., *L. mesenteroides), Pedicoccus, Bacillus* (e.g., *B*. *cereus, B. subtilis, B*. *thuringiensis), Corynebacterium* (e.g., *C. diphtheriae), Arcanobacterium, Actinomyces, Rhodococcus, Listeria* (e.g., *L. monocytogenes), Erysipelothrix, Gardnerella, Neisseria* (e.g., *N. meningitidis, N. gonorrhoeae), Campylobacter, Arcobacter, Wolinella, Helicobacter* (e.g., *H. pylori), Achromobacter, Acinetobacter, Agrobacterium* (e.g., *A. tumefaciens), Alcaligenes, Chryseomonas, Comamonas, Eikenella, Flavimonas, Flavobacterium, Moraxella, Oligella, Pseudomonas* (e.g., *P. aeruginosa), Shewanella, Weeksella, Xanthomonas, Bordetella, Franciesella, Brucella, Legionella, Afipia, Bartonella, Calymmatobacterium, Cardiobacterium, Streptobacillus, Spirillum, Peptostreptococcus, Peptococcus, Sarcinia, Coprococcus, Ruminococcus, Propionibacterium, Mobiluncus, Bifidobacterium, Eubacterium, Lactobacillus* (e.g., *L. lactis, L. acidophilus), Rothia, Clostridium* (e.g., *C. botulinum, C. perfringens), Bacteroides, Porphyromonas, Prevotella, Fusobacterium, Bilophila, Leptotrichia, Wolinella, Acidaminococcus, Megasphaera, Veilonella, Norcardia, Actinomadura, Norcardiopsis, Streptomyces, Micropolysporas, Thermoactinomycetes, Mycobacterium* (e.g., *M. tuberculosis, M. bovis, M. leprae), Treponema, Borrelia* (e.g., *B*. *burgdorferi), Leptospira, and Chlamydiae.* A bacteria can optionally be characterized as a pest/pathogen of a plant or animal (e.g., human) in certain embodiments. Bacteria can be comprised in a mixed microbial population (e.g., containing other bacteria, or containing yeast and/or other bacteria) in certain embodiments.

An archaeal cell in certain embodiments can be from any Archaeal phylum, such as Euryarchaeota, Crenarchaeota, Nanoarchaeota, Korarchaeota, Aigarchaeota, or Thaumarchaeota. Archaeal cells herein can be extremophilic (e.g., able to grow and/or thrive in physically or geochemically extreme conditions that are detrimental to most life), for example. Some examples of extremophilic archaea include those that are thermophilic (e.g., can grow at temperatures between 45-122 °C), hyperthermophilic (e.g., can grow at temperatures between 80-122 °C), acidophilic (e.g., can grow at pH levels of 3 or below), alkaliphilic (e.g., can grow at pH levels of 9 or above), and/or halophilic (e.g., can grow in high salt concentrations [e.g., 20-30% NaCl]). Examples of archaeal species include those of the genera *Halobacterium* (e.g., *H. volcanii), Sulfolobus* (e.g., *S. solfataricus, S. acidocaldarius), Thermococcus* (e.g., *T. alcaliphilus, T. celer, T. chitonophagus, T. gammatolerans, T. hydrothermalis, T. kodakarensis, T. litoralis, T. peptonophilus, T. profundus, T. stetteri), Methanocaldococcus* (e.g., *M. thermolithotrophicus, M. jannaschii), Methanococcus* (e.g., *M. maripaludis), Methanothermobacter* (e.g., *M. marburgensis, M. thermautotrophicus), Archaeoglobus* (e.g., *A. fulgidus), Nitrosopumilus* (e.g., *N. maritimus), Metallosphaera* (e.g., *M. sedula), Ferroplasma, Thermoplasma, Methanobrevibacter* (e.g., *M. smithii),* and *Methanosphaera* (e.g., *M. stadtmanae).*

Recombineering allows the editing of bacterial DNA using linear double and single stranded polynucleotide editing templates (Datsenko KA, Wanner BL. 2000. One-step inactivation of chromosomal genes in Escherichia coli K-12 using PCR products. Proceedings of the National Academy of Sciences of the United States of America 97:6640-6645; Thomason LC, Sawitzke JA, Li X, Costantino N, Court DL. 2014. Recombineering: genetic engineering in bacteria using homologous recombination. Current protocols in molecular biology / edited by Frederick M. Ausubel et al. 106:1 16 11-11 1639). In order to utilize a linear or single stranded editing template expression of exogenous phage recombinase proteins are required (Datsenko KA, Wanner BL. 2000. One-step inactivation of chromosomal genes in Escherichia coli K-12 using PCR products. Proceedings of the National Academy of Sciences of the United States of America 97:6640-6645; US patent 7,736,851 DNA cloning method, issued Jan 15, 2010). Typically, small changes such as point mutations or deletions can be generated using short single stranded oligonucleotide editing templates. However, for larger changes or insertions of genes the presence of a selectable marker on the polynucleotide editing template is required in order to isolate colonies containing the desired edit due to the low frequency of recombination (ca 10⁻⁵ to 10⁻⁷). Once the edit is made the selectable marker must be removed, often leaving scar in the genome (Datsenko KA, Wanner BL. 2000. One-step inactivation of chromosomal genes in Escherichia coli K-12 using PCR products. Proceedings of the National Academy of Sciences of the United States of America 97:6640-6645).

Exogenous recombinase(s) includes proteins of homologous recombination systems provided in addition the cells native homologous recombination machinery (ie. expressed via non-natural means).

A RecET protein includes proteins of the ATP-independent, recA-indpendent homolgous recombination pathway of the Rac prophage (Kuzminov A. 1999. Recombinational repair of DNA damage in Escherichia coli and bacteriophage lambda. Microbiology and molecular biology reviews: MMBR 63:751-813).

A lambda-red protein includes a red, redβ, and redγ proteins of the phage lambda (Smith GR. 1988. Homologous recombination in procaryotes. Microbiological reviews 52:1-28).

A RecBCD inhibitor includes a protein that binds to and inhibits RecBCD function (eg lambda Gam protein) (Murphy KC. 2007. The lambda Gam protein inhibits RecBCD binding to dsDNA ends. Journal of molecular biology 371:19-24).

A DNA polynucleotide sequence comprising (i) a promoter operably linked to (ii) a nucleotide sequence encoding a guide RNA or a Cas endonuclease can typically be used for stable and/or transient expression of the guide RNA or cas endonuclease described herein. Such a polynucleotide sequence can be comprised within a plasmid, cosmid, phagemid, bacterial artificial chromosome (BAC), virus, or linear DNA (e.g., linear PCR product), for example, or any other type of vector or construct useful for providing a polynucleotide sequence into a cell.

Bacterial promoters include bacteriophage λ promoter left (PL)(Menart V, Jevsevar S, Vilar M, Trobis A, Pavko A. 2003. Constitutive versus thermoinducible expression of heterologous proteins in Escherichia coli based on strong PR,PL promoters from phage lambda. Biotechnology and bioengineering 83:181-190), bacteriophage λ promoter right (PR)(Menart V, Jevsevar S, Vilar M, Trobis A, Pavko A. 2003. Constitutive versus thermoinducible expression of heterologous proteins in Escherichia coli based on strong PR, PL promoters from phage lambda. Biotechnology and bioengineering 83:181-190) the arabinose utilization operon promoter (PBAD)(Guzman LM, Belin D, Carson MJ, Beckwith J. 1995. Tight regulation, modulation, and high-level expression by vectors containing the arabinose PBAD promoter. Journal of bacteriology 177:4121-4130), phage T7 RNA polymerase controlled promoters(PT7)(Ikeda RA, Ligman CM, Warshamana S. 1992. T7 promoter contacts essential for promoter activity in vivo. Nucleic acids research 20:2517-2524), the promoter of the lactose utilization operon of E. coli (Plac,(Gronenborn B. 1976. Overproduction of phage lambda repressor under control of the lac promotor of Escherichia coli. Molecular & general genetics: MGG 148:243-250) hybrid trp and lac promoters (Ptac)(de Boer HA, Comstock LJ, Vasser M. 1983. The tac promoter: a functional hybrid derived from the trp and lac promoters. Proceedings of the National Academy of Sciences of the United States of America 80:21-25), and the phage T5 promoter (PT5)(Bujard H, Gentz R, Lanzer M, Stueber D, Mueller M, Ibrahimi I, Haeuptle MT, Dobberstein B. 1987. A T5 promoter-based transcription-translation system for the analysis of proteins in vitro and in vivo. Methods in enzymology 155:416-433). Other suitable promoters for expression in bacteria have been described (Green MR, Sambrook J. 2012. Molecular Clonine: A Laboratory Manual, Fourth Edition ed. Cold Spring Harbor Laboratory Press, Cold Spring Harbor, NY; Karp PD, et al. 2014. The EcoCyc Database. EcoSal Plus 2014; Keseler IM et al. 2011. EcoCyc: a comprehensive database of Escherichia coli biology. Nucleic acids research 39:D583-590.)

In certain embodiments, a DNA polynucleotide comprising a cassette for expressing an RNA component comprises a suitable transcription termination sequence downstream of the RNA component sequence. Examples of transcription termination sequences useful herein are disclosed in U.S. Pat. Appl. Publ. No. 2014/0186906. Such embodiments typically comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, or more residues following the end of the RNA component sequence, depending on the choice of terminator sequence. These additional residues can be all U residues, or at least 90%, 91%, 92%, 93%, 94%, 95%, 96%, 97%, 98%, or 99% U residues, for example, depending on the choice of terminator sequence. Alternatively, a ribozyme sequence (e.g., hammerhead or HDV ribozyme) can be 3' of (e.g., 1, 2, 3, 4, 5, 6, 7, 8, 9, 10 or more nucleotides downstream) the RNA component sequence, for example. A 3' ribozyme sequence can be positioned accordingly such that it cleaves itself from the RNA component sequence; such cleavage would render a transcript ending exactly at the end of the RNA component sequence, or with 1, 2, 3, 4, 5, 6, 7, 8, 9, 10, 11, 12, 13, 14, 15, or more residues following the end of the RNA component sequence, for example.

A targeting method herein can be performed in such a way that two or more DNA target sites are targeted in the method, for example. Such a method can optionally be characterized as a multiplex method. Two, three, four, five, six, seven, eight, nine, ten, or more target sites can be targeted at the same time in certain embodiments. A multiplex method is typically performed by a targeting method herein in which multiple different RNA components are provided, each designed to guide an RGEN to a unique DNA target site. For example, two or more different RNA components can be used to prepare a mix of RGEN-CPP complexes *in vitro* (e.g., following a procedure disclosed herein for associating an RNA component with an RGEN protein-CPP complex), which mix is then contacted with a cell.

Another aspect of multiplex targeting herein can comprise providing two or more different RNA components in a cell which associate with the RGEN protein components of RGEN protein-CPP complexes that have traversed into the cell. Such a method can comprise, for example, providing to the cell (i) individual DNA polynucleotides, each of which express a particular RNA component that, and/or (ii) at least one DNA polynucleotide encoding two or more RNA components (e.g., see below disclosure regarding tandem ribozyme-RNA component cassettes).

A multiplex method can optionally target DNA sites very close to the same sequence (e.g., a promoter or open reading frame, and/or sites that are distant from each other (e.g., in different genes and/or chromosomes). A multiplex method in other embodiments can be performed with (for HR) or without (for NHEJ leading to indel and/or base substitution) suitable polynucleotide modification templates depending on the desired outcome of the targeting (if an endonuclease- or nickase-competent RGEN is used). In still other embodiments, a multiplex method can be performed with a repressing or activating RGEN as disclosed herein. For example, multiple repressing RGENs can be provided that down-regulate a set of genes, such as genes involved in a particular metabolic pathway.

### EXAMPLES

The disclosed disclosure is further defined in the following Examples. It should be understood that these Examples, while indicating certain preferred aspects of the disclosure, are given by way of illustration only. From the above discussion and these Examples, one skilled in the art can ascertain the essential characteristics of this disclosure, and can make various changes and modifications of the disclosure to adapt it to various uses and conditions.

### EXAMPLE 1

### Construction of a Cas9 endonuclease expression vector for use in Escherichia coli.

In this example an inducible Cas9 expression vector for genome editing in *Escherichia coli* was constructed. Cas9 expression in response to an inducer was confirmed.

The Cas9 gene from *Streptococcus pyrogenes* M1 GAS SF370 (SEQ ID NO: 1) was *Yarrowia* codon optimized per standard techniques known in the art (SEQ ID NO: 2). In order to localize the Cas9 protein to the nucleus of the cells, *Simian virus 40* (SV40) monopartite (MAPKKKRKV, SEQ ID NO: 3) nuclear localization signal was incorporated at the carboxy terminus of the Cas9 open reading frame. The Yarrowia codon optimized Cas9 gene was fused to a Yarrowia constitutive promoter, FBA1 (SEQ ID NO: 4), by standard molecular biology techniques. An example of a Yarrowia codon optimized Cas9 expression cassette (SEQ ID NO: 5) containing the constitutive FBA promoter, Yarrowia codon optimized Cas9, and the SV40 nuclear localization signal. The Cas9 expression cassette was cloned into the plasmid pZuf and the new construct called pZufCas9 (SEQ ID NO: 6).

The Yarrowia codon optimized Cas9-SV40 fusion gene (SEQ ID NO: 7) was amplified from pZufCas9 using standard molecular biology techniques. Primers for the reaction were GGGGGAATTCGACAAGAAATACTCCATCGGCCTGG (Forward, SEQ ID NO: 8) and CCCCAAGCTTAGCGGCCGCTTAGACCTTTCG (Reverse, SEQ ID NO: 9) adding a 5' EcoRI site and a 3' HindIII site to the fusion. The PCR product (SEQ ID NO: 10) was purified using standard techniques. The purified fragment was cloned into the EcoRI and Hindlll sites of pBAD/HisB from life technologies (SEQ ID NO: 11) to create pRF48 (SEQ ID NO: 12).

*E. coli* Top10 cells (Life technologies) were transformed with pRF48. The transformed cells were maintained on L broth (1% (w/v) Tryptone, 0.5% (w/v) Yeast extract. 1% (w/v) NaCl) + 100µg/ml Ampicillin + 0.4% (w/v) glucose to repress expression of the Cas9 protein. Cells were grown at 37°C overnight at 220 RPM in L Broth +1 00µg/ml Ampicillin + 0.4%(w/v) glucose. The cells were diluted 1:100 in 1L of 2x YT medium (1.6% Tryptone, 1.0% (w/v) Yeast Extract, 0.5%(w/v) NaCl) in a 2.8L Fernbach flask. The culture was grown at 37°C 220RPM until OD₆₀₀ reached 0.438. 1ml of culture was pelleted and resuspend in 43.8µl of 1x Laemmli buffer and frozen at -20°C. L-arabinose was added to a final concentration of 0.2% (w/v) to induce the P_{BAD} promoter driving the *Yarrowia* optimized Cas9 gene. The culture was shifted to 18°C, 180 RPM for 20 hours.

After induction by L-arabinose the OD₆₀₀ was 3.01. An aliquot of 0.332ml of culture was pelleted. The cells were resuspended in 100µl of 1X laemmeli buffer. Both the pre-induction and post induction samples were heated to 95°C for 5 minutes and 10µl was loaded onto a 12.5% tris-glycine SDS polyacrylamide gel. 200 volts were applied to the gel for 30 minutes. The gel was stained using simply blue stain to resolve protein bands. Expression of the Yarrowia optimized Cas9 protein in *E. coli* under control of an arabinose inducible promoter was robust (Figure 4).

### EXAMPLE 2

### Construction of circular expression plasmids encoding single guide RNAs targeting the galK gene of E. coli

In order to modify (edit) the endogenous *galK* gene of *E. coli* four (4) Cas9 endonuclease target sites within the E. Coli *galK* gene were identified (Figure 5) : galK-1 (SEQ ID NO:13, table 1), galK-2 (SEQ ID NO: 14, table 1), galK-3 (SEQ ID NO: 15, table 1), and galK-4 (SEQ ID NO: 16, table 1).

**Table 1. Targeting sequences for galK gene editing in E. Coli.**

| Targeting sequence name | Gene | Targeting sequence | PAM | SEQ ID No: |
|---|---|---|---|---|
| galK-1 | *galK* | ATCAGCGGCAATGTGCCGCA | GGG | 13 |
| galK-2 | *galK* | ATGACCGGCGGCGGATTTGG | CGG | 14 |
| galK-3 | *galK* | ATAGTTTTCATGTGCGACAA | TGG | 15 |
| galK-4 | *galK* | ATGATCTTTCTTGCCGAGCG | CGG | 16 |

DNA fragments corresponding to the genomic galK1 target sequences lacking the PAM domain (defined in Table 1) were fused to the a *Streptococcus pyrogenes* Cas Recognition domain (SEQ ID NO: 17) making complete DNA templates for single guide RNAs . The DNA fragments encoding the guide RNAs are show in SEQ ID NO: 18-21. The sgRNAs for gal1 K-1 to gal1-K4 are shown in SEQ ID Nos: 22-25.

In order to express the sgRNAs in *E. coli* cells four sgRNA expression cassettes were constructed (SEQ ID NOs: 28-31). The sgRNAs were put under control of the P_{L} promoter of the bacteriophage lambda (SEQ ID NO: 26). In order to induce transcriptional termination of the sgRNA the 3' end of the CR domain was fused to the strong bacteriophage lambda terminator (SEQ ID NO: 27). The GalK-1 sgRNA expression cassette (SEQ ID NO: 28) was designed to target the galK-1 genomic target site (SEQ ID NO: 13). The GalK-2 sgRNA expression cassette (SEQ ID NO: 29) was designed to target the galK-1 genomic target site (SEQ ID NO:14). The GalK-3 sgRNA expression cassette (SEQ ID NO:30) was designed to target the galK-3 genomic target site (SEQ ID NO:15). The GalK-4 sgRNA expression cassette, (SEQ ID NO:31) was designed to target the galK-4 genomic target site (SEQ ID NO:16).

Each sgRNA expression cassette contained a 5' HinDIII restriction site (AAGCTT) and a 3' BamHI restriction site (GGATCC). Each sgRNA expression cassette was cloned into the HinDIII/BamHI sites of pACYC184 (SEQ ID NO: 32) to generate the circular plasmids (see guide RNA plasmid, Figure 1 and Figure 2) pRF50 (targeting galK-1, SEQ ID NO: 33), pRF51 (targeting galK-2, SEQ ID NO: 34), pRF53 (targeting galK-3, SEQ ID NO: 35), and pRF55 (targeting galK-4, SEQ ID NO: 36).

### EXAMPLE 3

### Construction of circular plasmids containing the polynucleotide modification template for gene editing in E. coli.

To enable gene editing (modification) in *E. coli* using (e/g/. gene deletion of the galK gene), a polynucleotide modification template was prepared that lacked a portion of the galK gene (referred to as the galK deletion template) as follows:
The 454 bp fragment directly 5' of the translational start site of the *E. coli galK* gene (SEQ ID NO: 37) was amplified using standard PCR techniques using a forward primer (GGGaagcttggattatgttcagcgcgagc, SEQ ID NO: 38) adding a 5' HinDIII restriction site for cloning and a reverse primer
(tgccagtgcgggagtttcgtTTCTTACACTCCGGATTCGC, SEQ ID NO: 39) adding 20 bp of the sequencing directly 3' of the stop codon of the *galK* gene to produce the upstream overlap extension product (SEQ ID NO: 40). The 376 bp directly 3' of the translational stop site of the *E. coli galK* gene (SEQ ID NO: 41) was amplified using standard PCR techniques using forward primer
(GCGAATCCGGAGTGTAAGAAacgaaactcccgcactggca, SEQ ID NO: 42) adding 20bp of the sequence directly 5' of the start codon of the *galK* gene and a reverse primer (GGGaagcttGCAAACAGCACCTGACGATCG, SEQ ID NO: 43) adding a 3' HinDIII restriction site producing the downstream overlap extension product (SEQ ID NO: 44) The PCR products were purified using Zymo clean and concentrate columns. 10ng of each PCR product were used to extend the overlapping 20nt using the forward primer for the 5' fragment (GGGaagcttggattatgttcagcgcgagc, SEQ ID NO: 38) and the reverse primer of the 3' fragment
(GGGaagcttGCAAACAGCACCTGACGATCG, SEQ ID NO: 43). The full length *galK* deletion template (SEQ ID NO: 45) was cloned into the HinDIII sites of the conditionally replicating plasmid pKD3 (SEQ ID NO: 46) to create a circular *gal*K deletion template plasmid pRF113 (SEQ ID NO: 47) The *gal*K deletion template plasmid pRF113 (referred to as template plasmid in Figure 1 and Figure 2) lacks an expression cassette for a Pi protein (Inuzuka M. 1985. Plasmid-encoded initiation protein is required for activity at all three origins of plasmid R6K DNA replication in vitro. FEBS letters 181:236-240) thereby rendering it unable to replicate autonomously. Hence, once this circular template is provided to an *E. coli* cell it can function as a template for RGEN mediated gene editing but it will not be replicated and therefore be absent in any progeny cells that are cultured from said E. coli cell.

### EXAMPLE 4

### Efficient genome editing of the galk gene in E. coli using a guide RNA /Cas endonuclease system in combination with a circular plasmid containing a polynucleotide modification template.

Strain EF44 containing a deletion of the *galE* gene of *E. coli* is sensitive to the presence of galactose in the growth medium due to accumulation of the toxic product phosphor-galactose (Incorporate E. coli and S.typhimurium: Cellular and Molecular Biology Authors: Frederick C. Neidhardt, John L. Ingraham, Roy Curtiss III. ASM Press Washington D.C. 1987)). In this strain, mutations causing a loss of function in the gene encoding the galactose kinase (*galK*) rescue the galactose sensitivity allowing the strain to grow in the presence of galactose.

To create an *E. coli* strain containing a Cas9 plasmid comprising a Cas9 expression cassette (as depicted in Figure 1), the plasmid pRF48 was introduced into the *E. coli* strain EF44 as follows. Strain EF44 strain was transformed with pRF48 (SEQ ID NO: 12) and colonies were selected on L broth agar plates containing 100µg/ml Ampicillin and 0.4% (W/V) glucose to repress the expression of the Cas9 gene from the pBAD promoter to create the *E. coli* strain EF56 (Δ*ga*/*E* pRF48) containing the Cas9 plasmid.

A single colony of EF56 was inoculated in L Broth containing 100µg/ml ampicillin and 0.4% (W/V) Glucose and grown for 18 hours at 37°C 230 RPM. The strain was then diluted into fresh L broth containing 100µg/ml ampicillin and grown at 37°C 230RPM for 2 hours. L-arabinose was added to a final concentration of 0.2% (W/V) to induce expression of Cas9 from the P_{BAD} promoter and the cells were grown for an additional 1 hour. Cells were made electrocompetent via standard protocols. 100 µl of induced electrocompetent EF56 cells were transformed with 200ng of pACYC184 (SEQ ID NO: 32), pRF50 (SEQ ID NO: 33), pRF51 (SEQ ID NO: 34), pRF53 (SEQ ID NO: 35), or pRF55 (SEQ ID NO: 36) and either 1µg pRF113 (SEQ ID NO: 47), 1µg of linear polynucleotide modification template (SEQ ID NO: 44), or no polynucleotide modification template plasmid DNA. Cells were electroporated in a 1mM gap cuvette at 1750 volts. 1 ml of SOC medium was added and cells were allowed to recover for 3 hours at 37°C 230RPM. Cells were plated on L broth plates solidified with 1.5% (w/v) agar containing 100 µg/ml Ampicillin and 25µg/ml Chloramphenicol to select for cells containing both pRF48 (SEQ ID NO:12) and the corresponding pACYC184 (SEQ ID NO: 32), pRF50 (SEQ ID NO: 33), pRF51 (SEQ ID NO: 34), pRF53 (SEQ ID NO: 35), or pRF55 (SEQ ID NO: 36). Plates were incubated for 20 hours at 37°C.

Colonies were transferred from the L broth 100µg/ml Ampicillin/25µg/ml Chloramphenicol plates to Minimal A medium solidified with 1.5% (w/v) agar containing 0.2% (w/v) glycerol and 0.2% (w/v) galactose using replica plating to screen for galactose resistant isolates. For each transformation the frequency of galactose resistance was calculated by dividing the number of galactose resistant colonies by the total number of colonies on the original plate (Table 2).

**Table 2: Frequency of Galactose resistant colonies.**

| gRNA plasmid | polynucleotide modification template | Percent (%) Galactose resistant±Standard error of the mean | Percent (%) Galactose resistant colonies from Homologous recombination |
|---|---|---|---|
| pACYC 184 | None | 0±0 | 0 |
| pACYC 184 | pRF113 | 0±0 | 0 |
| pACYC 184 | Linear | 0 | 0 |
| pRF50 | None | 0.6±0.4 | 0 |
| pRF50 | pRF113 | 06±0.3 | 84.5 |
| pRF50 | Linear | 0.4 | 0 |
| pRF51 | None | 0.5±0.5 | 0 |
| pRF51 | pRF113 | 0.2±0.2 | 0 |
| pRF51 | Linear | 0.3 | 0 |
| pRF53 | None | 0.3±0.2 | 0 |
| pRF53 | pRF113 | 3.6±1.9 | 41.5 |
| pRF53 | Linear | 0 | 0 |
| pRF55 | None | 2.4±1.5 | 0 |
| pRF55 | pRF113 | 1.9±1.7 | 9 |
| pRF55 | Linear | 0.2 | 0 |

Frequencies were dependent on target site. In order to determine the frequency of Homologous recombination the *galK* locus (SEQ ID NO: 48) using standard PCR techniques using a forward primer (ggcgaagagaatcaacactgg, SEQ ID NO: 49) and a reverse primer (GCAAACAGCACCTGACGATCG, SEQ ID NO: 50). In a WT strain the entire *galK* locus is amplified (SEQ ID NO: 48) leading to a PCR product that is 1717bp in length. In cells where recombination has occurred between the *galK* locus and the HR polynucleotide modification template pRF113 the PCR product is 569bp in length (SEQ ID NO: 50). Figure 6 shows a gel from the amplification of colonies from a pRF50/pRF113 editing experiment with an HR frequency of 75%. The HR frequency was determined by dividing the number of colonies where the deletion allele of *galK* was amplified, indicating precise editing by the total number of colonies assayed by colony PCR. Colonies that are Gal^{R} in the absence of polynucleotide modification template fail to allow amplification of the *galK* locus.

This example shows that efficient genome editing of the galK gene in *E. coli* was successfully accomplished using a guide RNA/Cas endonuclease system in combination with a circular plasmid containing a polynucleotide modification template.

## Claims

1. A method for editing a target sequence in the genome of an *Escherichia coli* cell, the method comprising providing at least one recombinant DNA construct comprising a DNA sequence encoding a guide RNA and a circular polynucleotide modification template to an *E.coli* cell comprising a Cas9 endonuclease DNA sequence operably linked to an inducible promoter, wherein said Cas9 endonuclease DNA sequence encodes a Cas9 endonuclease, wherein the guide RNA and the Cas9 endonuclease can form a guide RNA/Cas9 endonuclease complex (RGEN) that is capable of mediating cleavage of the target sequence to allow for homologous recombination mediated gene editing at a target site in the genome of said *E*. *coli* cell, wherein said polynucleotide modification template comprises at least one nucleotide modification of said target sequence.

2. The method of claim 1, wherein the target sequence in the genome of an *E*. *coli* cell is selected from the group consisting of a promoter sequence, a terminator sequence, a regulatory element sequence, a coding sequence, a prophage, a pseudogene, and an exogenous gene.

3. The method of claim 1 or claim 2, wherein said recombinant DNA construct comprising a DNA sequence encoding a guide RNA is provided via a circular plasmid.

4. The method of any one of claims 1 to 3, wherein the recombinant DNA construct and the circular polynucleotide modification template are each provided on separate plasmids or are provided on a single plasmid.

5. The method of any one of claims 1 to 4, wherein the recombinant DNA construct and the circular polynucleotide template are provided via one means selected from the group consisting of electroporation, heat-shock, phage delivery, mating, conjugation and transduction.

6. The method of any one of claims 1 to 5, wherein said target site is flanked by a first genomic region and a second genomic region, wherein the circular polynucleotide template further comprises a first region of homology to said first genomic region and a second region of homology to said second genomic region.

7. The method of any one of claims 1 to 6, wherein the *E*. *coli* cell does not express an exogenous recombinase protein.

8. The method of any one of claims 1 to 7, wherein the *E*. *coli* cell does not express a protein selected from the group comprising a RecET protein, a lambda-red protein, and a RecBCD inhibitor.

9. The method of any one of claims 1 to 8, further comprising growing progeny cells from said *E*. *coli* cell, wherein the progeny cell comprises the at least one nucleotide modification of said target sequence.

10. The method of any one of claims 1 to 9 wherein the target site is located in an *E*. *coli* galK gene.

11. The method of claim 1, being a method for producing a galK mutant E.coli cell, the method comprising:
a) providing at least one circular recombinant DNA construct comprising a DNA sequence encoding a guide RNA and at least one circular polynucleotide modification template to an *E*. *coli* cell comprising a Cas9 endonuclease DNA sequence operably linked to an inducible promoter, wherein said Cas9 endonuclease DNA sequence encodes a Cas endonuclease that is capable of introducing a double-strand break at a target site within a *galK* genomic sequence in the *E*. *coli* genome, wherein said circular polynucleotide modification template comprises at least one nucleotide modification of said *galK* genomic sequence;
b) growing progeny cells from the *E*. *coli* cell of (a); and,
c) evaluating the progeny cells of (b) for the presence of said at least one nucleotide modification.

12. The method of claim 1, being a method for editing a target sequence in the genome of an *Escherichia coli* cell, the method comprising providing at least a first recombinant DNA construct comprising a DNA sequence encoding a guide RNA, a circular polynucleotide modification template, and a second recombinant DNA construct comprising a DNA sequence encoding Cas9 endonuclease operably linked to an inducible promoter, to an *E.coli* cell, wherein the Cas9 endonuclease introduces a double-strand break at a target site in the genome of said *E*. *coli* cell, wherein said polynucleotide modification template comprises at least one nucleotide modification of said target sequence.

13. The method of claim 12, wherein the first recombinant DNA construct, the second recombinant DNA construct, and the circular polynucleotide modification template are each provided on separate plasmids or are provided on a single plasmid.

## Patentansprüche

1. Verfahren zum Editieren einer Nucleotidsequenz in dem Genom einer *Escherichia coli*-Zelle, wobei das Verfahren das Bereitstellen mindestens eines rekombinanten DNA-Konstrukts umfasst, umfassend eine DNA-Sequenz, codierend eine Lotsen-RNA und ein zirkuläres Polynucleotid-Modifikationstemplate für eine *E. coli*-Zelle, umfassend eine Cas9-Endonuclease DNA-Sequenz, funktionsfähig verknüpft mit einem induzierbaren Promotor, wobei die Cas9-Endonuclease DNA-Sequenz eine Cas9-Endonuclease codiert, wobei die Lotsen-RNA und die Cas9-Endonuclease einen Lotsen-RNA/Cas9-Endonuclease-Komplex (RGEN) bilden können, der in der Lage ist, Aufspaltung der Targetsequenz zu vermitteln, um homologe Rekombination vermitteltes Gen-Editieren an einer Zielstelle in dem Genom der *E. coli-Zelle* zu ermöglichen, wobei das Polynucleotid-Modifikationstemplate mindestens eine Nucleotidmodifikation der Targetsequenz umfasst.

2. Verfahren nach Anspruch 1, wobei die Targetsequenz in dem Genom einer *E. coli-*Zelle ausgewählt ist aus der Gruppe bestehend aus einer Promotorsequenz, einer Terminatorsequenz, einer regulatorischen Elementsequenz, einer codierenden Sequenz, einem Prophagen, einem Pseudogen und einem exogenen Gen.

3. Verfahren nach Anspruch 1 oder Anspruch 2, wobei das rekombinante DNA-Konstrukt, umfassend eine DNA-Sequenz, die eine Lotsen-RNA codiert, über ein zirkuläres Plasmid bereitgestellt wird.

4. Verfahren nach einem der Ansprüche 1 bis 3, wobei das rekombinante DNA-Konstrukt und das zirkuläre Polynucleotid-Modifikationstemplate jeweils auf separaten Plasmiden bereitgestellt werden oder auf einem Einzelplasmid bereitgestellt werden.

5. Verfahren nach einem der Ansprüche 1 bis 4, wobei das rekombinante DNA-Konstrukt und das zirkuläre Polynucleotid-Template über ein Mittel bereitgestellt werden, das ausgewählt ist aus der Gruppe bestehend aus Elektroporation, Wärmeschock, Phagen-Zuführung, Paarung, Konjugation und Transduktion.

6. Verfahren nach einem der Ansprüche 1 bis 5, wobei die Zielstelle von einer ersten genomischen Region und einer zweiten genomischen Region flankiert wird, wobei das zirkuläre Polynucleotid-Template ferner eine erste Region von Homologie zu der ersten genomischen Region und eine zweite Region von Homologie zu der zweiten genomischen Region umfasst.

7. Verfahren nach einem der Ansprüche 1 bis 6, wobei die *E. coli*-Zelle ein exogenes Rekombinase-Protein nicht exprimiert.

8. Verfahren nach einem der Ansprüche 1 bis 7, wobei die *E. coli*-Zelle ein Protein nicht exprimiert, das ausgewählt ist aus der Gruppe, umfassend ein RecET-Protein, ein Lambda-Rot-Protein und einen RecBCD-Inhibitor.

9. Verfahren nach einem der Ansprüche 1 bis 8, weiter umfassend Aufziehen von Nachkommenzellen von der *E. coli-*Zelle, wobei die Nachkommenzelle die mindestens eine Nucleotidmodifikation der Targetsequenz umfasst.

10. Verfahren nach einem der Ansprüche 1 bis 9, wobei sich die Zielstelle in einem *E*. *coli*-galK-Gen befindet.

11. Verfahren nach Anspruch 1, das ein Verfahren zum Erzeugen einer galK-Gen mutanten *E. coli*-Zelle ist, wobei das Verfahren umfasst:
a) Bereitstellen mindestens eines zirkulären rekombinanten DNA-Konstrukts, umfassend eine DNA-Sequenz, codierend eine Lotsen-RNA und mindestens ein zirkuläres Polynucleotid-Modifikationstemplate für eine *E. coli*-Zelle, umfassend eine Cas9-Endonuclease DNA-Sequenz, funktionsfähig verknüpft mit einem induzierbaren Promotor, wobei die Cas9-Endonuclease DNA-Sequenz eine Cas-Endonuclease codiert, die zum Einführen eines Doppelstrangbruchs an einer Zielstelle in einer *galK*-genomischen Sequenz in dem *E*. *coli*-Genom in der Lage ist, wobei das zirkuläre Polynucleotid-Modifikationstemplate mindestens eine Nucleotidmodifikation der *galK*-genomischen Sequenz umfasst;
b) Aufziehen von Nachkommenzellen aus der *E. coli*-Zelle von (a); und
c) Bewerten der Nachkommenzellen von (b) auf das Vorhandensein der mindestens einen Nucleotidmodifikation.

12. Verfahren nach Anspruch 1, das ein Verfahren zum Editieren einer Targetsequenz in dem Genom einer *Escherichia coli*-Zelle ist, wobei das Verfahren Bereitstellen mindestens eines ersten rekombinanten DNA-Konstrukts umfasst, umfassend eine DNA-Sequenz, codierend eine Lotsen-RNA, ein zirkuläres Polynucleotid-Modifikationstemplate, und ein zweites rekombinantes DNA-Konstrukt, umfassend eine DNA-Sequenz, codierend eine Cas9-Endonuclease, funktionsfähig mit einem induzierbaren Promotor verknüpft, an eine *E. coli*-Zelle, wobei die Cas9-Endonuclease einen Doppelstrangbruch an einer Zielstelle in dem Genom der *E. coli*-Zelle einführt, wobei das Polynucleotid-Modifikationstemplate mindestens eine Nucleotidmodifikation der Targetsequenz umfasst.

13. Verfahren nach Anspruch 12, wobei das erste rekombinante DNA-Konstrukt, das zweite rekombinante DNA-Konstrukt und das zirkuläre Polynucleotid-Modifikationstemplate jeweils auf separaten Plasmiden bereitgestellt werden oder auf einem Einzelplasmid bereitgestellt werden.

## Revendications

1. Procédé pour l'édition d'une séquence cible dans le génome d'une cellule d'*Escherichia coli*, le procédé comprenant la fourniture d'au moins une construction d'ADN recombinant comprenant une séquence d'ADN codant pour un ARN guide et d'une matrice de modification de polynucléotide circulaire à une cellule d'*E*. *coli* comprenant une séquence d'ADN d'endonucléase Cas9 liée de manière fonctionnelle à un promoteur inductible, dans lequel ladite séquence d'ADN d'endonucléase Cas9 code pour une endonucléase Cas9, dans lequel l'ARN guide et l'endonucléase Cas9 peuvent former un complexe d'ARN guide/endonucléase Cas9 (RGEN) qui est capable d'induire le clivage de la séquence cible pour permettre une édition de gène induite par une recombinaison homologue à un site cible dans le génome de ladite cellule d'*E*. *coli,* dans lequel ladite matrice de modification de polynucléotide comprend au moins une modification de nucléotide de ladite séquence cible.

2. Procédé selon la revendication 1, dans lequel la séquence cible dans le génome d'une cellule d'*E*. *coli* est choisie dans le groupe constitué d'une séquence de promoteur, d'une séquence de terminateur, d'une séquence d'élément régulateur, d'une séquence de codage, d'un prophage, d'un pseudogène et d'un gène exogène.

3. Procédé selon la revendication 1 ou la revendication 2, dans lequel ladite construction d'ADN recombinant comprenant une séquence d'ADN codant pour un ARN guide est fournie via un plasmide circulaire.

4. Procédé selon l'une quelconque des revendications 1 à 3, dans lequel la construction d'ADN recombinant et la matrice de modification de polynucléotide circulaire sont chacune fournies sur des plasmides séparés ou sont fournies sur un seul plasmide.

5. Procédé selon l'une quelconque des revendications 1 à 4, dans lequel la construction d'ADN recombinant et la matrice de modification de polynucléotide circulaire sont fournies via un moyen choisi dans le groupe constitué de : électroporation, choc thermique, délivrance de phage, croisement, conjugaison et transduction.

6. Procédé selon l'une quelconque des revendications 1 à 5, dans lequel ledit site cible est flanqué par une première région génomique et une deuxième région génomique, dans lequel la matrice de polynucléotide circulaire comprend en outre une première région d'homologie à ladite première région génomique et une deuxième région d'homologie à ladite deuxième région génomique.

7. Procédé selon l'une quelconque des revendications 1 à 6, dans lequel la cellule d'*E*. *coli* n'exprime pas une protéine de recombinase exogène.

8. Procédé selon l'une quelconque des revendications 1 à 7, dans lequel la cellule d'*E*. *coli* n'exprime pas une protéine choisie dans le groupe comprenant une protéine RecET, une protéine lambda-rouge et un inhibiteur de RecBCD.

9. Procédé selon l'une quelconque des revendications 1 à 8, comprenant en outre la croissance de cellules descendantes à partir de ladite cellule d'*E*. *coli,* dans lequel la cellule descendante comprend la au moins une modification de nucléotide de ladite séquence cible.

10. Procédé selon l'une quelconque des revendications 1 à 9, dans lequel le site cible est localisé dans un gène galK d'*E*. *coli.*

11. Procédé selon la revendication 1, qui est un procédé pour la production d'une cellule d'*E. coli* à galK mutant, le procédé comprenant :
a) la fourniture d'au moins une construction d'ADN recombinant circulaire comprenant une séquence d'ADN codant pour un ARN guide et d'au moins une matrice de modification de polynucléotide circulaire à une cellule d'*E*. *coli* comprenant une séquence d'ADN d'endonucléase Cas9 liée de manière fonctionnelle à un promoteur inductible, dans lequel ladite séquence d'ADN d'endonucléase Cas9 code pour une endonucléase Cas qui est capable d'introduire une cassure double brin à un site cible dans une séquence génomique *galK* dans le génome de l'*E*. *coli,* dans lequel ladite matrice de modification de polynucléotide circulaire comprend au moins une modification de nucléotide de ladite séquence génomique *galK* ;
b) la croissance de cellules descendantes à partir de la cellule d'*E*. *coli* de (a) ; et
c) l'évaluation des cellules descendantes de (b) sur la présence de ladite au moins une modification de nucléotide.

12. Procédé selon la revendication 1, qui est un procédé pour l'édition d'une séquence cible dans le génome d'une cellule d'*Escherichia coli,* le procédé comprenant la fourniture d'au moins une première construction d'ADN recombinant comprenant une séquence d'ADN codant pour un ARN guide, d'une matrice de modification de polynucléotide circulaire et d'une deuxième construction d'ADN recombinant comprenant une séquence d'ADN codant pour une endonucléase Cas9 liée de manière fonctionnelle à un promoteur inductible, à une cellule d'*E*. *coli,* dans lequel l'endonucléase Cas9 introduit une cassure double brin à un site cible dans le génome de ladite cellule d'*E*. *coli,* dans lequel ladite matrice de modification de polynucléotide comprend au moins une modification de nucléotide de ladite séquence cible.

13. Procédé selon la revendication 12, dans lequel la première construction d'ADN recombinant, la deuxième construction d'ADN recombinant et la matrice de modification de polynucléotide circulaire sont chacune fournies sur des plasmides séparés ou sont fournies sur un seul plasmide.


### SEQUENCE LISTING

<110> E. I. du Pont de Nemours and Company Frisch, Ryan L. Jackson, Ethel N.
<120> COMPOSITIONS AND METHODS FOR EFFICIENT GENE EDITING IN E. COLI USING GUIDE RNA/CAS ENDONUCLEASE SYSTEMS IN COMBINATION WITH CIRCULAR POLYNUCLEOTIDE MODIFICATION TEMPLATES.
<130> CL6256 PCT
<150> 62/092914
   <151> 2014-12-17
<160> 52
<170> PatentIn version 3.5
<210> 1
   <211> 4107
   <212> DNA
   <213> Streptococcus pyogenes
<400> 1
   atggataaga aatactcaat aggcttagat atcggcacaa atagcgtcgg atgggcggtg      60
   atcactgatg aatataaggt tccgtctaaa aagttcaagg ttctgggaaa tacagaccgc     120
   cacagtatca aaaaaaatct tataggggct cttttatttg acagtggaga gacagcggaa     180
   gcgactcgtc tcaaacggac agctcgtaga aggtatacac gtcggaagaa tcgtatttgt     240
   tatctacagg agattttttc aaatgagatg gcgaaagtag atgatagttt ctttcatcga     300
   cttgaagagt cttttttggt ggaagaagac aagaagcatg aacgtcatcc tatttttgga     360
   aatatagtag atgaagttgc ttatcatgag aaatatccaa ctatctatca tctgcgaaaa     420
   aaattggtag attctactga taaagcggat ttgcgcttaa tctatttggc cttagcgcat     480
   atgattaagt ttcgtggtca ttttttgatt gagggagatt taaatcctga taatagtgat     540
   gtggacaaac tatttatcca gttggtacaa acctacaatc aattatttga agaaaaccct     600
   attaacgcaa gtggagtaga tgctaaagcg attctttctg cacgattgag taaatcaaga     660
   cgattagaaa atctcattgc tcagctcccc ggtgagaaga aaaatggctt atttgggaat     720
   ctcattgctt tgtcattggg tttgacccct aattttaaat caaattttga tttggcagaa     780
   gatgctaaat tacagctttc aaaagatact tacgatgatg atttagataa tttattggcg     840
   caaattggag atcaatatgc tgatttgttt ttggcagcta agaatttatc agatgctatt     900
   ttactttcag atatcctaag agtaaatact gaaataacta aggctcccct atcagcttca     960
   atgattaaac gctacgatga acatcatcaa gacttgactc ttttaaaagc tttagttcga    1020
   caacaacttc cagaaaagta taaagaaatc ttttttgatc aatcaaaaaa cggatatgca    1080
   ggttatattg atgggggagc tagccaagaa gaattttata aatttatcaa accaatttta    1140
   gaaaaaatgg atggtactga ggaattattg gtgaaactaa atcgtgaaga tttgctgcgc    1200
   aagcaacgga cctttgacaa cggctctatt ccccatcaaa ttcacttggg tgagctgcat    1260
   gctattttga gaagacaaga agacttttat ccatttttaa aagacaatcg tgagaagatt    1320
   gaaaaaatct tgacttttcg aattccttat tatgttggtc cattggcgcg tggcaatagt    1380
   cgttttgcat ggatgactcg gaagtctgaa gaaacaatta ccccatggaa ttttgaagaa    1440
   gttgtcgata aaggtgcttc agctcaatca tttattgaac gcatgacaaa ctttgataaa    1500
   aatcttccaa atgaaaaagt actaccaaaa catagtttgc tttatgagta ttttacggtt    1560
   tataacgaat tgacaaaggt caaatatgtt actgaaggaa tgcgaaaacc agcatttctt    1620
   tcaggtgaac agaagaaagc cattgttgat ttactcttca aaacaaatcg aaaagtaacc    1680
   gttaagcaat taaaagaaga ttatttcaaa aaaatagaat gttttgatag tgttgaaatt    1740
   tcaggagttg aagatagatt taatgcttca ttaggtacct accatgattt gctaaaaatt    1800
   attaaagata aagatttttt ggataatgaa gaaaatgaag atatcttaga ggatattgtt    1860
   ttaacattga ccttatttga agatagggag atgattgagg aaagacttaa aacatatgct    1920
   cacctctttg atgataaggt gatgaaacag cttaaacgtc gccgttatac tggttgggga    1980
   cgtttgtctc gaaaattgat taatggtatt agggataagc aatctggcaa aacaatatta    2040
   gattttttga aatcagatgg ttttgccaat cgcaatttta tgcagctgat ccatgatgat    2100
   agtttgacat ttaaagaaga cattcaaaaa gcacaagtgt ctggacaagg cgatagttta    2160
   catgaacata ttgcaaattt agctggtagc cctgctatta aaaaaggtat tttacagact    2220
   gtaaaagttg ttgatgaatt ggtcaaagta atggggcggc ataagccaga aaatatcgtt    2280
   attgaaatgg cacgtgaaaa tcagacaact caaaagggcc agaaaaattc gcgagagcgt    2340
   atgaaacgaa tcgaagaagg tatcaaagaa ttaggaagtc agattcttaa agagcatcct    2400
   gttgaaaata ctcaattgca aaatgaaaag ctctatctct attatctcca aaatggaaga    2460
   gacatgtatg tggaccaaga attagatatt aatcgtttaa gtgattatga tgtcgatcac    2520
   attgttccac aaagtttcct taaagacgat tcaatagaca ataaggtctt aacgcgttct    2580
   gataaaaatc gtggtaaatc ggataacgtt ccaagtgaag aagtagtcaa aaagatgaaa    2640
   aactattgga gacaacttct aaacgccaag ttaatcactc aacgtaagtt tgataattta    2700
   acgaaagctg aacgtggagg tttgagtgaa cttgataaag ctggttttat caaacgccaa    2760
   ttggttgaaa ctcgccaaat cactaagcat gtggcacaaa ttttggatag tcgcatgaat    2820
   actaaatacg atgaaaatga taaacttatt cgagaggtta aagtgattac cttaaaatct    2880
   aaattagttt ctgacttccg aaaagatttc caattctata aagtacgtga gattaacaat    2940
   taccatcatg cccatgatgc gtatctaaat gccgtcgttg gaactgcttt gattaagaaa    3000
   tatccaaaac ttgaatcgga gtttgtctat ggtgattata aagtttatga tgttcgtaaa    3060
   atgattgcta agtctgagca agaaataggc aaagcaaccg caaaatattt cttttactct    3120
   aatatcatga acttcttcaa aacagaaatt acacttgcaa atggagagat tcgcaaacgc    3180
   cctctaatcg aaactaatgg ggaaactgga gaaattgtct gggataaagg gcgagatttt    3240
   gccacagtgc gcaaagtatt gtccatgccc caagtcaata ttgtcaagaa aacagaagta    3300
   cagacaggcg gattctccaa ggagtcaatt ttaccaaaaa gaaattcgga caagcttatt    3360
   gctcgtaaaa aagactggga tccaaaaaaa tatggtggtt ttgatagtcc aacggtagct    3420
   tattcagtcc tagtggttgc taaggtggaa aaagggaaat cgaagaagtt aaaatccgtt    3480
   aaagagttac tagggatcac aattatggaa agaagttcct ttgaaaaaaa tccgattgac    3540
   tttttagaag ctaaaggata taaggaagtt aaaaaagact taatcattaa actacctaaa    3600
   tatagtcttt ttgagttaga aaacggtcgt aaacggatgc tggctagtgc cggagaatta    3660
   caaaaaggaa atgagctggc tctgccaagc aaatatgtga attttttata tttagctagt    3720
   cattatgaaa agttgaaggg tagtccagaa gataacgaac aaaaacaatt gtttgtggag    3780
   cagcataagc attatttaga tgagattatt gagcaaatca gtgaattttc taagcgtgtt    3840
   attttagcag atgccaattt agataaagtt cttagtgcat ataacaaaca tagagacaaa    3900
   ccaatacgtg aacaagcaga aaatattatt catttattta cgttgacgaa tcttggagct    3960
   cccgctgctt ttaaatattt tgatacaaca attgatcgta aacgatatac gtctacaaaa    4020
   gaagttttag atgccactct tatccatcaa tccatcactg gtctttatga aacacgcatt    4080
   gatttgagtc agctaggagg tgactga                                        4107
<210> 2
   <211> 4140
   <212> DNA
   <213> Artificial sequence
<220>
   <223> Yarrowia optimized Cas9
<400> 2
   atggacaaga aatactccat cggcctggac attggaacca actctgtcgg ctgggctgtc      60
   atcaccgacg agtacaaggt gccctccaag aaattcaagg tcctcggaaa caccgatcga     120
   cactccatca agaaaaacct cattggtgcc ctgttgttcg attctggcga gactgccgaa     180
   gctaccagac tcaagcgaac tgctcggcga cgttacaccc gacggaagaa ccgaatctgc     240
   tacctgcagg agatcttttc caacgagatg gccaaggtgg acgattcgtt ctttcatcga     300
   ctggaggaat ccttcctcgt cgaggaagac aagaaacacg agcgtcatcc catctttggc     360
   aacattgtgg acgaggttgc ttaccacgag aagtatccta ccatctacca cctgcgaaag     420
   aaactcgtcg attccaccga caaggcggat ctcagactta tctacctcgc tctggcacac     480
   atgatcaagt ttcgaggtca tttcctcatc gagggcgatc tcaatcccga caacagcgat     540
   gtggacaagc tgttcattca gctcgttcag acctacaacc agctgttcga ggaaaacccc     600
   atcaatgcct ccggagtcga tgcaaaggcc atcttgtctg ctcgactctc gaagagcaga     660
   cgactggaga acctcattgc ccaacttcct ggcgagaaaa agaacggact gtttggcaac     720
   ctcattgccc tttctcttgg tctcacaccc aacttcaagt ccaacttcga tctggcggag     780
   gacgccaagc tccagctgtc caaggacacc tacgacgatg acctcgacaa cctgcttgca     840
   cagattggcg atcagtacgc cgacctgttt ctcgctgcca agaacctttc ggatgctatt     900
   ctcttgtctg acattctgcg agtcaacacc gagatcacaa aggctcccct ttctgcctcc     960
   atgatcaagc gatacgacga gcaccatcag gatctcacac tgctcaaggc tcttgtccga    1020
   cagcaactgc ccgagaagta caaggagatc tttttcgatc agtcgaagaa cggctacgct    1080
   ggatacatcg acggcggagc ctctcaggaa gagttctaca agttcatcaa gccaattctc    1140
   gagaagatgg acggaaccga ggaactgctt gtcaagctca atcgagagga tctgcttcgg    1200
   aagcaacgaa ccttcgacaa cggcagcatt cctcatcaga tccacctcgg tgagctgcac    1260
   gccattcttc gacgtcagga agacttctac ccctttctca aggacaaccg agagaagatc    1320
   gagaagattc ttacctttcg aatcccctac tatgttggtc ctcttgccag aggaaactct      1380
   cgatttgctt ggatgactcg aaagtccgag gaaaccatca ctccctggaa cttcgaggaa      1440
   gtcgtggaca agggtgcctc tgcacagtcc ttcatcgagc gaatgaccaa cttcgacaag      1500
   aatctgccca acgagaaggt tcttcccaag cattcgctgc tctacgagta ctttacagtc      1560
   tacaacgaac tcaccaaagt caagtacgtt accgagggaa tgcgaaagcc tgccttcttg      1620
   tctggcgaac agaagaaagc cattgtcgat ctcctgttca agaccaaccg aaaggtcact      1680
   gttaagcagc tcaaggagga ctacttcaag aaaatcgagt gtttcgacag cgtcgagatt      1740
   tccggagttg aggaccgatt caacgcctct ttgggcacct atcacgatct gctcaagatt      1800
   atcaaggaca aggattttct cgacaacgag gaaaacgagg acattctgga ggacatcgtg      1860
   ctcactctta ccctgttcga agatcgggag atgatcgagg aacgactcaa gacatacgct      1920
   cacctgttcg acgacaaggt catgaaacaa ctcaagcgac gtagatacac cggctgggga      1980
   agactttcgc gaaagctcat caacggcatc agagacaagc agtccggaaa gaccattctg      2040
   gactttctca agtccgatgg ctttgccaac cgaaacttca tgcagctcat tcacgacgat      2100
   tctcttacct tcaaggagga catccagaag gcacaagtgt ccggtcaggg cgacagcttg      2160
   cacgaacata ttgccaacct ggctggttcg ccagccatca agaaaggcat tctccagact      2220
   gtcaaggttg tcgacgagct ggtgaaggtc atgggacgtc acaagcccga gaacattgtg      2280
   atcgagatgg ccagagagaa ccagacaact caaaagggtc agaaaaactc gcgagagcgg      2340
   atgaagcgaa tcgaggaagg catcaaggag ctgggatccc agattctcaa ggagcatccc      2400
   gtcgagaaca ctcaactgca gaacgagaag ctgtatctct actatctgca gaatggtcga      2460
   gacatgtacg tggatcagga actggacatc aatcgtctca gcgactacga tgtggaccac      2520
   attgtccctc aatcctttct caaggacgat tctatcgaca acaaggtcct tacacgatcc      2580
   gacaagaaca gaggcaagtc ggacaacgtt cccagcgaag aggtggtcaa aaagatgaag      2640
   aactactggc gacagctgct caacgccaag ctcattaccc agcgaaagtt cgacaatctt      2700
   accaaggccg agcgaggcgg tctgtccgag ctcgacaagg ctggcttcat caagcgtcaa      2760
   ctcgtcgaga ccagacagat cacaaagcac gtcgcacaga ttctcgattc tcggatgaac      2820
   accaagtacg acgagaacga caagctcatc cgagaggtca aggtgattac tctcaagtcc      2880
   aaactggtct ccgatttccg aaaggacttt cagttctaca aggtgcgaga gatcaacaat      2940
   taccaccatg cccacgatgc ttacctcaac gccgtcgttg gcactgcgct catcaagaaa      3000
   taccccaagc tcgaaagcga gttcgtttac ggcgattaca aggtctacga cgttcgaaag      3060
   atgattgcca agtccgaaca ggagattggc aaggctactg ccaagtactt cttttactcc      3120
   aacatcatga actttttcaa gaccgagatc accttggcca acggagagat tcgaaagaga      3180
   ccacttatcg agaccaacgg cgaaactgga gagatcgtgt gggacaaggg tcgagacttt      3240
   gcaaccgtgc gaaaggttct gtcgatgcct caggtcaaca tcgtcaagaa aaccgaggtt      3300
   cagactggcg gattctccaa ggagtcgatt ctgcccaagc gaaactccga caagctcatc      3360
   gctcgaaaga aagactggga tcccaagaaa tacggtggct tcgattctcc taccgtcgcc      3420
   tattccgtgc ttgtcgttgc gaaggtcgag aagggcaagt ccaaaaagct caagtccgtc      3480
   aaggagctgc tcggaattac catcatggag cgatcgagct tcgagaagaa tcccatcgac      3540
   ttcttggaag ccaagggtta caaggaggtc aagaaagacc tcattatcaa gctgcccaag      3600
   tactctctgt tcgaactgga gaacggtcga aagcgtatgc tcgcctccgc tggcgagctg      3660
   cagaagggaa acgagcttgc cttgccttcg aagtacgtca actttctcta tctggcttct      3720
   cactacgaga agctcaaggg ttctcccgag gacaacgaac agaagcaact cttcgttgag      3780
   cagcacaaac attacctcga cgagattatc gagcagattt ccgagttttc gaagcgagtc      3840
   atcctggctg atgccaactt ggacaaggtg ctctctgcct acaacaagca tcgggacaaa      3900
   cccattcgag aacaggcgga gaacatcatt cacctgttta ctcttaccaa cctgggtgct      3960
   cctgcagctt tcaagtactt cgataccact atcgaccgaa agcggtacac atccaccaag      4020
   gaggttctcg atgccaccct gattcaccag tccatcactg gcctgtacga gacccgaatc      4080
   gacctgtctc agcttggtgg cgactccaga gccgatccca agaaaaagcg aaaggtctaa      4140
<210> 3
   <211> 9
   <212> PRT
   <213> Simian virus 40
<400> 3
   Met Ala Pro Lys Lys Lys Arg Lys Val
   1               5
<210> 4
   <211> 546
   <212> DNA
   <213> Yarrowia lipolytica
<400> 4
   tcgacgttta aaccatcatc taagggcctc aaaactacct cggaactgct gcgctgatct      60
   ggacaccaca gaggttccga gcactttagg ttgcaccaaa tgtcccacca ggtgcaggca     120
   gaaaacgctg gaacagcgtg tacagtttgt cttaacaaaa agtgagggcg ctgaggtcga     180
   gcagggtggt gtgacttgtt atagccttta gagctgcgaa agcgcgtatg gatttggctc     240
   atcaggccag attgagggtc tgtggacaca tgtcatgtta gtgtacttca atcgccccct     300
   ggatatagcc ccgacaatag gccgtggcct catttttttg ccttccgcac atttccattg     360
   ctcggtaccc acaccttgct tctcctgcac ttgccaacct taatactggt ttacattgac     420
   caacatctta caagcggggg gcttgtctag ggtatatata aacagtggct ctcccaatcg     480
   gttgccagtc tcttttttcc tttctttccc cacagattcg aaatctaaac tacacatcac     540
   accatg                                                                546
<210> 5
   <211> 4683
   <212> DNA
   <213> artificial sequence
<220>
   <223> Yarrowia optimized Cas9 expression cassette
<400> 5
<210> 6
   <211> 10706
   <212> DNA
   <213> artificial sequence
<220>
   <223> pZufCas9CS
<400> 6
   catggacaag aaatactcca tcggcctgga cattggaacc aactctgtcg gctgggctgt      60
   catcaccgac gagtacaagg tgccctccaa gaaattcaag gtcctcggaa acaccgatcg     120
   acactccatc aagaaaaacc tcattggtgc cctgttgttc gattctggcg agactgccga     180
   agctaccaga ctcaagcgaa ctgctcggcg acgttacacc cgacggaaga accgaatctg     240
   ctacctgcag gagatctttt ccaacgagat ggccaaggtg gacgattcgt tctttcatcg     300
   actggaggaa tccttcctcg tcgaggaaga caagaaacac gagcgtcatc ccatctttgg     360
   caacattgtg gacgaggttg cttaccacga gaagtatcct accatctacc acctgcgaaa     420
   gaaactcgtc gattccaccg acaaggcgga tctcagactt atctacctcg ctctggcaca     480
   catgatcaag tttcgaggtc atttcctcat cgagggcgat ctcaatcccg acaacagcga     540
   tgtggacaag ctgttcattc agctcgttca gacctacaac cagctgttcg aggaaaaccc     600
   catcaatgcc tccggagtcg atgcaaaggc catcttgtct gctcgactct cgaagagcag     660
   acgactggag aacctcattg cccaacttcc tggcgagaaa aagaacggac tgtttggcaa     720
   cctcattgcc ctttctcttg gtctcacacc caacttcaag tccaacttcg atctggcgga     780
   ggacgccaag ctccagctgt ccaaggacac ctacgacgat gacctcgaca acctgcttgc     840
   acagattggc gatcagtacg ccgacctgtt tctcgctgcc aagaaccttt cggatgctat     900
   tctcttgtct gacattctgc gagtcaacac cgagatcaca aaggctcccc tttctgcctc     960
   catgatcaag cgatacgacg agcaccatca ggatctcaca ctgctcaagg ctcttgtccg    1020
   acagcaactg cccgagaagt acaaggagat ctttttcgat cagtcgaaga acggctacgc 1080
   tggatacatc gacggcggag cctctcagga agagttctac aagttcatca agccaattct 1140
   cgagaagatg gacggaaccg aggaactgct tgtcaagctc aatcgagagg atctgcttcg 1200
   gaagcaacga accttcgaca acggcagcat tcctcatcag atccacctcg gtgagctgca 1260
   cgccattctt cgacgtcagg aagacttcta cccctttctc aaggacaacc gagagaagat 1320
   cgagaagatt cttacctttc gaatccccta ctatgttggt cctcttgcca gaggaaactc 1380
   tcgatttgct tggatgactc gaaagtccga ggaaaccatc actccctgga acttcgagga 1440
   agtcgtggac aagggtgcct ctgcacagtc cttcatcgag cgaatgacca acttcgacaa 1500
   gaatctgccc aacgagaagg ttcttcccaa gcattcgctg ctctacgagt actttacagt 1560
   ctacaacgaa ctcaccaaag tcaagtacgt taccgaggga atgcgaaagc ctgccttctt 1620
   gtctggcgaa cagaagaaag ccattgtcga tctcctgttc aagaccaacc gaaaggtcac 1680
   tgttaagcag ctcaaggagg actacttcaa gaaaatcgag tgtttcgaca gcgtcgagat 1740
   ttccggagtt gaggaccgat tcaacgcctc tttgggcacc tatcacgatc tgctcaagat 1800
   tatcaaggac aaggattttc tcgacaacga ggaaaacgag gacattctgg aggacatcgt 1860
   gctcactctt accctgttcg aagatcggga gatgatcgag gaacgactca agacatacgc 1920
   tcacctgttc gacgacaagg tcatgaaaca actcaagcga cgtagataca ccggctgggg 1980
   aagactttcg cgaaagctca tcaacggcat cagagacaag cagtccggaa agaccattct 2040
   ggactttctc aagtccgatg gctttgccaa ccgaaacttc atgcagctca ttcacgacga 2100
   ttctcttacc ttcaaggagg acatccagaa ggcacaagtg tccggtcagg gcgacagctt 2160
   gcacgaacat attgccaacc tggctggttc gccagccatc aagaaaggca ttctccagac 2220
   tgtcaaggtt gtcgacgagc tggtgaaggt catgggacgt cacaagcccg agaacattgt 2280
   gatcgagatg gccagagaga accagacaac tcaaaagggt cagaaaaact cgcgagagcg 2340
   gatgaagcga atcgaggaag gcatcaagga gctgggatcc cagattctca aggagcatcc 2400
   cgtcgagaac actcaactgc agaacgagaa gctgtatctc tactatctgc agaatggtcg 2460
   agacatgtac gtggatcagg aactggacat caatcgtctc agcgactacg atgtggacca 2520
   cattgtccct caatcctttc tcaaggacga ttctatcgac aacaaggtcc ttacacgatc 2580
   cgacaagaac agaggcaagt cggacaacgt tcccagcgaa gaggtggtca aaaagatgaa 2640
   gaactactgg cgacagctgc tcaacgccaa gctcattacc cagcgaaagt tcgacaatct 2700
   taccaaggcc gagcgaggcg gtctgtccga gctcgacaag gctggcttca tcaagcgtca 2760
   actcgtcgag accagacaga tcacaaagca cgtcgcacag attctcgatt ctcggatgaa 2820
   caccaagtac gacgagaacg acaagctcat ccgagaggtc aaggtgatta ctctcaagtc 2880
   caaactggtc tccgatttcc gaaaggactt tcagttctac aaggtgcgag agatcaacaa 2940
   ttaccaccat gcccacgatg cttacctcaa cgccgtcgtt ggcactgcgc tcatcaagaa 3000
   ataccccaag ctcgaaagcg agttcgttta cggcgattac aaggtctacg acgttcgaaa 3060
   gatgattgcc aagtccgaac aggagattgg caaggctact gccaagtact tcttttactc 3120
   caacatcatg aactttttca agaccgagat caccttggcc aacggagaga ttcgaaagag 3180
   accacttatc gagaccaacg gcgaaactgg agagatcgtg tgggacaagg gtcgagactt 3240
   tgcaaccgtg cgaaaggttc tgtcgatgcc tcaggtcaac atcgtcaaga aaaccgaggt 3300
   tcagactggc ggattctcca aggagtcgat tctgcccaag cgaaactccg acaagctcat 3360
   cgctcgaaag aaagactggg atcccaagaa atacggtggc ttcgattctc ctaccgtcgc 3420
   ctattccgtg cttgtcgttg cgaaggtcga gaagggcaag tccaaaaagc tcaagtccgt 3480
   caaggagctg ctcggaatta ccatcatgga gcgatcgagc ttcgagaaga atcccatcga 3540
   cttcttggaa gccaagggtt acaaggaggt caagaaagac ctcattatca agctgcccaa 3600
   gtactctctg ttcgaactgg agaacggtcg aaagcgtatg ctcgcctccg ctggcgagct 3660
   gcagaaggga aacgagcttg ccttgccttc gaagtacgtc aactttctct atctggcttc 3720
   tcactacgag aagctcaagg gttctcccga ggacaacgaa cagaagcaac tcttcgttga 3780
   gcagcacaaa cattacctcg acgagattat cgagcagatt tccgagtttt cgaagcgagt 3840
   catcctggct gatgccaact tggacaaggt gctctctgcc tacaacaagc atcgggacaa 3900
   acccattcga gaacaggcgg agaacatcat tcacctgttt actcttacca acctgggtgc 3960
   tcctgcagct ttcaagtact tcgataccac tatcgaccga aagcggtaca catccaccaa 4020
   ggaggttctc gatgccaccc tgattcacca gtccatcact ggcctgtacg agacccgaat 4080
   cgacctgtct cagcttggtg gcgactccag agccgatccc aagaaaaagc gaaaggtcta 4140
   agcggccgca agtgtggatg gggaagtgag tgcccggttc tgtgtgcaca attggcaatc 4200
   caagatggat ggattcaaca cagggatata gcgagctacg tggtggtgcg aggatatagc 4260
   aacggatatt tatgtttgac acttgagaat gtacgataca agcactgtcc aagtacaata 4320
   ctaaacatac tgtacatact catactcgta cccgggcaac ggtttcactt gagtgcagtg 4380
   gctagtgctc ttactcgtac agtgtgcaat actgcgtatc atagtctttg atgtatatcg 4440
   tattcattca tgttagttgc gtacgagccg gaagcataaa gtgtaaagcc tggggtgcct 4500
   aatgagtgag ctaactcaca ttaattgcgt tgcgctcact gcccgctttc cagtcgggaa 4560
   acctgtcgtg ccagctgcat taatgaatcg gccaacgcgc ggggagaggc ggtttgcgta 4620
   ttgggcgctc ttccgcttcc tcgctcactg actcgctgcg ctcggtcgtt cggctgcggc 4680
   gagcggtatc agctcactca aaggcggtaa tacggttatc cacagaatca ggggataacg 4740
   caggaaagaa catgtgagca aaaggccagc aaaaggccag gaaccgtaaa aaggccgcgt 4800
   tgctggcgtt tttccatagg ctccgccccc ctgacgagca tcacaaaaat cgacgctcaa     4860
   gtcagaggtg gcgaaacccg acaggactat aaagatacca ggcgtttccc cctggaagct     4920
   ccctcgtgcg ctctcctgtt ccgaccctgc cgcttaccgg atacctgtcc gcctttctcc     4980
   cttcgggaag cgtggcgctt tctcatagct cacgctgtag gtatctcagt tcggtgtagg     5040
   tcgttcgctc caagctgggc tgtgtgcacg aaccccccgt tcagcccgac cgctgcgcct     5100
   tatccggtaa ctatcgtctt gagtccaacc cggtaagaca cgacttatcg ccactggcag     5160
   cagccactgg taacaggatt agcagagcga ggtatgtagg cggtgctaca gagttcttga     5220
   agtggtggcc taactacggc tacactagaa ggacagtatt tggtatctgc gctctgctga     5280
   agccagttac cttcggaaaa agagttggta gctcttgatc cggcaaacaa accaccgctg     5340
   gtagcggtgg tttttttgtt tgcaagcagc agattacgcg cagaaaaaaa ggatctcaag     5400
   aagatccttt gatcttttct acggggtctg acgctcagtg gaacgaaaac tcacgttaag     5460
   ggattttggt catgagatta tcaaaaagga tcttcaccta gatcctttta aattaaaaat     5520
   gaagttttaa atcaatctaa agtatatatg agtaaacttg gtctgacagt taccaatgct     5580
   taatcagtga ggcacctatc tcagcgatct gtctatttcg ttcatccata gttgcctgac     5640
   tccccgtcgt gtagataact acgatacggg agggcttacc atctggcccc agtgctgcaa     5700
   tgataccgcg agacccacgc tcaccggctc cagatttatc agcaataaac cagccagccg     5760
   gaagggccga gcgcagaagt ggtcctgcaa ctttatccgc ctccatccag tctattaatt     5820
   gttgccggga agctagagta agtagttcgc cagttaatag tttgcgcaac gttgttgcca     5880
   ttgctacagg catcgtggtg tcacgctcgt cgtttggtat ggcttcattc agctccggtt     5940
   cccaacgatc aaggcgagtt acatgatccc ccatgttgtg caaaaaagcg gttagctcct     6000
   tcggtcctcc gatcgttgtc agaagtaagt tggccgcagt gttatcactc atggttatgg     6060
   cagcactgca taattctctt actgtcatgc catccgtaag atgcttttct gtgactggtg     6120
   agtactcaac caagtcattc tgagaatagt gtatgcggcg accgagttgc tcttgcccgg     6180
   cgtcaatacg ggataatacc gcgccacata gcagaacttt aaaagtgctc atcattggaa     6240
   aacgttcttc ggggcgaaaa ctctcaagga tcttaccgct gttgagatcc agttcgatgt     6300
   aacccactcg tgcacccaac tgatcttcag catcttttac tttcaccagc gtttctgggt     6360
   gagcaaaaac aggaaggcaa aatgccgcaa aaaagggaat aagggcgaca cggaaatgtt     6420
   gaatactcat actcttcctt tttcaatatt attgaagcat ttatcagggt tattgtctca     6480
   tgagcggata catatttgaa tgtatttaga aaaataaaca aataggggtt ccgcgcacat     6540
   ttccccgaaa agtgccacct gacgcgccct gtagcggcgc attaagcgcg gcgggtgtgg     6600
   tggttacgcg cagcgtgacc gctacacttg ccagcgccct agcgcccgct cctttcgctt     6660
   tcttcccttc ctttctcgcc acgttcgccg gctttccccg tcaagctcta aatcgggggc     6720
   tccctttagg gttccgattt agtgctttac ggcacctcga ccccaaaaaa cttgattagg     6780
   gtgatggttc acgtagtggg ccatcgccct gatagacggt ttttcgccct ttgacgttgg     6840
   agtccacgtt ctttaatagt ggactcttgt tccaaactgg aacaacactc aaccctatct     6900
   cggtctattc ttttgattta taagggattt tgccgatttc ggcctattgg ttaaaaaatg     6960
   agctgattta acaaaaattt aacgcgaatt ttaacaaaat attaacgctt acaatttcca     7020
   ttcgccattc aggctgcgca actgttggga agggcgatcg gtgcgggcct cttcgctatt     7080
   acgccagctg gcgaaagggg gatgtgctgc aaggcgatta agttgggtaa cgccagggtt     7140
   ttcccagtca cgacgttgta aaacgacggc cagtgaattg taatacgact cactataggg     7200
   cgaattgggt accgggcccc ccctcgaggt cgatggtgtc gataagcttg atatcgaatt     7260
   catgtcacac aaaccgatct tcgcctcaag gaaacctaat tctacatccg agagactgcc     7320
   gagatccagt ctacactgat taattttcgg gccaataatt taaaaaaatc gtgttatata     7380
   atattatatg tattatatat atacatcatg atgatactga cagtcatgtc ccattgctaa     7440
   atagacagac tccatctgcc gcctccaact gatgttctca atatttaagg ggtcatctcg     7500
   cattgtttaa taataaacag actccatcta ccgcctccaa atgatgttct caaaatatat     7560
   tgtatgaact tatttttatt acttagtatt attagacaac ttacttgctt tatgaaaaac     7620
   acttcctatt taggaaacaa tttataatgg cagttcgttc atttaacaat ttatgtagaa     7680
   taaatgttat aaatgcgtat gggaaatctt aaatatggat agcataaatg atatctgcat     7740
   tgcctaattc gaaatcaaca gcaacgaaaa aaatcccttg tacaacataa atagtcatcg     7800
   agaaatatca actatcaaag aacagctatt cacacgttac tattgagatt attattggac     7860
   gagaatcaca cactcaactg tctttctctc ttctagaaat acaggtacaa gtatgtacta     7920
   ttctcattgt tcatacttct agtcatttca tcccacatat tccttggatt tctctccaat     7980
   gaatgacatt ctatcttgca aattcaacaa ttataataag atataccaaa gtagcggtat     8040
   agtggcaatc aaaaagcttc tctggtgtgc ttctcgtatt tatttttatt ctaatgatcc     8100
   attaaaggta tatatttatt tcttgttata taatcctttt gtttattaca tgggctggat     8160
   acataaaggt attttgattt aattttttgc ttaaattcaa tcccccctcg ttcagtgtca     8220
   actgtaatgg taggaaatta ccatactttt gaagaagcaa aaaaaatgaa agaaaaaaaa     8280
   aatcgtattt ccaggttaga cgttccgcag aatctagaat gcggtatgcg gtacattgtt     8340
   cttcgaacgt aaaagttgcg ctccctgaga tattgtacat ttttgctttt acaagtacaa     8400
   gtacatcgta caactatgta ctactgttga tgcatccaca acagtttgtt ttgttttttt     8460
   ttgttttttt tttttctaat gattcattac cgctatgtat acctacttgt acttgtagta     8520
   agccgggtta ttggcgttca attaatcata gacttatgaa tctgcacggt gtgcgctgcg     8580
   agttactttt agcttatgca tgctacttgg gtgtaatatt gggatctgtt cggaaatcaa      8640
   cggatgctca atcgatttcg acagtaatta attaagtcat acacaagtca gctttcttcg      8700
   agcctcatat aagtataagt agttcaacgt attagcactg tacccagcat ctccgtatcg      8760
   agaaacacaa caacatgccc cattggacag atcatgcgga tacacaggtt gtgcagtatc      8820
   atacatactc gatcagacag gtcgtctgac catcatacaa gctgaacaag cgctccatac      8880
   ttgcacgctc tctatataca cagttaaatt acatatccat agtctaacct ctaacagtta      8940
   atcttctggt aagcctccca gccagccttc tggtatcgct tggcctcctc aataggatct      9000
   cggttctggc cgtacagacc tcggccgaca attatgatat ccgttccggt agacatgaca      9060
   tcctcaacag ttcggtactg ctgtccgaga gcgtctccct tgtcgtcaag acccaccccg      9120
   ggggtcagaa taagccagtc ctcagagtcg cccttaggtc ggttctgggc aatgaagcca     9180
   accacaaact cggggtcgga tcgggcaagc tcaatggtct gcttggagta ctcgccagtg      9240
   gccagagagc ccttgcaaga cagctcggcc agcatgagca gacctctggc cagcttctcg      9300
   ttgggagagg ggactaggaa ctccttgtac tgggagttct cgtagtcaga gacgtcctcc      9360
   ttcttctgtt cagagacagt ttcctcggca ccagctcgca ggccagcaat gattccggtt      9420
   ccgggtacac cgtgggcgtt ggtgatatcg gaccactcgg cgattcggtg acaccggtac      9480
   tggtgcttga cagtgttgcc aatatctgcg aactttctgt cctcgaacag gaagaaaccg      9540
   tgcttaagag caagttcctt gagggggagc acagtgccgg cgtaggtgaa gtcgtcaatg     9600
   atgtcgatat gggttttgat catgcacaca taaggtccga ccttatcggc aagctcaatg      9660
   agctccttgg tggtggtaac atccagagaa gcacacaggt tggttttctt ggctgccacg      9720
   agcttgagca ctcgagcggc aaaggcggac ttgtggacgt tagctcgagc ttcgtaggag      9780
   ggcattttgg tggtgaagag gagactgaaa taaatttagt ctgcagaact ttttatcgga      9840
   accttatctg gggcagtgaa gtatatgtta tggtaatagt tacgagttag ttgaacttat      9900
   agatagactg gactatacgg ctatcggtcc aaattagaaa gaacgtcaat ggctctctgg      9960
   gcgtcgcctt tgccgacaaa aatgtgatca tgatgaaagc cagcaatgac gttgcagctg     10020
   atattgttgt cggccaaccg cgccgaaaac gcagctgtca gacccacagc ctccaacgaa     10080
   gaatgtatcg tcaaagtgat ccaagcacac tcatagttgg agtcgtactc caaaggcggc     10140
   aatgacgagt cagacagata ctcgtcgacg tttaaaccat catctaaggg cctcaaaact     10200
   acctcggaac tgctgcgctg atctggacac cacagaggtt ccgagcactt taggttgcac     10260
   caaatgtccc accaggtgca ggcagaaaac gctggaacag cgtgtacagt ttgtcttaac     10320
   aaaaagtgag ggcgctgagg tcgagcaggg tggtgtgact tgttatagcc tttagagctg     10380
   cgaaagcgcg tatggatttg gctcatcagg ccagattgag ggtctgtgga cacatgtcat     10440
   gttagtgtac ttcaatcgcc ccctggatat agccccgaca ataggccgtg gcctcatttt     10500
   tttgccttcc gcacatttcc attgctcggt acccacacct tgcttctcct gcacttgcca     10560
   accttaatac tggtttacat tgaccaacat cttacaagcg gggggcttgt ctagggtata     10620
   tataaacagt ggctctccca atcggttgcc agtctctttt ttcctttctt tccccacaga     10680
   ttcgaaatct aaactacaca tcacac                                          10706
<210> 7
   <211> 4144
   <212> DNA
   <213> artificial sequence
<220>
   <223> Cas9-SV40 fusion
<400> 7
   acaagaaata ctccatcggc ctggacattg gaaccaactc tgtcggctgg gctgtcatca     60
   ccgacgagta caaggtgccc tccaagaaat tcaaggtcct cggaaacacc gatcgacact    120
   ccatcaagaa aaacctcatt ggtgccctgt tgttcgattc tggcgagact gccgaagcta    180
   ccagactcaa gcgaactgct cggcgacgtt acacccgacg gaagaaccga atctgctacc    240
   tgcaggagat cttttccaac gagatggcca aggtggacga ttcgttcttt catcgactgg    300
   aggaatcctt cctcgtcgag gaagacaaga aacacgagcg tcatcccatc tttggcaaca    360
   ttgtggacga ggttgcttac cacgagaagt atcctaccat ctaccacctg cgaaagaaac    420
   tcgtcgattc caccgacaag gcggatctca gacttatcta cctcgctctg gcacacatga    480
   tcaagtttcg aggtcatttc ctcatcgagg gcgatctcaa tcccgacaac agcgatgtgg    540
   acaagctgtt cattcagctc gttcagacct acaaccagct gttcgaggaa aaccccatca    600
   atgcctccgg agtcgatgca aaggccatct tgtctgctcg actctcgaag agcagacgac    660
   tggagaacct cattgcccaa cttcctggcg agaaaaagaa cggactgttt ggcaacctca    720
   ttgccctttc tcttggtctc acacccaact tcaagtccaa cttcgatctg gcggaggacg    780
   ccaagctcca gctgtccaag gacacctacg acgatgacct cgacaacctg cttgcacaga    840
   ttggcgatca gtacgccgac ctgtttctcg ctgccaagaa cctttcggat gctattctct    900
   tgtctgacat tctgcgagtc aacaccgaga tcacaaaggc tcccctttct gcctccatga    960
   tcaagcgata cgacgagcac catcaggatc tcacactgct caaggctctt gtccgacagc   1020
   aactgcccga gaagtacaag gagatctttt tcgatcagtc gaagaacggc tacgctggat      1080
   acatcgacgg cggagcctct caggaagagt tctacaagtt catcaagcca attctcgaga      1140
   agatggacgg aaccgaggaa ctgcttgtca agctcaatcg agaggatctg cttcggaagc      1200
   aacgaacctt cgacaacggc agcattcctc atcagatcca cctcggtgag ctgcacgcca      1260
   ttcttcgacg tcaggaagac ttctacccct ttctcaagga caaccgagag aagatcgaga      1320
   agattcttac ctttcgaatc ccctactatg ttggtcctct tgccagagga aactctcgat      1380
   ttgcttggat gactcgaaag tccgaggaaa ccatcactcc ctggaacttc gaggaagtcg      1440
   tggacaaggg tgcctctgca cagtccttca tcgagcgaat gaccaacttc gacaagaatc      1500
   tgcccaacga gaaggttctt cccaagcatt cgctgctcta cgagtacttt acagtctaca      1560
   acgaactcac caaagtcaag tacgttaccg agggaatgcg aaagcctgcc ttcttgtctg      1620
   gcgaacagaa gaaagccatt gtcgatctcc tgttcaagac caaccgaaag gtcactgtta      1680
   agcagctcaa ggaggactac ttcaagaaaa tcgagtgttt cgacagcgtc gagatttccg      1740
   gagttgagga ccgattcaac gcctctttgg gcacctatca cgatctgctc aagattatca      1800
   aggacaagga ttttctcgac aacgaggaaa acgaggacat tctggaggac atcgtgctca      1860
   ctcttaccct gttcgaagat cgggagatga tcgaggaacg actcaagaca tacgctcacc      1920
   tgttcgacga caaggtcatg aaacaactca agcgacgtag atacaccggc tggggaagac      1980
   tttcgcgaaa gctcatcaac ggcatcagag acaagcagtc cggaaagacc attctggact      2040
   ttctcaagtc cgatggcttt gccaaccgaa acttcatgca gctcattcac gacgattctc      2100
   ttaccttcaa ggaggacatc cagaaggcac aagtgtccgg tcagggcgac agcttgcacg      2160
   aacatattgc caacctggct ggttcgccag ccatcaagaa aggcattctc cagactgtca      2220
   aggttgtcga cgagctggtg aaggtcatgg gacgtcacaa gcccgagaac attgtgatcg      2280
   agatggccag agagaaccag acaactcaaa agggtcagaa aaactcgcga gagcggatga      2340
   agcgaatcga ggaaggcatc aaggagctgg gatcccagat tctcaaggag catcccgtcg      2400
   agaacactca actgcagaac gagaagctgt atctctacta tctgcagaat ggtcgagaca      2460
   tgtacgtgga tcaggaactg gacatcaatc gtctcagcga ctacgatgtg gaccacattg      2520
   tccctcaatc ctttctcaag gacgattcta tcgacaacaa ggtccttaca cgatccgaca      2580
   agaacagagg caagtcggac aacgttccca gcgaagaggt ggtcaaaaag atgaagaact      2640
   actggcgaca gctgctcaac gccaagctca ttacccagcg aaagttcgac aatcttacca      2700
   aggccgagcg aggcggtctg tccgagctcg acaaggctgg cttcatcaag cgtcaactcg      2760
   tcgagaccag acagatcaca aagcacgtcg cacagattct cgattctcgg atgaacacca      2820
   agtacgacga gaacgacaag ctcatccgag aggtcaaggt gattactctc aagtccaaac      2880
   tggtctccga tttccgaaag gactttcagt tctacaaggt gcgagagatc aacaattacc      2940
   accatgccca cgatgcttac ctcaacgccg tcgttggcac tgcgctcatc aagaaatacc      3000
   ccaagctcga aagcgagttc gtttacggcg attacaaggt ctacgacgtt cgaaagatga      3060
   ttgccaagtc cgaacaggag attggcaagg ctactgccaa gtacttcttt tactccaaca      3120
   tcatgaactt tttcaagacc gagatcacct tggccaacgg agagattcga aagagaccac      3180
   ttatcgagac caacggcgaa actggagaga tcgtgtggga caagggtcga gactttgcaa      3240
   ccgtgcgaaa ggttctgtcg atgcctcagg tcaacatcgt caagaaaacc gaggttcaga      3300
   ctggcggatt ctccaaggag tcgattctgc ccaagcgaaa ctccgacaag ctcatcgctc      3360
   gaaagaaaga ctgggatccc aagaaatacg gtggcttcga ttctcctacc gtcgcctatt      3420
   ccgtgcttgt cgttgcgaag gtcgagaagg gcaagtccaa aaagctcaag tccgtcaagg      3480
   agctgctcgg aattaccatc atggagcgat cgagcttcga gaagaatccc atcgacttct      3540
   tggaagccaa gggttacaag gaggtcaaga aagacctcat tatcaagctg cccaagtact      3600
   ctctgttcga actggagaac ggtcgaaagc gtatgctcgc ctccgctggc gagctgcaga      3660
   agggaaacga gcttgccttg ccttcgaagt acgtcaactt tctctatctg gcttctcact      3720
   acgagaagct caagggttct cccgaggaca acgaacagaa gcaactcttc gttgagcagc      3780
   acaaacatta cctcgacgag attatcgagc agatttccga gttttcgaag cgagtcatcc      3840
   tggctgatgc caacttggac aaggtgctct ctgcctacaa caagcatcgg gacaaaccca      3900
   ttcgagaaca ggcggagaac atcattcacc tgtttactct taccaacctg ggtgctcctg      3960
   cagctttcaa gtacttcgat accactatcg accgaaagcg gtacacatcc accaaggagg      4020
   ttctcgatgc caccctgatt caccagtcca tcactggcct gtacgagacc cgaatcgacc      4080
   tgtctcagct tggtggcgac tccagagccg atcccaagaa aaagcgaaag gtctaagcgg      4140
   ccgc                                                                  4144
<210> 8
   <211> 35
   <212> DNA
   <213> Artificial sequence
<220>
   <223> Cas9 forward primer
<400> 8
   gggggaattc gacaagaaat actccatcgg cctgg 35
<210> 9
   <211> 31
   <212> DNA
   <213> Artificial sequence
<220>
   <223> Cas9 reverse primer
<400> 9
   ccccaagctt agcggccgct tagacctttc g 31
<210> 10
   <211> 4166
   <212> DNA
   <213> Artificial sequence
<220>
   <223> Cas9 PCR product
<400> 10
   gggggaattc gacaagaaat actccatcgg cctggacatt ggaaccaact ctgtcggctg      60
   ggctgtcatc accgacgagt acaaggtgcc ctccaagaaa ttcaaggtcc tcggaaacac     120
   cgatcgacac tccatcaaga aaaacctcat tggtgccctg ttgttcgatt ctggcgagac     180
   tgccgaagct accagactca agcgaactgc tcggcgacgt tacacccgac ggaagaaccg     240
   aatctgctac ctgcaggaga tcttttccaa cgagatggcc aaggtggacg attcgttctt     300
   tcatcgactg gaggaatcct tcctcgtcga ggaagacaag aaacacgagc gtcatcccat     360
   ctttggcaac attgtggacg aggttgctta ccacgagaag tatcctacca tctaccacct     420
   gcgaaagaaa ctcgtcgatt ccaccgacaa ggcggatctc agacttatct acctcgctct     480
   ggcacacatg atcaagtttc gaggtcattt cctcatcgag ggcgatctca atcccgacaa     540
   cagcgatgtg gacaagctgt tcattcagct cgttcagacc tacaaccagc tgttcgagga     600
   aaaccccatc aatgcctccg gagtcgatgc aaaggccatc ttgtctgctc gactctcgaa     660
   gagcagacga ctggagaacc tcattgccca acttcctggc gagaaaaaga acggactgtt     720
   tggcaacctc attgcccttt ctcttggtct cacacccaac ttcaagtcca acttcgatct     780
   ggcggaggac gccaagctcc agctgtccaa ggacacctac gacgatgacc tcgacaacct     840
   gcttgcacag attggcgatc agtacgccga cctgtttctc gctgccaaga acctttcgga     900
   tgctattctc ttgtctgaca ttctgcgagt caacaccgag atcacaaagg ctcccctttc     960
   tgcctccatg atcaagcgat acgacgagca ccatcaggat ctcacactgc tcaaggctct    1020
   tgtccgacag caactgcccg agaagtacaa ggagatcttt ttcgatcagt cgaagaacgg    1080
   ctacgctgga tacatcgacg gcggagcctc tcaggaagag ttctacaagt tcatcaagcc    1140
   aattctcgag aagatggacg gaaccgagga actgcttgtc aagctcaatc gagaggatct    1200
   gcttcggaag caacgaacct tcgacaacgg cagcattcct catcagatcc acctcggtga    1260
   gctgcacgcc attcttcgac gtcaggaaga cttctacccc tttctcaagg acaaccgaga    1320
   gaagatcgag aagattctta cctttcgaat cccctactat gttggtcctc ttgccagagg    1380
   aaactctcga tttgcttgga tgactcgaaa gtccgaggaa accatcactc cctggaactt    1440
   cgaggaagtc gtggacaagg gtgcctctgc acagtccttc atcgagcgaa tgaccaactt    1500
   cgacaagaat ctgcccaacg agaaggttct tcccaagcat tcgctgctct acgagtactt    1560
   tacagtctac aacgaactca ccaaagtcaa gtacgttacc gagggaatgc gaaagcctgc    1620
   cttcttgtct ggcgaacaga agaaagccat tgtcgatctc ctgttcaaga ccaaccgaaa    1680
   ggtcactgtt aagcagctca aggaggacta cttcaagaaa atcgagtgtt tcgacagcgt    1740
   cgagatttcc ggagttgagg accgattcaa cgcctctttg ggcacctatc acgatctgct    1800
   caagattatc aaggacaagg attttctcga caacgaggaa aacgaggaca ttctggagga    1860
   catcgtgctc actcttaccc tgttcgaaga tcgggagatg atcgaggaac gactcaagac    1920
   atacgctcac ctgttcgacg acaaggtcat gaaacaactc aagcgacgta gatacaccgg    1980
   ctggggaaga ctttcgcgaa agctcatcaa cggcatcaga gacaagcagt ccggaaagac    2040
   cattctggac tttctcaagt ccgatggctt tgccaaccga aacttcatgc agctcattca    2100
   cgacgattct cttaccttca aggaggacat ccagaaggca caagtgtccg gtcagggcga    2160
   cagcttgcac gaacatattg ccaacctggc tggttcgcca gccatcaaga aaggcattct    2220
   ccagactgtc aaggttgtcg acgagctggt gaaggtcatg ggacgtcaca agcccgagaa    2280
   cattgtgatc gagatggcca gagagaacca gacaactcaa aagggtcaga aaaactcgcg    2340
   agagcggatg aagcgaatcg aggaaggcat caaggagctg ggatcccaga ttctcaagga    2400
   gcatcccgtc gagaacactc aactgcagaa cgagaagctg tatctctact atctgcagaa    2460
   tggtcgagac atgtacgtgg atcaggaact ggacatcaat cgtctcagcg actacgatgt      2520
   ggaccacatt gtccctcaat cctttctcaa ggacgattct atcgacaaca aggtccttac      2580
   acgatccgac aagaacagag gcaagtcgga caacgttccc agcgaagagg tggtcaaaaa      2640
   gatgaagaac tactggcgac agctgctcaa cgccaagctc attacccagc gaaagttcga      2700
   caatcttacc aaggccgagc gaggcggtct gtccgagctc gacaaggctg gcttcatcaa      2760
   gcgtcaactc gtcgagacca gacagatcac aaagcacgtc gcacagattc tcgattctcg      2820
   gatgaacacc aagtacgacg agaacgacaa gctcatccga gaggtcaagg tgattactct      2880
   caagtccaaa ctggtctccg atttccgaaa ggactttcag ttctacaagg tgcgagagat      2940
   caacaattac caccatgccc acgatgctta cctcaacgcc gtcgttggca ctgcgctcat      3000
   caagaaatac cccaagctcg aaagcgagtt cgtttacggc gattacaagg tctacgacgt      3060
   tcgaaagatg attgccaagt ccgaacagga gattggcaag gctactgcca agtacttctt      3120
   ttactccaac atcatgaact ttttcaagac cgagatcacc ttggccaacg gagagattcg      3180
   aaagagacca cttatcgaga ccaacggcga aactggagag atcgtgtggg acaagggtcg      3240
   agactttgca accgtgcgaa aggttctgtc gatgcctcag gtcaacatcg tcaagaaaac      3300
   cgaggttcag actggcggat tctccaagga gtcgattctg cccaagcgaa actccgacaa      3360
   gctcatcgct cgaaagaaag actgggatcc caagaaatac ggtggcttcg attctcctac      3420
   cgtcgcctat tccgtgcttg tcgttgcgaa ggtcgagaag ggcaagtcca aaaagctcaa      3480
   gtccgtcaag gagctgctcg gaattaccat catggagcga tcgagcttcg agaagaatcc      3540
   catcgacttc ttggaagcca agggttacaa ggaggtcaag aaagacctca ttatcaagct      3600
   gcccaagtac tctctgttcg aactggagaa cggtcgaaag cgtatgctcg cctccgctgg      3660
   cgagctgcag aagggaaacg agcttgcctt gccttcgaag tacgtcaact ttctctatct      3720
   ggcttctcac tacgagaagc tcaagggttc tcccgaggac aacgaacaga agcaactctt      3780
   cgttgagcag cacaaacatt acctcgacga gattatcgag cagatttccg agttttcgaa      3840
   gcgagtcatc ctggctgatg ccaacttgga caaggtgctc tctgcctaca acaagcatcg      3900
   ggacaaaccc attcgagaac aggcggagaa catcattcac ctgtttactc ttaccaacct      3960
   gggtgctcct gcagctttca agtacttcga taccactatc gaccgaaagc ggtacacatc      4020
   caccaaggag gttctcgatg ccaccctgat tcaccagtcc atcactggcc tgtacgagac      4080
   ccgaatcgac ctgtctcagc ttggtggcga ctccagagcc gatcccaaga aaaagcgaaa      4140
   ggtctaagcg gccgctaagc ttgggg                                           4166
<210> 11
   <211> 4092
   <212> DNA
   <213> Artificial sequence
<220>
   <223> pBAD/HisB
<400> 11
   aagaaaccaa ttgtccatat tgcatcagac attgccgtca ctgcgtcttt tactggctct      60
   tctcgctaac caaaccggta accccgctta ttaaaagcat tctgtaacaa agcgggacca     120
   aagccatgac aaaaacgcgt aacaaaagtg tctataatca cggcagaaaa gtccacattg     180
   attatttgca cggcgtcaca ctttgctatg ccatagcatt tttatccata agattagcgg     240
   atcctacctg acgcttttta tcgcaactct ctactgtttc tccatacccg ttttttgggc      300
   taacaggagg aattaaccat ggggggttct catcatcatc atcatcatgg tatggctagc    360
   atgactggtg gacagcaaat gggtcgggat ctgtacgacg atgacgataa ggatccgagc     420
   tcgagatctg cagctggtac catatgggaa ttcgaagctt ggctgttttg gcggatgaga     480
   gaagattttc agcctgatac agattaaatc agaacgcaga agcggtctga taaaacagaa     540
   tttgcctggc ggcagtagcg cggtggtccc acctgacccc atgccgaact cagaagtgaa     600
   acgccgtagc gccgatggta gtgtggggtc tccccatgcg agagtaggga actgccaggc     660
   atcaaataaa acgaaaggct cagtcgaaag actgggcctt tcgttttatc tgttgtttgt     720
   cggtgaacgc tctcctgagt aggacaaatc cgccgggagc ggatttgaac gttgcgaagc     780
   aacggcccgg agggtggcgg gcaggacgcc cgccataaac tgccaggcat caaattaagc     840
   agaaggccat cctgacggat ggcctttttg cgtttctaca aactcttttg tttatttttc     900
   taaatacatt caaatatgta tccgctcatg agacaataac cctgataaat gcttcaataa     960
   tattgaaaaa ggaagagtat gagtattcaa catttccgtg tcgcccttat tccctttttt    1020
   gcggcatttt gccttcctgt ttttgctcac ccagaaacgc tggtgaaagt aaaagatgct    1080
   gaagatcagt tgggtgcacg agtgggttac atcgaactgg atctcaacag cggtaagatc    1140
   cttgagagtt ttcgccccga agaacgtttt ccaatgatga gcacttttaa agttctgcta    1200
   tgtggcgcgg tattatcccg tgttgacgcc gggcaagagc aactcggtcg ccgcatacac    1260
   tattctcaga atgacttggt tgagtactca ccagtcacag aaaagcatct tacggatggc    1320
   atgacagtaa gagaattatg cagtgctgcc ataaccatga gtgataacac tgcggccaac    1380
   ttacttctga caacgatcgg aggaccgaag gagctaaccg cttttttgca caacatgggg    1440
   gatcatgtaa ctcgccttga tcgttgggaa ccggagctga atgaagccat accaaacgac      1500
   gagcgtgaca ccacgatgcc tgtagcaatg gcaacaacgt tgcgcaaact attaactggc      1560
   gaactactta ctctagcttc ccggcaacaa ttaatagact ggatggaggc ggataaagtt      1620
   gcaggaccac ttctgcgctc ggcccttccg gctggctggt ttattgctga taaatctgga      1680
   gccggtgagc gtgggtctcg cggtatcatt gcagcactgg ggccagatgg taagccctcc      1740
   cgtatcgtag ttatctacac gacggggagt caggcaacta tggatgaacg aaatagacag      1800
   atcgctgaga taggtgcctc actgattaag cattggtaac tgtcagacca agtttactca      1860
   tatatacttt agattgattt aaaacttcat ttttaattta aaaggatcta ggtgaagatc      1920
   ctttttgata atctcatgac caaaatccct taacgtgagt tttcgttcca ctgagcgtca      1980
   gaccccgtag aaaagatcaa aggatcttct tgagatcctt tttttctgcg cgtaatctgc      2040
   tgcttgcaaa caaaaaaacc accgctacca gcggtggttt gtttgccgga tcaagagcta      2100
   ccaactcttt ttccgaaggt aactggcttc agcagagcgc agataccaaa tactgtcctt      2160
   ctagtgtagc cgtagttagg ccaccacttc aagaactctg tagcaccgcc tacatacctc      2220
   gctctgctaa tcctgttacc agtggctgct gccagtggcg ataagtcgtg tcttaccggg      2280
   ttggactcaa gacgatagtt accggataag gcgcagcggt cgggctgaac ggggggttcg      2340
   tgcacacagc ccagcttgga gcgaacgacc tacaccgaac tgagatacct acagcgtgag      2400
   ctatgagaaa gcgccacgct tcccgaaggg agaaaggcgg acaggtatcc ggtaagcggc      2460
   agggtcggaa caggagagcg cacgagggag cttccagggg gaaacgcctg gtatctttat      2520
   agtcctgtcg ggtttcgcca cctctgactt gagcgtcgat ttttgtgatg ctcgtcaggg      2580
   gggcggagcc tatggaaaaa cgccagcaac gcggcctttt tacggttcct ggccttttgc      2640
   tggccttttg ctcacatgtt ctttcctgcg ttatcccctg attctgtgga taaccgtatt      2700
   accgcctttg agtgagctga taccgctcgc cgcagccgaa cgaccgagcg cagcgagtca      2760
   gtgagcgagg aagcggaaga gcgcctgatg cggtattttc tccttacgca tctgtgcggt      2820
   atttcacacc gcatatggtg cactctcagt acaatctgct ctgatgccgc atagttaagc      2880
   cagtatacac tccgctatcg ctacgtgact gggtcatggc tgcgccccga cacccgccaa      2940
   cacccgctga cgcgccctga cgggcttgtc tgctcccggc atccgcttac agacaagctg      3000
   tgaccgtctc cgggagctgc atgtgtcaga ggttttcacc gtcatcaccg aaacgcgcga      3060
   ggcagcagat caattcgcgc gcgaaggcga agcggcatgc ataatgtgcc tgtcaaatgg      3120
   acgaagcagg gattctgcaa accctatgct actccgtcaa gccgtcaatt gtctgattcg      3180
   ttaccaatta tgacaacttg acggctacat cattcacttt ttcttcacaa ccggcacgga      3240
   actcgctcgg gctggccccg gtgcattttt taaatacccg cgagaaatag agttgatcgt      3300
   caaaaccaac attgcgaccg acggtggcga taggcatccg ggtggtgctc aaaagcagct      3360
   tcgcctggct gatacgttgg tcctcgcgcc agcttaagac gctaatccct aactgctggc      3420
   ggaaaagatg tgacagacgc gacggcgaca agcaaacatg ctgtgcgacg ctggcgatat      3480
   caaaattgct gtctgccagg tgatcgctga tgtactgaca agcctcgcgt acccgattat      3540
   ccatcggtgg atggagcgac tcgttaatcg cttccatgcg ccgcagtaac aattgctcaa      3600
   gcagatttat cgccagcagc tccgaatagc gcccttcccc ttgcccggcg ttaatgattt      3660
   gcccaaacag gtcgctgaaa tgcggctggt gcgcttcatc cgggcgaaag aaccccgtat      3720
   tggcaaatat tgacggccag ttaagccatt catgccagta ggcgcgcgga cgaaagtaaa      3780
   cccactggtg ataccattcg cgagcctccg gatgacgacc gtagtgatga atctctcctg      3840
   gcgggaacag caaaatatca cccggtcggc aaacaaattc tcgtccctga tttttcacca      3900
   ccccctgacc gcgaatggtg agattgagaa tataaccttt cattcccagc ggtcggtcga      3960
   taaaaaaatc gagataaccg ttggcctcaa tcggcgttaa acccgccacc agatgggcat      4020
   taaacgagta tcccggcagc aggggatcat tttgcgcttc agccatactt ttcatactcc      4080
   cgccattcag ag                                                          4092
<210> 12
   <211> 8237
   <212> DNA
   <213> Artificial sequence
<220>
   <223> pRF48
<400> 12
   aattcgacaa gaaatactcc atcggcctgg acattggaac caactctgtc ggctgggctg      60
   tcatcaccga cgagtacaag gtgccctcca agaaattcaa ggtcctcgga aacaccgatc     120
   gacactccat caagaaaaac ctcattggtg ccctgttgtt cgattctggc gagactgccg     180
   aagctaccag actcaagcga actgctcggc gacgttacac ccgacggaag aaccgaatct     240
   gctacctgca ggagatcttt tccaacgaga tggccaaggt ggacgattcg ttctttcatc     300
   gactggagga atccttcctc gtcgaggaag acaagaaaca cgagcgtcat cccatctttg     360
   gcaacattgt ggacgaggtt gcttaccacg agaagtatcc taccatctac cacctgcgaa     420
   agaaactcgt cgattccacc gacaaggcgg atctcagact tatctacctc gctctggcac     480
   acatgatcaa gtttcgaggt catttcctca tcgagggcga tctcaatccc gacaacagcg      540
   atgtggacaa gctgttcatt cagctcgttc agacctacaa ccagctgttc gaggaaaacc      600
   ccatcaatgc ctccggagtc gatgcaaagg ccatcttgtc tgctcgactc tcgaagagca      660
   gacgactgga gaacctcatt gcccaacttc ctggcgagaa aaagaacgga ctgtttggca      720
   acctcattgc cctttctctt ggtctcacac ccaacttcaa gtccaacttc gatctggcgg      780
   aggacgccaa gctccagctg tccaaggaca cctacgacga tgacctcgac aacctgcttg      840
   cacagattgg cgatcagtac gccgacctgt ttctcgctgc caagaacctt tcggatgcta      900
   ttctcttgtc tgacattctg cgagtcaaca ccgagatcac aaaggctccc ctttctgcct      960
   ccatgatcaa gcgatacgac gagcaccatc aggatctcac actgctcaag gctcttgtcc     1020
   gacagcaact gcccgagaag tacaaggaga tctttttcga tcagtcgaag aacggctacg     1080
   ctggatacat cgacggcgga gcctctcagg aagagttcta caagttcatc aagccaattc     1140
   tcgagaagat ggacggaacc gaggaactgc ttgtcaagct caatcgagag gatctgcttc     1200
   ggaagcaacg aaccttcgac aacggcagca ttcctcatca gatccacctc ggtgagctgc     1260
   acgccattct tcgacgtcag gaagacttct acccctttct caaggacaac cgagagaaga     1320
   tcgagaagat tcttaccttt cgaatcccct actatgttgg tcctcttgcc agaggaaact     1380
   ctcgatttgc ttggatgact cgaaagtccg aggaaaccat cactccctgg aacttcgagg     1440
   aagtcgtgga caagggtgcc tctgcacagt ccttcatcga gcgaatgacc aacttcgaca     1500
   agaatctgcc caacgagaag gttcttccca agcattcgct gctctacgag tactttacag     1560
   tctacaacga actcaccaaa gtcaagtacg ttaccgaggg aatgcgaaag cctgccttct     1620
   tgtctggcga acagaagaaa gccattgtcg atctcctgtt caagaccaac cgaaaggtca     1680
   ctgttaagca gctcaaggag gactacttca agaaaatcga gtgtttcgac agcgtcgaga     1740
   tttccggagt tgaggaccga ttcaacgcct ctttgggcac ctatcacgat ctgctcaaga     1800
   ttatcaagga caaggatttt ctcgacaacg aggaaaacga ggacattctg gaggacatcg     1860
   tgctcactct taccctgttc gaagatcggg agatgatcga ggaacgactc aagacatacg     1920
   ctcacctgtt cgacgacaag gtcatgaaac aactcaagcg acgtagatac accggctggg     1980
   gaagactttc gcgaaagctc atcaacggca tcagagacaa gcagtccgga aagaccattc     2040
   tggactttct caagtccgat ggctttgcca accgaaactt catgcagctc attcacgacg     2100
   attctcttac cttcaaggag gacatccaga aggcacaagt gtccggtcag ggcgacagct     2160
   tgcacgaaca tattgccaac ctggctggtt cgccagccat caagaaaggc attctccaga     2220
   ctgtcaaggt tgtcgacgag ctggtgaagg tcatgggacg tcacaagccc gagaacattg     2280
   tgatcgagat ggccagagag aaccagacaa ctcaaaaggg tcagaaaaac tcgcgagagc     2340
   ggatgaagcg aatcgaggaa ggcatcaagg agctgggatc ccagattctc aaggagcatc     2400
   ccgtcgagaa cactcaactg cagaacgaga agctgtatct ctactatctg cagaatggtc     2460
   gagacatgta cgtggatcag gaactggaca tcaatcgtct cagcgactac gatgtggacc     2520
   acattgtccc tcaatccttt ctcaaggacg attctatcga caacaaggtc cttacacgat     2580
   ccgacaagaa cagaggcaag tcggacaacg ttcccagcga agaggtggtc aaaaagatga     2640
   agaactactg gcgacagctg ctcaacgcca agctcattac ccagcgaaag ttcgacaatc     2700
   ttaccaaggc cgagcgaggc ggtctgtccg agctcgacaa ggctggcttc atcaagcgtc     2760
   aactcgtcga gaccagacag atcacaaagc acgtcgcaca gattctcgat tctcggatga     2820
   acaccaagta cgacgagaac gacaagctca tccgagaggt caaggtgatt actctcaagt     2880
   ccaaactggt ctccgatttc cgaaaggact ttcagttcta caaggtgcga gagatcaaca     2940
   attaccacca tgcccacgat gcttacctca acgccgtcgt tggcactgcg ctcatcaaga     3000
   aataccccaa gctcgaaagc gagttcgttt acggcgatta caaggtctac gacgttcgaa     3060
   agatgattgc caagtccgaa caggagattg gcaaggctac tgccaagtac ttcttttact     3120
   ccaacatcat gaactttttc aagaccgaga tcaccttggc caacggagag attcgaaaga     3180
   gaccacttat cgagaccaac ggcgaaactg gagagatcgt gtgggacaag ggtcgagact     3240
   ttgcaaccgt gcgaaaggtt ctgtcgatgc ctcaggtcaa catcgtcaag aaaaccgagg     3300
   ttcagactgg cggattctcc aaggagtcga ttctgcccaa gcgaaactcc gacaagctca     3360
   tcgctcgaaa gaaagactgg gatcccaaga aatacggtgg cttcgattct cctaccgtcg     3420
   cctattccgt gcttgtcgtt gcgaaggtcg agaagggcaa gtccaaaaag ctcaagtccg     3480
   tcaaggagct gctcggaatt accatcatgg agcgatcgag cttcgagaag aatcccatcg     3540
   acttcttgga agccaagggt tacaaggagg tcaagaaaga cctcattatc aagctgccca     3600
   agtactctct gttcgaactg gagaacggtc gaaagcgtat gctcgcctcc gctggcgagc     3660
   tgcagaaggg aaacgagctt gccttgcctt cgaagtacgt caactttctc tatctggctt     3720
   ctcactacga gaagctcaag ggttctcccg aggacaacga acagaagcaa ctcttcgttg     3780
   agcagcacaa acattacctc gacgagatta tcgagcagat ttccgagttt tcgaagcgag     3840
   tcatcctggc tgatgccaac ttggacaagg tgctctctgc ctacaacaag catcgggaca     3900
   aacccattcg agaacaggcg gagaacatca ttcacctgtt tactcttacc aacctgggtg     3960
   ctcctgcagc tttcaagtac ttcgatacca ctatcgaccg aaagcggtac acatccacca     4020
   aggaggttct cgatgccacc ctgattcacc agtccatcac tggcctgtac gagacccgaa     4080
   tcgacctgtc tcagcttggt ggcgactcca gagccgatcc caagaaaaag cgaaaggtct     4140
   aagcggccgc taagcttggc tgttttggcg gatgagagaa gattttcagc ctgatacaga     4200
   ttaaatcaga acgcagaagc ggtctgataa aacagaattt gcctggcggc agtagcgcgg     4260
   tggtcccacc tgaccccatg ccgaactcag aagtgaaacg ccgtagcgcc gatggtagtg 4320
   tggggtctcc ccatgcgaga gtagggaact gccaggcatc aaataaaacg aaaggctcag 4380
   tcgaaagact gggcctttcg ttttatctgt tgtttgtcgg tgaacgctct cctgagtagg 4440
   acaaatccgc cgggagcgga tttgaacgtt gcgaagcaac ggcccggagg gtggcgggca 4500
   ggacgcccgc cataaactgc caggcatcaa attaagcaga aggccatcct gacggatggc 4560
   ctttttgcgt ttctacaaac tcttttgttt atttttctaa atacattcaa atatgtatcc 4620
   gctcatgaga caataaccct gataaatgct tcaataatat tgaaaaagga agagtatgag 4680
   tattcaacat ttccgtgtcg cccttattcc cttttttgcg gcattttgcc ttcctgtttt 4740
   tgctcaccca gaaacgctgg tgaaagtaaa agatgctgaa gatcagttgg gtgcacgagt 4800
   gggttacatc gaactggatc tcaacagcgg taagatcctt gagagttttc gccccgaaga 4860
   acgttttcca atgatgagca cttttaaagt tctgctatgt ggcgcggtat tatcccgtgt 4920
   tgacgccggg caagagcaac tcggtcgccg catacactat tctcagaatg acttggttga 4980
   gtactcacca gtcacagaaa agcatcttac ggatggcatg acagtaagag aattatgcag 5040
   tgctgccata accatgagtg ataacactgc ggccaactta cttctgacaa cgatcggagg 5100
   accgaaggag ctaaccgctt ttttgcacaa catgggggat catgtaactc gccttgatcg 5160
   ttgggaaccg gagctgaatg aagccatacc aaacgacgag cgtgacacca cgatgcctgt 5220
   agcaatggca acaacgttgc gcaaactatt aactggcgaa ctacttactc tagcttcccg 5280
   gcaacaatta atagactgga tggaggcgga taaagttgca ggaccacttc tgcgctcggc 5340
   ccttccggct ggctggttta ttgctgataa atctggagcc ggtgagcgtg ggtctcgcgg 5400
   tatcattgca gcactggggc cagatggtaa gccctcccgt atcgtagtta tctacacgac 5460
   ggggagtcag gcaactatgg atgaacgaaa tagacagatc gctgagatag gtgcctcact 5520
   gattaagcat tggtaactgt cagaccaagt ttactcatat atactttaga ttgatttaaa 5580
   acttcatttt taatttaaaa ggatctaggt gaagatcctt tttgataatc tcatgaccaa 5640
   aatcccttaa cgtgagtttt cgttccactg agcgtcagac cccgtagaaa agatcaaagg 5700
   atcttcttga gatccttttt ttctgcgcgt aatctgctgc ttgcaaacaa aaaaaccacc 5760
   gctaccagcg gtggtttgtt tgccggatca agagctacca actctttttc cgaaggtaac 5820
   tggcttcagc agagcgcaga taccaaatac tgtccttcta gtgtagccgt agttaggcca 5880
   ccacttcaag aactctgtag caccgcctac atacctcgct ctgctaatcc tgttaccagt 5940
   ggctgctgcc agtggcgata agtcgtgtct taccgggttg gactcaagac gatagttacc 6000
   ggataaggcg cagcggtcgg gctgaacggg gggttcgtgc acacagccca gcttggagcg 6060
   aacgacctac accgaactga gatacctaca gcgtgagcta tgagaaagcg ccacgcttcc 6120
   cgaagggaga aaggcggaca ggtatccggt aagcggcagg gtcggaacag gagagcgcac 6180
   gagggagctt ccagggggaa acgcctggta tctttatagt cctgtcgggt ttcgccacct 6240
   ctgacttgag cgtcgatttt tgtgatgctc gtcagggggg cggagcctat ggaaaaacgc 6300
   cagcaacgcg gcctttttac ggttcctggc cttttgctgg ccttttgctc acatgttctt 6360
   tcctgcgtta tcccctgatt ctgtggataa ccgtattacc gcctttgagt gagctgatac 6420
   cgctcgccgc agccgaacga ccgagcgcag cgagtcagtg agcgaggaag cggaagagcg 6480
   cctgatgcgg tattttctcc ttacgcatct gtgcggtatt tcacaccgca tatggtgcac 6540
   tctcagtaca atctgctctg atgccgcata gttaagccag tatacactcc gctatcgcta 6600
   cgtgactggg tcatggctgc gccccgacac ccgccaacac ccgctgacgc gccctgacgg 6660
   gcttgtctgc tcccggcatc cgcttacaga caagctgtga ccgtctccgg gagctgcatg 6720
   tgtcagaggt tttcaccgtc atcaccgaaa cgcgcgaggc agcagatcaa ttcgcgcgcg 6780
   aaggcgaagc ggcatgcata atgtgcctgt caaatggacg aagcagggat tctgcaaacc 6840
   ctatgctact ccgtcaagcc gtcaattgtc tgattcgtta ccaattatga caacttgacg 6900
   gctacatcat tcactttttc ttcacaaccg gcacggaact cgctcgggct ggccccggtg 6960
   cattttttaa atacccgcga gaaatagagt tgatcgtcaa aaccaacatt gcgaccgacg 7020
   gtggcgatag gcatccgggt ggtgctcaaa agcagcttcg cctggctgat acgttggtcc 7080
   tcgcgccagc ttaagacgct aatccctaac tgctggcgga aaagatgtga cagacgcgac 7140
   ggcgacaagc aaacatgctg tgcgacgctg gcgatatcaa aattgctgtc tgccaggtga 7200
   tcgctgatgt actgacaagc ctcgcgtacc cgattatcca tcggtggatg gagcgactcg 7260
   ttaatcgctt ccatgcgccg cagtaacaat tgctcaagca gatttatcgc cagcagctcc 7320
   gaatagcgcc cttccccttg cccggcgtta atgatttgcc caaacaggtc gctgaaatgc 7380
   ggctggtgcg cttcatccgg gcgaaagaac cccgtattgg caaatattga cggccagtta 7440
   agccattcat gccagtaggc gcgcggacga aagtaaaccc actggtgata ccattcgcga 7500
   gcctccggat gacgaccgta gtgatgaatc tctcctggcg ggaacagcaa aatatcaccc 7560
   ggtcggcaaa caaattctcg tccctgattt ttcaccaccc cctgaccgcg aatggtgaga 7620
   ttgagaatat aacctttcat tcccagcggt cggtcgataa aaaaatcgag ataaccgttg 7680
   gcctcaatcg gcgttaaacc cgccaccaga tgggcattaa acgagtatcc cggcagcagg 7740
   ggatcatttt gcgcttcagc catacttttc atactcccgc cattcagaga agaaaccaat 7800
   tgtccatatt gcatcagaca ttgccgtcac tgcgtctttt actggctctt ctcgctaacc 7860
   aaaccggtaa ccccgcttat taaaagcatt ctgtaacaaa gcgggaccaa agccatgaca 7920
   aaaacgcgta acaaaagtgt ctataatcac ggcagaaaag tccacattga ttatttgcac 7980
   ggcgtcacac tttgctatgc catagcattt ttatccataa gattagcgga tcctacctga 8040
   cgctttttat cgcaactctc tactgtttct ccatacccgt tttttgggct aacaggagga     8100
   attaaccatg gggggttctc atcatcatca tcatcatggt atggctagca tgactggtgg     8160
   acagcaaatg ggtcgggatc tgtacgacga tgacgataag gatccgagct cgagatctgc     8220
   agctggtacc atatggg                                                    8237
<210> 13
   <211> 23
   <212> DNA
   <213> Escherichia coli
<220>
   <221> misc_feature
   <222> (1)..(23)
   <223> GalK-1 target site
<400> 13
   atcagcggca atgtgccgca ggg 23
<210> 14
   <211> 23
   <212> DNA
   <213> Escherichia coli
<220>
   <221> misc_feature
   <222> (1)..(23)
   <223> GalK-2 target site
<400> 14
   atgaccggcg gcggatttgg cgg 23
<210> 15
   <211> 23
   <212> DNA
   <213> Escherichia coli
<220>
   <221> misc_feature
   <222> (1)..(23)
   <223> GalK-3 target site
<400> 15
   atagttttca tgtgcgacaa tgg 23
<210> 16
   <211> 23
   <212> DNA
   <213> Escherichia coli
<220>
   <221> misc_feature
   <222> (1)..(23)
   <223> GalK-4 target site
<400> 16
   atgatctttc ttgccgagcg cgg 23
<210> 17
   <211> 80
   <212> DNA
   <213> Streptococcus pyogenes
<400> 17
   gttttagagc tagaaatagc aagttaaaat aaggctagtc cgttatcaac ttgaaaaagt        60
   ggcaccgagt cggtgctttt                                                    80
<210> 18
   <211> 100
   <212> DNA
   <213> Artificial sequence
<220>
   <223> GalK-1 sgRNA template
<400> 18
   atcagcggca atgtgccgca gttttagagc tagaaatagc aagttaaaat aaggctagtc        60
   cgttatcaac ttgaaaaagt ggcaccgagt cggtgctttt                             100
<210> 19
   <211> 100
   <212> DNA
   <213> Artificial sequence
<220>
   <223> GalK-2 sgRNA template
<400> 19
   atgaccggcg gcggatttgg gttttagagc tagaaatagc aagttaaaat aaggctagtc        60
   cgttatcaac ttgaaaaagt ggcaccgagt cggtgctttt                             100
<210> 20
   <211> 100
   <212> DNA
   <213> Artificial sequence
<220>
   <223> GalK-3 sgRNA template
<400> 20
   atagttttca tgtgcgacaa gttttagagc tagaaatagc aagttaaaat aaggctagtc        60
   cgttatcaac ttgaaaaagt ggcaccgagt cggtgctttt                             100
<210> 21
   <211> 100
   <212> DNA
   <213> Artificial sequence
<220>
   <223> GalK-4 sgRNA template
<400> 21
   atgatctttc ttgccgagcg gttttagagc tagaaatagc aagttaaaat aaggctagtc      60
   cgttatcaac ttgaaaaagt ggcaccgagt cggtgctttt                           100
<210> 22
   <211> 100
   <212> RNA
   <213> Artificial sequence
<220>
   <223> GalK-1 sgRNA
<400> 22
   aucagcggca augugccgca guuuuagagc uagaaauagc aaguuaaaau aaggcuaguc      60
   cguuaucaac uugaaaaagu ggcaccgagu cggugcuuuu                           100
<210> 23
   <211> 100
   <212> RNA
   <213> Artificial sequence
<220>
   <223> GalK-2 sgRNA
<400> 23
   augaccggcg gcggauuugg guuuuagagc uagaaauagc aaguuaaaau aaggcuaguc      60
   cguuaucaac uugaaaaagu ggcaccgagu cggugcuuuu                           100
<210> 24
   <211> 100
   <212> RNA
   <213> Artificial sequence
<220>
   <223> GalK-3 sgRNA
<400> 24
   auaguuuuca ugugcgacaa guuuuagagc uagaaauagc aaguuaaaau aaggcuaguc      60
   cguuaucaac uugaaaaagu ggcaccgagu cggugcuuuu                           100
<210> 25
   <211> 100
   <212> RNA
   <213> Artificial sequence
<220>
   <223> GalK-4 sgRNA
<400> 25
   augaucuuuc uugccgagcg guuuuagagc uagaaauagc aaguuaaaau aaggcuaguc 60
   cguuaucaac uugaaaaagu ggcaccgagu cggugcuuuu 100
<210> 26
   <211> 52
   <212> DNA
   <213> bacteriophage lambda
<400> 26
   ggttatctct ggcggtgttg acataaatac cactggcggt gatactgagc ac 52
<210> 27
   <211> 43
   <212> DNA
   <213> bacteriophage lambda
<400> 27
   gttaataaca ggcctgctgg taatcgcagg cctttttatt ttt 43
<210> 28
   <211> 212
   <212> DNA
   <213> Artificial sequence
<220>
   <223> GalK-1 sgRNA expression cassette
<400> 28
   gggaagcttg gttatctctg gcggtgttga cataaatacc actggcggtg atactgagca 60
   catcagcggc aatgtgccgc agttttagag ctagaaatag caagttaaaa taaggctagt 120
   ccgttatcaa cttgaaaaag tggcaccgag tcggtggtgc gttaataaca ggcctgctgg 180
   taatcgcagg cctttttatt tttggatccg gg 212
<210> 29
   <211> 212
   <212> DNA
   <213> Artificial sequence
<220>
   <223> GalK-2 sgRNA expression cassette
<400> 29
   gggaagcttg gttatctctg gcggtgttga cataaatacc actggcggtg atactgagca 60
   catgaccggc ggcggatttg ggttttagag ctagaaatag caagttaaaa taaggctagt 120
   ccgttatcaa cttgaaaaag tggcaccgag tcggtggtgc gttaataaca ggcctgctgg 180
   taatcgcagg cctttttatt tttggatccg gg 212
<210> 30
   <211> 212
   <212> DNA
   <213> Artificial sequence
<220>
   <223> GalK-3 sgRNA expression cassette
<400> 30
   gggaagcttg gttatctctg gcggtgttga cataaatacc actggcggtg atactgagca      60
   catagttttc atgtgcgaca agttttagag ctagaaatag caagttaaaa taaggctagt     120
   ccgttatcaa cttgaaaaag tggcaccgag tcggtggtgc gttaataaca ggcctgctgg     180
   taatcgcagg cctttttatt tttggatccg gg                                   212
<210> 31
   <211> 212
   <212> DNA
   <213> Artificial sequence
<220>
   <223> GalK-4 sgRNA expression cassette
<400> 31
   gggaagcttg gttatctctg gcggtgttga cataaatacc actggcggtg atactgagca      60
   catgatcttt cttgccgagc ggttttagag ctagaaatag caagttaaaa taaggctagt     120
   ccgttatcaa cttgaaaaag tggcaccgag tcggtggtgc gttaataaca ggcctgctgg     180
   taatcgcagg cctttttatt tttggatccg gg                                   212
<210> 32
   <211> 4245
   <212> DNA
   <213> Artificial sequence
<220>
   <223> pACYC184
<400> 32
   gaattccgga tgagcattca tcaggcgggc aagaatgtga ataaaggccg gataaaactt      60
   gtgcttattt ttctttacgg tctttaaaaa ggccgtaata tccagctgaa cggtctggtt     120
   ataggtacat tgagcaactg actgaaatgc ctcaaaatgt tctttacgat gccattggga     180
   tatatcaacg gtggtatatc cagtgatttt tttctccatt ttagcttcct tagctcctga     240
   aaatctcgat aactcaaaaa atacgcccgg tagtgatctt atttcattat ggtgaaagtt     300
   ggaacctctt acgtgccgat caacgtctca ttttcgccaa aagttggccc agggcttccc     360
   ggtatcaaca gggacaccag gatttattta ttctgcgaag tgatcttccg tcacaggtat     420
   ttattcggcg caaagtgcgt cgggtgatgc tgccaactta ctgatttagt gtatgatggt     480
   gtttttgagg tgctccagtg gcttctgttt ctatcagctg tccctcctgt tcagctactg     540
   acggggtggt gcgtaacggc aaaagcaccg ccggacatca gcgctagcgg agtgtatact 600
   ggcttactat gttggcactg atgagggtgt cagtgaagtg cttcatgtgg caggagaaaa 660
   aaggctgcac cggtgcgtca gcagaatatg tgatacagga tatattccgc ttcctcgctc 720
   actgactcgc tacgctcggt cgttcgactg cggcgagcgg aaatggctta cgaacggggc 780
   ggagatttcc tggaagatgc caggaagata cttaacaggg aagtgagagg gccgcggcaa 840
   agccgttttt ccataggctc cgcccccctg acaagcatca cgaaatctga cgctcaaatc 900
   agtggtggcg aaacccgaca ggactataaa gataccaggc gtttccccct ggcggctccc 960
   tcgtgcgctc tcctgttcct gcctttcggt ttaccggtgt cattccgctg ttatggccgc 1020
   gtttgtctca ttccacgcct gacactcagt tccgggtagg cagttcgctc caagctggac 1080
   tgtatgcacg aaccccccgt tcagtccgac cgctgcgcct tatccggtaa ctatcgtctt 1140
   gagtccaacc cggaaagaca tgcaaaagca ccactggcag cagccactgg taattgattt 1200
   agaggagtta gtcttgaagt catgcgccgg ttaaggctaa actgaaagga caagttttgg 1260
   tgactgcgct cctccaagcc agttacctcg gttcaaagag ttggtagctc agagaacctt 1320
   cgaaaaaccg ccctgcaagg cggttttttc gttttcagag caagagatta cgcgcagacc 1380
   aaaacgatct caagaagatc atcttattaa tcagataaaa tatttctaga tttcagtgca 1440
   atttatctct tcaaatgtag cacctgaagt cagccccata cgatataagt tgtaattctc 1500
   atgtttgaca gcttatcatc gataagcttt aatgcggtag tttatcacag ttaaattgct 1560
   aacgcagtca ggcaccgtgt atgaaatcta acaatgcgct catcgtcatc ctcggcaccg 1620
   tcaccctgga tgctgtaggc ataggcttgg ttatgccggt actgccgggc ctcttgcggg 1680
   atatcgtcca ttccgacagc atcgccagtc actatggcgt gctgctagcg ctatatgcgt 1740
   tgatgcaatt tctatgcgca cccgttctcg gagcactgtc cgaccgcttt ggccgccgcc 1800
   cagtcctgct cgcttcgcta cttggagcca ctatcgacta cgcgatcatg gcgaccacac 1860
   ccgtcctgtg gatcctctac gccggacgca tcgtggccgg catcaccggc gccacaggtg 1920
   cggttgctgg cgcctatatc gccgacatca ccgatgggga agatcgggct cgccacttcg 1980
   ggctcatgag cgcttgtttc ggcgtgggta tggtggcagg ccccgtggcc gggggactgt 2040
   tgggcgccat ctccttgcat gcaccattcc ttgcggcggc ggtgctcaac ggcctcaacc 2100
   tactactggg ctgcttccta atgcaggagt cgcataaggg agagcgtcga ccgatgccct 2160
   tgagagcctt caacccagtc agctccttcc ggtgggcgcg gggcatgact atcgtcgccg 2220
   cacttatgac tgtcttcttt atcatgcaac tcgtaggaca ggtgccggca gcgctctggg 2280
   tcattttcgg cgaggaccgc tttcgctgga gcgcgacgat gatcggcctg tcgcttgcgg 2340
   tattcggaat cttgcacgcc ctcgctcaag ccttcgtcac tggtcccgcc accaaacgtt 2400
   tcggcgagaa gcaggccatt atcgccggca tggcggccga cgcgctgggc tacgtcttgc 2460
   tggcgttcgc gacgcgaggc tggatggcct tccccattat gattcttctc gcttccggcg 2520
   gcatcgggat gcccgcgttg caggccatgc tgtccaggca ggtagatgac gaccatcagg 2580
   gacagcttca aggatcgctc gcggctctta ccagcctaac ttcgatcact ggaccgctga 2640
   tcgtcacggc gatttatgcc gcctcggcga gcacatggaa cgggttggca tggattgtag 2700
   gcgccgccct ataccttgtc tgcctccccg cgttgcgtcg cggtgcatgg agccgggcca 2760
   cctcgacctg aatggaagcc ggcggcacct cgctaacgga ttcaccactc caagaattgg 2820
   agccaatcaa ttcttgcgga gaactgtgaa tgcgcaaacc aacccttggc agaacatatc 2880
   catcgcgtcc gccatctcca gcagccgcac gcggcgcatc tcgggcagcg ttgggtcctg 2940
   gccacgggtg cgcatgatcg tgctcctgtc gttgaggacc cggctaggct ggcggggttg 3000
   ccttactggt tagcagaatg aatcaccgat acgcgagcga acgtgaagcg actgctgctg 3060
   caaaacgtct gcgacctgag caacaacatg aatggtcttc ggtttccgtg tttcgtaaag 3120
   tctggaaacg cggaagtccc ctacgtgctg ctgaagttgc ccgcaacaga gagtggaacc 3180
   aaccggtgat accacgatac tatgactgag agtcaacgcc atgagcggcc tcatttctta 3240
   ttctgagtta caacagtccg caccgctgtc cggtagctcc ttccggtggg cgcggggcat 3300
   gactatcgtc gccgcactta tgactgtctt ctttatcatg caactcgtag gacaggtgcc 3360
   ggcagcgccc aacagtcccc cggccacggg gcctgccacc atacccacgc cgaaacaagc 3420
   gccctgcacc attatgttcc ggatctgcat cgcaggatgc tgctggctac cctgtggaac 3480
   acctacatct gtattaacga agcgctaacc gtttttatca ggctctggga ggcagaataa 3540
   atgatcatat cgtcaattat tacctccacg gggagagcct gagcaaactg gcctcaggca 3600
   tttgagaagc acacggtcac actgcttccg gtagtcaata aaccggtaaa ccagcaatag 3660
   acataagcgg ctatttaacg accctgccct gaaccgacga ccgggtcgaa tttgctttcg 3720
   aatttctgcc attcatccgc ttattatcac ttattcaggc gtagcaccag gcgtttaagg 3780
   gcaccaataa ctgccttaaa aaaattacgc cccgccctgc cactcatcgc agtactgttg 3840
   taattcatta agcattctgc cgacatggaa gccatcacag acggcatgat gaacctgaat 3900
   cgccagcggc atcagcacct tgtcgccttg cgtataatat ttgcccatgg tgaaaacggg 3960
   ggcgaagaag ttgtccatat tggccacgtt taaatcaaaa ctggtgaaac tcacccaggg 4020
   attggctgag acgaaaaaca tattctcaat aaacccttta gggaaatagg ccaggttttc 4080
   accgtaacac gccacatctt gcgaatatat gtgtagaaac tgccggaaat cgtcgtggta 4140
   ttcactccag agcgatgaaa acgtttcagt ttgctcatgg aaaacggtgt aacaagggtg 4200
   aacactatcc catatcacca gctcaccgtc tttcattgcc atacg 4245
<210> 33
   <211> 4099
   <212> DNA
   <213> Artificial sequence
<220>
   <223> pRF50
<400> 33
   gatcctctac gccggacgca tcgtggccgg catcaccggc gccacaggtg cggttgctgg      60
   cgcctatatc gccgacatca ccgatgggga agatcgggct cgccacttcg ggctcatgag     120
   cgcttgtttc ggcgtgggta tggtggcagg ccccgtggcc gggggactgt tgggcgccat     180
   ctccttgcat gcaccattcc ttgcggcggc ggtgctcaac ggcctcaacc tactactggg     240
   ctgcttccta atgcaggagt cgcataaggg agagcgtcga ccgatgccct tgagagcctt     300
   caacccagtc agctccttcc ggtgggcgcg gggcatgact atcgtcgccg cacttatgac     360
   tgtcttcttt atcatgcaac tcgtaggaca ggtgccggca gcgctctggg tcattttcgg     420
   cgaggaccgc tttcgctgga gcgcgacgat gatcggcctg tcgcttgcgg tattcggaat     480
   cttgcacgcc ctcgctcaag ccttcgtcac tggtcccgcc accaaacgtt tcggcgagaa     540
   gcaggccatt atcgccggca tggcggccga cgcgctgggc tacgtcttgc tggcgttcgc     600
   gacgcgaggc tggatggcct tccccattat gattcttctc gcttccggcg gcatcgggat     660
   gcccgcgttg caggccatgc tgtccaggca ggtagatgac gaccatcagg gacagcttca     720
   aggatcgctc gcggctctta ccagcctaac ttcgatcact ggaccgctga tcgtcacggc     780
   gatttatgcc gcctcggcga gcacatggaa cgggttggca tggattgtag gcgccgccct     840
   ataccttgtc tgcctccccg cgttgcgtcg cggtgcatgg agccgggcca cctcgacctg     900
   aatggaagcc ggcggcacct cgctaacgga ttcaccactc caagaattgg agccaatcaa     960
   ttcttgcgga gaactgtgaa tgcgcaaacc aacccttggc agaacatatc catcgcgtcc    1020
   gccatctcca gcagccgcac gcggcgcatc tcgggcagcg ttgggtcctg gccacgggtg    1080
   cgcatgatcg tgctcctgtc gttgaggacc cggctaggct ggcggggttg ccttactggt    1140
   tagcagaatg aatcaccgat acgcgagcga acgtgaagcg actgctgctg caaaacgtct    1200
   gcgacctgag caacaacatg aatggtcttc ggtttccgtg tttcgtaaag tctggaaacg    1260
   cggaagtccc ctacgtgctg ctgaagttgc ccgcaacaga gagtggaacc aaccggtgat    1320
   accacgatac tatgactgag agtcaacgcc atgagcggcc tcatttctta ttctgagtta    1380
   caacagtccg caccgctgtc cggtagctcc ttccggtggg cgcggggcat gactatcgtc    1440
   gccgcactta tgactgtctt ctttatcatg caactcgtag gacaggtgcc ggcagcgccc    1500
   aacagtcccc cggccacggg gcctgccacc atacccacgc cgaaacaagc gccctgcacc    1560
   attatgttcc ggatctgcat cgcaggatgc tgctggctac cctgtggaac acctacatct    1620
   gtattaacga agcgctaacc gtttttatca ggctctggga ggcagaataa atgatcatat    1680
   cgtcaattat tacctccacg gggagagcct gagcaaactg gcctcaggca tttgagaagc    1740
   acacggtcac actgcttccg gtagtcaata aaccggtaaa ccagcaatag acataagcgg    1800
   ctatttaacg accctgccct gaaccgacga ccgggtcgaa tttgctttcg aatttctgcc    1860
   attcatccgc ttattatcac ttattcaggc gtagcaccag gcgtttaagg gcaccaataa    1920
   ctgccttaaa aaaattacgc cccgccctgc cactcatcgc agtactgttg taattcatta    1980
   agcattctgc cgacatggaa gccatcacag acggcatgat gaacctgaat cgccagcggc    2040
   atcagcacct tgtcgccttg cgtataatat ttgcccatgg tgaaaacggg ggcgaagaag    2100
   ttgtccatat tggccacgtt taaatcaaaa ctggtgaaac tcacccaggg attggctgag    2160
   acgaaaaaca tattctcaat aaacccttta gggaaatagg ccaggttttc accgtaacac    2220
   gccacatctt gcgaatatat gtgtagaaac tgccggaaat cgtcgtggta ttcactccag    2280
   agcgatgaaa acgtttcagt ttgctcatgg aaaacggtgt aacaagggtg aacactatcc    2340
   catatcacca gctcaccgtc tttcattgcc atacggaatt ccggatgagc attcatcagg    2400
   cgggcaagaa tgtgaataaa ggccggataa aacttgtgct tatttttctt tacggtcttt    2460
   aaaaaggccg taatatccag ctgaacggtc tggttatagg tacattgagc aactgactga    2520
   aatgcctcaa aatgttcttt acgatgccat tgggatatat caacggtggt atatccagtg    2580
   atttttttct ccattttagc ttccttagct cctgaaaatc tcgataactc aaaaaatacg    2640
   cccggtagtg atcttatttc attatggtga aagttggaac ctcttacgtg ccgatcaacg    2700
   tctcattttc gccaaaagtt ggcccagggc ttcccggtat caacagggac accaggattt    2760
   atttattctg cgaagtgatc ttccgtcaca ggtatttatt cggcgcaaag tgcgtcgggt    2820
   gatgctgcca acttactgat ttagtgtatg atggtgtttt tgaggtgctc cagtggcttc    2880
   tgtttctatc agctgtccct cctgttcagc tactgacggg gtggtgcgta acggcaaaag    2940
   caccgccgga catcagcgct agcggagtgt atactggctt actatgttgg cactgatgag    3000
   ggtgtcagtg aagtgcttca tgtggcagga gaaaaaaggc tgcaccggtg cgtcagcaga    3060
   atatgtgata caggatatat tccgcttcct cgctcactga ctcgctacgc tcggtcgttc    3120
   gactgcggcg agcggaaatg gcttacgaac ggggcggaga tttcctggaa gatgccagga    3180
   agatacttaa cagggaagtg agagggccgc ggcaaagccg tttttccata ggctccgccc    3240
   ccctgacaag catcacgaaa tctgacgctc aaatcagtgg tggcgaaacc cgacaggact 3300
   ataaagatac caggcgtttc cccctggcgg ctccctcgtg cgctctcctg ttcctgcctt 3360
   tcggtttacc ggtgtcattc cgctgttatg gccgcgtttg tctcattcca cgcctgacac 3420
   tcagttccgg gtaggcagtt cgctccaagc tggactgtat gcacgaaccc cccgttcagt 3480
   ccgaccgctg cgccttatcc ggtaactatc gtcttgagtc caacccggaa agacatgcaa 3540
   aagcaccact ggcagcagcc actggtaatt gatttagagg agttagtctt gaagtcatgc 3600
   gccggttaag gctaaactga aaggacaagt tttggtgact gcgctcctcc aagccagtta 3660
   cctcggttca aagagttggt agctcagaga accttcgaaa aaccgccctg caaggcggtt 3720
   ttttcgtttt cagagcaaga gattacgcgc agaccaaaac gatctcaaga agatcatctt 3780
   attaatcaga taaaatattt ctagatttca gtgcaattta tctcttcaaa tgtagcacct 3840
   gaagtcagcc ccatacgata taagttgtaa ttctcatgtt tgacagctta tcatcgataa 3900
   gcttggttat ctctggcggt gttgacataa ataccactgg cggtgatact gagcacatca 3960
   gcggcaatgt gccgcagttt tagagctaga aatagcaagt taaaataagg ctagtccgtt 4020
   atcaacttga aaaagtggca ccgagtcggt ggtgcgttaa taacaggcct gctggtaatc 4080
   gcaggccttt ttatttttg                                              4099
<210> 34
   <211> 4099
   <212> DNA
   <213> artificial sequence
<220>
   <223> pRF51
<400> 34
   agcttggtta tctctggcgg tgttgacata aataccactg gcggtgatac tgagcacatg      60
   accggcggcg gatttgggtt ttagagctag aaatagcaag ttaaaataag gctagtccgt     120
   tatcaacttg aaaaagtggc accgagtcgg tggtgcgtta ataacaggcc tgctggtaat     180
   cgcaggcctt tttatttttg gatcctctac gccggacgca tcgtggccgg catcaccggc     240
   gccacaggtg cggttgctgg cgcctatatc gccgacatca ccgatgggga agatcgggct     300
   cgccacttcg ggctcatgag cgcttgtttc ggcgtgggta tggtggcagg ccccgtggcc     360
   gggggactgt tgggcgccat ctccttgcat gcaccattcc ttgcggcggc ggtgctcaac     420
   ggcctcaacc tactactggg ctgcttccta atgcaggagt cgcataaggg agagcgtcga     480
   ccgatgccct tgagagcctt caacccagtc agctccttcc ggtgggcgcg gggcatgact     540
   atcgtcgccg cacttatgac tgtcttcttt atcatgcaac tcgtaggaca ggtgccggca     600
   gcgctctggg tcattttcgg cgaggaccgc tttcgctgga gcgcgacgat gatcggcctg     660
   tcgcttgcgg tattcggaat cttgcacgcc ctcgctcaag ccttcgtcac tggtcccgcc     720
   accaaacgtt tcggcgagaa gcaggccatt atcgccggca tggcggccga cgcgctgggc     780
   tacgtcttgc tggcgttcgc gacgcgaggc tggatggcct tccccattat gattcttctc     840
   gcttccggcg gcatcgggat gcccgcgttg caggccatgc tgtccaggca ggtagatgac     900
   gaccatcagg gacagcttca aggatcgctc gcggctctta ccagcctaac ttcgatcact     960
   ggaccgctga tcgtcacggc gatttatgcc gcctcggcga gcacatggaa cgggttggca    1020
   tggattgtag gcgccgccct ataccttgtc tgcctccccg cgttgcgtcg cggtgcatgg    1080
   agccgggcca cctcgacctg aatggaagcc ggcggcacct cgctaacgga ttcaccactc    1140
   caagaattgg agccaatcaa ttcttgcgga gaactgtgaa tgcgcaaacc aacccttggc    1200
   agaacatatc catcgcgtcc gccatctcca gcagccgcac gcggcgcatc tcgggcagcg    1260
   ttgggtcctg gccacgggtg cgcatgatcg tgctcctgtc gttgaggacc cggctaggct    1320
   ggcggggttg ccttactggt tagcagaatg aatcaccgat acgcgagcga acgtgaagcg    1380
   actgctgctg caaaacgtct gcgacctgag caacaacatg aatggtcttc ggtttccgtg    1440
   tttcgtaaag tctggaaacg cggaagtccc ctacgtgctg ctgaagttgc ccgcaacaga    1500
   gagtggaacc aaccggtgat accacgatac tatgactgag agtcaacgcc atgagcggcc    1560
   tcatttctta ttctgagtta caacagtccg caccgctgtc cggtagctcc ttccggtggg    1620
   cgcggggcat gactatcgtc gccgcactta tgactgtctt ctttatcatg caactcgtag    1680
   gacaggtgcc ggcagcgccc aacagtcccc cggccacggg gcctgccacc atacccacgc    1740
   cgaaacaagc gccctgcacc attatgttcc ggatctgcat cgcaggatgc tgctggctac    1800
   cctgtggaac acctacatct gtattaacga agcgctaacc gtttttatca ggctctggga    1860
   ggcagaataa atgatcatat cgtcaattat tacctccacg gggagagcct gagcaaactg    1920
   gcctcaggca tttgagaagc acacggtcac actgcttccg gtagtcaata aaccggtaaa    1980
   ccagcaatag acataagcgg ctatttaacg accctgccct gaaccgacga ccgggtcgaa    2040
   tttgctttcg aatttctgcc attcatccgc ttattatcac ttattcaggc gtagcaccag    2100
   gcgtttaagg gcaccaataa ctgccttaaa aaaattacgc cccgccctgc cactcatcgc    2160
   agtactgttg taattcatta agcattctgc cgacatggaa gccatcacag acggcatgat    2220
   gaacctgaat cgccagcggc atcagcacct tgtcgccttg cgtataatat ttgcccatgg    2280
   tgaaaacggg ggcgaagaag ttgtccatat tggccacgtt taaatcaaaa ctggtgaaac    2340
   tcacccaggg attggctgag acgaaaaaca tattctcaat aaacccttta gggaaatagg    2400
   ccaggttttc accgtaacac gccacatctt gcgaatatat gtgtagaaac tgccggaaat    2460
   cgtcgtggta ttcactccag agcgatgaaa acgtttcagt ttgctcatgg aaaacggtgt    2520
   aacaagggtg aacactatcc catatcacca gctcaccgtc tttcattgcc atacggaatt    2580
   ccggatgagc attcatcagg cgggcaagaa tgtgaataaa ggccggataa aacttgtgct    2640
   tatttttctt tacggtcttt aaaaaggccg taatatccag ctgaacggtc tggttatagg    2700
   tacattgagc aactgactga aatgcctcaa aatgttcttt acgatgccat tgggatatat    2760
   caacggtggt atatccagtg atttttttct ccattttagc ttccttagct cctgaaaatc    2820
   tcgataactc aaaaaatacg cccggtagtg atcttatttc attatggtga aagttggaac    2880
   ctcttacgtg ccgatcaacg tctcattttc gccaaaagtt ggcccagggc ttcccggtat    2940
   caacagggac accaggattt atttattctg cgaagtgatc ttccgtcaca ggtatttatt    3000
   cggcgcaaag tgcgtcgggt gatgctgcca acttactgat ttagtgtatg atggtgtttt    3060
   tgaggtgctc cagtggcttc tgtttctatc agctgtccct cctgttcagc tactgacggg    3120
   gtggtgcgta acggcaaaag caccgccgga catcagcgct agcggagtgt atactggctt    3180
   actatgttgg cactgatgag ggtgtcagtg aagtgcttca tgtggcagga gaaaaaaggc    3240
   tgcaccggtg cgtcagcaga atatgtgata caggatatat tccgcttcct cgctcactga    3300
   ctcgctacgc tcggtcgttc gactgcggcg agcggaaatg gcttacgaac ggggcggaga    3360
   tttcctggaa gatgccagga agatacttaa cagggaagtg agagggccgc ggcaaagccg    3420
   tttttccata ggctccgccc ccctgacaag catcacgaaa tctgacgctc aaatcagtgg    3480
   tggcgaaacc cgacaggact ataaagatac caggcgtttc cccctggcgg ctccctcgtg    3540
   cgctctcctg ttcctgcctt tcggtttacc ggtgtcattc cgctgttatg gccgcgtttg    3600
   tctcattcca cgcctgacac tcagttccgg gtaggcagtt cgctccaagc tggactgtat    3660
   gcacgaaccc cccgttcagt ccgaccgctg cgccttatcc ggtaactatc gtcttgagtc    3720
   caacccggaa agacatgcaa aagcaccact ggcagcagcc actggtaatt gatttagagg    3780
   agttagtctt gaagtcatgc gccggttaag gctaaactga aaggacaagt tttggtgact    3840
   gcgctcctcc aagccagtta cctcggttca aagagttggt agctcagaga accttcgaaa    3900
   aaccgccctg caaggcggtt ttttcgtttt cagagcaaga gattacgcgc agaccaaaac    3960
   gatctcaaga agatcatctt attaatcaga taaaatattt ctagatttca gtgcaattta    4020
   tctcttcaaa tgtagcacct gaagtcagcc ccatacgata taagttgtaa ttctcatgtt    4080
   tgacagctta tcatcgata                                                 4099
<210> 35
   <211> 4099
   <212> DNA
   <213> artificial sequence
<220>
   <223> pRF53
<400> 35
   gatcctctac gccggacgca tcgtggccgg catcaccggc gccacaggtg cggttgctgg      60
   cgcctatatc gccgacatca ccgatgggga agatcgggct cgccacttcg ggctcatgag     120
   cgcttgtttc ggcgtgggta tggtggcagg ccccgtggcc gggggactgt tgggcgccat     180
   ctccttgcat gcaccattcc ttgcggcggc ggtgctcaac ggcctcaacc tactactggg     240
   ctgcttccta atgcaggagt cgcataaggg agagcgtcga ccgatgccct tgagagcctt     300
   caacccagtc agctccttcc ggtgggcgcg gggcatgact atcgtcgccg cacttatgac     360
   tgtcttcttt atcatgcaac tcgtaggaca ggtgccggca gcgctctggg tcattttcgg     420
   cgaggaccgc tttcgctgga gcgcgacgat gatcggcctg tcgcttgcgg tattcggaat     480
   cttgcacgcc ctcgctcaag ccttcgtcac tggtcccgcc accaaacgtt tcggcgagaa     540
   gcaggccatt atcgccggca tggcggccga cgcgctgggc tacgtcttgc tggcgttcgc     600
   gacgcgaggc tggatggcct tccccattat gattcttctc gcttccggcg gcatcgggat     660
   gcccgcgttg caggccatgc tgtccaggca ggtagatgac gaccatcagg gacagcttca     720
   aggatcgctc gcggctctta ccagcctaac ttcgatcact ggaccgctga tcgtcacggc     780
   gatttatgcc gcctcggcga gcacatggaa cgggttggca tggattgtag gcgccgccct     840
   ataccttgtc tgcctccccg cgttgcgtcg cggtgcatgg agccgggcca cctcgacctg     900
   aatggaagcc ggcggcacct cgctaacgga ttcaccactc caagaattgg agccaatcaa     960
   ttcttgcgga gaactgtgaa tgcgcaaacc aacccttggc agaacatatc catcgcgtcc    1020
   gccatctcca gcagccgcac gcggcgcatc tcgggcagcg ttgggtcctg gccacgggtg    1080
   cgcatgatcg tgctcctgtc gttgaggacc cggctaggct ggcggggttg ccttactggt    1140
   tagcagaatg aatcaccgat acgcgagcga acgtgaagcg actgctgctg caaaacgtct    1200
   gcgacctgag caacaacatg aatggtcttc ggtttccgtg tttcgtaaag tctggaaacg    1260
   cggaagtccc ctacgtgctg ctgaagttgc ccgcaacaga gagtggaacc aaccggtgat    1320
   accacgatac tatgactgag agtcaacgcc atgagcggcc tcatttctta ttctgagtta    1380
   caacagtccg caccgctgtc cggtagctcc ttccggtggg cgcggggcat gactatcgtc    1440
   gccgcactta tgactgtctt ctttatcatg caactcgtag gacaggtgcc ggcagcgccc    1500
   aacagtcccc cggccacggg gcctgccacc atacccacgc cgaaacaagc gccctgcacc    1560
   attatgttcc ggatctgcat cgcaggatgc tgctggctac cctgtggaac acctacatct    1620
   gtattaacga agcgctaacc gtttttatca ggctctggga ggcagaataa atgatcatat    1680
   cgtcaattat tacctccacg gggagagcct gagcaaactg gcctcaggca tttgagaagc    1740
   acacggtcac actgcttccg gtagtcaata aaccggtaaa ccagcaatag acataagcgg    1800
   ctatttaacg accctgccct gaaccgacga ccgggtcgaa tttgctttcg aatttctgcc    1860
   attcatccgc ttattatcac ttattcaggc gtagcaccag gcgtttaagg gcaccaataa    1920
   ctgccttaaa aaaattacgc cccgccctgc cactcatcgc agtactgttg taattcatta    1980
   agcattctgc cgacatggaa gccatcacag acggcatgat gaacctgaat cgccagcggc    2040
   atcagcacct tgtcgccttg cgtataatat ttgcccatgg tgaaaacggg ggcgaagaag    2100
   ttgtccatat tggccacgtt taaatcaaaa ctggtgaaac tcacccaggg attggctgag    2160
   acgaaaaaca tattctcaat aaacccttta gggaaatagg ccaggttttc accgtaacac    2220
   gccacatctt gcgaatatat gtgtagaaac tgccggaaat cgtcgtggta ttcactccag    2280
   agcgatgaaa acgtttcagt ttgctcatgg aaaacggtgt aacaagggtg aacactatcc    2340
   catatcacca gctcaccgtc tttcattgcc atacggaatt ccggatgagc attcatcagg    2400
   cgggcaagaa tgtgaataaa ggccggataa aacttgtgct tatttttctt tacggtcttt    2460
   aaaaaggccg taatatccag ctgaacggtc tggttatagg tacattgagc aactgactga    2520
   aatgcctcaa aatgttcttt acgatgccat tgggatatat caacggtggt atatccagtg    2580
   atttttttct ccattttagc ttccttagct cctgaaaatc tcgataactc aaaaaatacg    2640
   cccggtagtg atcttatttc attatggtga aagttggaac ctcttacgtg ccgatcaacg    2700
   tctcattttc gccaaaagtt ggcccagggc ttcccggtat caacagggac accaggattt    2760
   atttattctg cgaagtgatc ttccgtcaca ggtatttatt cggcgcaaag tgcgtcgggt    2820
   gatgctgcca acttactgat ttagtgtatg atggtgtttt tgaggtgctc cagtggcttc    2880
   tgtttctatc agctgtccct cctgttcagc tactgacggg gtggtgcgta acggcaaaag    2940
   caccgccgga catcagcgct agcggagtgt atactggctt actatgttgg cactgatgag    3000
   ggtgtcagtg aagtgcttca tgtggcagga gaaaaaaggc tgcaccggtg cgtcagcaga    3060
   atatgtgata caggatatat tccgcttcct cgctcactga ctcgctacgc tcggtcgttc    3120
   gactgcggcg agcggaaatg gcttacgaac ggggcggaga tttcctggaa gatgccagga    3180
   agatacttaa cagggaagtg agagggccgc ggcaaagccg tttttccata ggctccgccc    3240
   ccctgacaag catcacgaaa tctgacgctc aaatcagtgg tggcgaaacc cgacaggact    3300
   ataaagatac caggcgtttc cccctggcgg ctccctcgtg cgctctcctg ttcctgcctt    3360
   tcggtttacc ggtgtcattc cgctgttatg gccgcgtttg tctcattcca cgcctgacac    3420
   tcagttccgg gtaggcagtt cgctccaagc tggactgtat gcacgaaccc cccgttcagt    3480
   ccgaccgctg cgccttatcc ggtaactatc gtcttgagtc caacccggaa agacatgcaa    3540
   aagcaccact ggcagcagcc actggtaatt gatttagagg agttagtctt gaagtcatgc    3600
   gccggttaag gctaaactga aaggacaagt tttggtgact gcgctcctcc aagccagtta    3660
   cctcggttca aagagttggt agctcagaga accttcgaaa aaccgccctg caaggcggtt    3720
   ttttcgtttt cagagcaaga gattacgcgc agaccaaaac gatctcaaga agatcatctt    3780
   attaatcaga taaaatattt ctagatttca gtgcaattta tctcttcaaa tgtagcacct    3840
   gaagtcagcc ccatacgata taagttgtaa ttctcatgtt tgacagctta tcatcgataa    3900
   gcttggttat ctctggcggt gttgacataa ataccactgg cggtgatact gagcacatag    3960
   ttttcatgtg cgacaagttt tagagctaga aatagcaagt taaaataagg ctagtccgtt    4020
   atcaacttga aaaagtggca ccgagtcggt ggtgcgttaa taacaggcct gctggtaatc    4080
   gcaggccttt ttatttttg                                                 4099
<210> 36
   <211> 4099
   <212> DNA
   <213> Artificial sequence
<220>
   <223> pRF55
<400> 36
   agcttggtta tctctggcgg tgttgacata aataccactg gcggtgatac tgagcacatg      60
   atctttcttg ccgagcggtt ttagagctag aaatagcaag ttaaaataag gctagtccgt     120
   tatcaacttg aaaaagtggc accgagtcgg tggtgcgtta ataacaggcc tgctggtaat     180
   cgcaggcctt tttatttttg gatcctctac gccggacgca tcgtggccgg catcaccggc     240
   gccacaggtg cggttgctgg cgcctatatc gccgacatca ccgatgggga agatcgggct     300
   cgccacttcg ggctcatgag cgcttgtttc ggcgtgggta tggtggcagg ccccgtggcc     360
   gggggactgt tgggcgccat ctccttgcat gcaccattcc ttgcggcggc ggtgctcaac      420
   ggcctcaacc tactactggg ctgcttccta atgcaggagt cgcataaggg agagcgtcga      480
   ccgatgccct tgagagcctt caacccagtc agctccttcc ggtgggcgcg gggcatgact      540
   atcgtcgccg cacttatgac tgtcttcttt atcatgcaac tcgtaggaca ggtgccggca      600
   gcgctctggg tcattttcgg cgaggaccgc tttcgctgga gcgcgacgat gatcggcctg      660
   tcgcttgcgg tattcggaat cttgcacgcc ctcgctcaag ccttcgtcac tggtcccgcc      720
   accaaacgtt tcggcgagaa gcaggccatt atcgccggca tggcggccga cgcgctgggc      780
   tacgtcttgc tggcgttcgc gacgcgaggc tggatggcct tccccattat gattcttctc      840
   gcttccggcg gcatcgggat gcccgcgttg caggccatgc tgtccaggca ggtagatgac      900
   gaccatcagg gacagcttca aggatcgctc gcggctctta ccagcctaac ttcgatcact      960
   ggaccgctga tcgtcacggc gatttatgcc gcctcggcga gcacatggaa cgggttggca     1020
   tggattgtag gcgccgccct ataccttgtc tgcctccccg cgttgcgtcg cggtgcatgg     1080
   agccgggcca cctcgacctg aatggaagcc ggcggcacct cgctaacgga ttcaccactc     1140
   caagaattgg agccaatcaa ttcttgcgga gaactgtgaa tgcgcaaacc aacccttggc     1200
   agaacatatc catcgcgtcc gccatctcca gcagccgcac gcggcgcatc tcgggcagcg     1260
   ttgggtcctg gccacgggtg cgcatgatcg tgctcctgtc gttgaggacc cggctaggct     1320
   ggcggggttg ccttactggt tagcagaatg aatcaccgat acgcgagcga acgtgaagcg     1380
   actgctgctg caaaacgtct gcgacctgag caacaacatg aatggtcttc ggtttccgtg     1440
   tttcgtaaag tctggaaacg cggaagtccc ctacgtgctg ctgaagttgc ccgcaacaga     1500
   gagtggaacc aaccggtgat accacgatac tatgactgag agtcaacgcc atgagcggcc     1560
   tcatttctta ttctgagtta caacagtccg caccgctgtc cggtagctcc ttccggtggg     1620
   cgcggggcat gactatcgtc gccgcactta tgactgtctt ctttatcatg caactcgtag     1680
   gacaggtgcc ggcagcgccc aacagtcccc cggccacggg gcctgccacc atacccacgc     1740
   cgaaacaagc gccctgcacc attatgttcc ggatctgcat cgcaggatgc tgctggctac     1800
   cctgtggaac acctacatct gtattaacga agcgctaacc gtttttatca ggctctggga     1860
   ggcagaataa atgatcatat cgtcaattat tacctccacg gggagagcct gagcaaactg     1920
   gcctcaggca tttgagaagc acacggtcac actgcttccg gtagtcaata aaccggtaaa     1980
   ccagcaatag acataagcgg ctatttaacg accctgccct gaaccgacga ccgggtcgaa     2040
   tttgctttcg aatttctgcc attcatccgc ttattatcac ttattcaggc gtagcaccag     2100
   gcgtttaagg gcaccaataa ctgccttaaa aaaattacgc cccgccctgc cactcatcgc     2160
   agtactgttg taattcatta agcattctgc cgacatggaa gccatcacag acggcatgat     2220
   gaacctgaat cgccagcggc atcagcacct tgtcgccttg cgtataatat ttgcccatgg     2280
   tgaaaacggg ggcgaagaag ttgtccatat tggccacgtt taaatcaaaa ctggtgaaac     2340
   tcacccaggg attggctgag acgaaaaaca tattctcaat aaacccttta gggaaatagg     2400
   ccaggttttc accgtaacac gccacatctt gcgaatatat gtgtagaaac tgccggaaat     2460
   cgtcgtggta ttcactccag agcgatgaaa acgtttcagt ttgctcatgg aaaacggtgt     2520
   aacaagggtg aacactatcc catatcacca gctcaccgtc tttcattgcc atacggaatt     2580
   ccggatgagc attcatcagg cgggcaagaa tgtgaataaa ggccggataa aacttgtgct     2640
   tatttttctt tacggtcttt aaaaaggccg taatatccag ctgaacggtc tggttatagg     2700
   tacattgagc aactgactga aatgcctcaa aatgttcttt acgatgccat tgggatatat     2760
   caacggtggt atatccagtg atttttttct ccattttagc ttccttagct cctgaaaatc     2820
   tcgataactc aaaaaatacg cccggtagtg atcttatttc attatggtga aagttggaac     2880
   ctcttacgtg ccgatcaacg tctcattttc gccaaaagtt ggcccagggc ttcccggtat     2940
   caacagggac accaggattt atttattctg cgaagtgatc ttccgtcaca ggtatttatt     3000
   cggcgcaaag tgcgtcgggt gatgctgcca acttactgat ttagtgtatg atggtgtttt     3060
   tgaggtgctc cagtggcttc tgtttctatc agctgtccct cctgttcagc tactgacggg     3120
   gtggtgcgta acggcaaaag caccgccgga catcagcgct agcggagtgt atactggctt     3180
   actatgttgg cactgatgag ggtgtcagtg aagtgcttca tgtggcagga gaaaaaaggc     3240
   tgcaccggtg cgtcagcaga atatgtgata caggatatat tccgcttcct cgctcactga     3300
   ctcgctacgc tcggtcgttc gactgcggcg agcggaaatg gcttacgaac ggggcggaga     3360
   tttcctggaa gatgccagga agatacttaa cagggaagtg agagggccgc ggcaaagccg     3420
   tttttccata ggctccgccc ccctgacaag catcacgaaa tctgacgctc aaatcagtgg     3480
   tggcgaaacc cgacaggact ataaagatac caggcgtttc cccctggcgg ctccctcgtg     3540
   cgctctcctg ttcctgcctt tcggtttacc ggtgtcattc cgctgttatg gccgcgtttg     3600
   tctcattcca cgcctgacac tcagttccgg gtaggcagtt cgctccaagc tggactgtat     3660
   gcacgaaccc cccgttcagt ccgaccgctg cgccttatcc ggtaactatc gtcttgagtc     3720
   caacccggaa agacatgcaa aagcaccact ggcagcagcc actggtaatt gatttagagg     3780
   agttagtctt gaagtcatgc gccggttaag gctaaactga aaggacaagt tttggtgact     3840
   gcgctcctcc aagccagtta cctcggttca aagagttggt agctcagaga accttcgaaa     3900
   aaccgccctg caaggcggtt ttttcgtttt cagagcaaga gattacgcgc agaccaaaac     3960
   gatctcaaga agatcatctt attaatcaga taaaatattt ctagatttca gtgcaattta     4020
   tctcttcaaa tgtagcacct gaagtcagcc ccatacgata taagttgtaa ttctcatgtt     4080
   tgacagctta tcatcgata                                                  4099
<210> 37
   <211> 454
   <212> DNA
   <213> Escherichia coli
<220>
   <221> misc_feature
   <222> (1)..(454)
   <223> 454bp 5' galK
<400> 37
   ggattatgtt cagcgcgagc tggcagacgg tagccgtacc gttgtcgaaa ccgaacactg      60
   gttagccgtc gtgccttact gggctgcctg gccgttcgaa acgctactgc tgcccaaagc     120
   ccacgtttta cggatcaccg atttgaccga cgcccagcgc agcgatctgg cgctggcgtt     180
   gaaaaagctg accagtcgtt atgacaacct cttccagtgc tccttcccct actctatggg     240
   ctggcacggc gcgccattta atggcgaaga gaatcaacac tggcagctgc acgcgcactt     300
   ttatccgcct ctgctgcgct ccgccaccgt acgtaaattt atggttggtt atgaaatgct     360
   ggcagagacc cagcgagacc tgaccgcaga acaggcagca gagcgtttgc gcgcagtcag     420
   cgatatccat tttcgcgaat ccggagtgta agaa                                 454
<210> 38
   <211> 29
   <212> DNA
   <213> Artificial sequence
<220>
   <223> 5' forward primer
<400> 38
   gggaagcttg gattatgttc agcgcgagc 29
<210> 39
   <211> 40
   <212> DNA
   <213> Artificial sequence
<220>
   <223> 5' reverse primer
<400> 39
   tgccagtgcg ggagtttcgt ttcttacact ccggattcgc 40
<210> 40
   <211> 483
   <212> DNA
   <213> Artificial sequence
<220>
   <223> upstream overlap extension product
<400> 40
   gggaagcttg gattatgttc agcgcgagct ggcagacggt agccgtaccg ttgtcgaaac 60
   cgaacactgg ttagccgtcg tgccttactg ggctgcctgg ccgttcgaaa cgctactgct 120
   gcccaaagcc cacgttttac ggatcaccga tttgaccgac gcccagcgca gcgatctggc 180
   gctggcgttg aaaaagctga ccagtcgtta tgacaacctc ttccagtgct ccttccccta 240
   ctctatgggc tggcacggcg cgccatttaa tggcgaagag aatcaacact ggcagctgca 300
   cgcgcacttt tatccgcctc tgctgcgctc cgccaccgta cgtaaattta tggttggtta 360
   tgaaatgctg gcagagaccc agcgagacct gaccgcagaa caggcagcag agcgtttgcg 420
   cgcagtcagc gatatccatt ttcgcgaatc cggagtgtaa gaaacgaaac tcccgcactg 480
   gca 483
<210> 41
   <211> 376
   <212> DNA
   <213> Escherichia coli
<220>
   <221> misc_feature
   <222> (1)..(376)
   <223> 376bp 3' galK
<400> 41
   acgaaactcc cgcactggca cccgatggtc agccgtaccg actgttaact ttgcgtaaca 60
   acgcagggat ggtagtcacg ctgatggact ggggtgcgac tttactttcc gcccgtattc 120
   cgctttccga tggcagcgtc cgcgaggcgc tgctcggctg tgccagcccg gaatgctatc 180
   aggatcaggc cgcgtttctg ggggcctcta ttggtcgtta tgccaaccgt atcgccaata 240
   gccgttatac ctttgacggt gaaaccgtga cgctttcgcc aagtcagggc gttaaccagc 300
   tgcacggcgg gccggaaggg ttcgacaaac gtcgctggca gattgtgaac cagaacgatc 360
   gtcaggtgct gtttgc 376
<210> 42
   <211> 40
   <212> DNA
   <213> Artificial sequence
<220>
   <223> 3' forward primer
<400> 42
   gcgaatccgg agtgtaagaa acgaaactcc cgcactggca 40
<210> 43
   <211> 30
   <212> DNA
   <213> Artificial sequence
<220>
   <223> 3' reverse primer
<400> 43
   gggaagcttg caaacagcac ctgacgatcg 30
<210> 44
   <211> 405
   <212> DNA
   <213> Artificial Sequence
<220>
   <223> downstream overlap extension product
<400> 44
   gcgaatccgg agtgtaagaa acgaaactcc cgcactggca cccgatggtc agccgtaccg      60
   actgttaact ttgcgtaaca acgcagggat ggtagtcacg ctgatggact ggggtgcgac     120
   tttactttcc gcccgtattc cgctttccga tggcagcgtc cgcgaggcgc tgctcggctg     180
   tgccagcccg gaatgctatc aggatcaggc cgcgtttctg ggggcctcta ttggtcgtta      240
   tgccaaccgt atcgccaata gccgttatac ctttgacggt gaaaccgtga cgctttcgcc     300
   aagtcagggc gttaaccagc tgcacggcgg gccggaaggg ttcgacaaac gtcgctggca     360
   gattgtgaac cagaacgatc gtcaggtgct gtttgcaagc ttccc                     405
<210> 45
   <211> 848
   <212> DNA
   <213> Artificial sequence
<220>
   <223> galK deletion polynucleotide modification template
<400> 45
   gggaagcttg gattatgttc agcgcgagct ggcagacggt agccgtaccg ttgtcgaaac      60
   cgaacactgg ttagccgtcg tgccttactg ggctgcctgg ccgttcgaaa cgctactgct     120
   gcccaaagcc cacgttttac ggatcaccga tttgaccgac gcccagcgca gcgatctggc     180
   gctggcgttg aaaaagctga ccagtcgtta tgacaacctc ttccagtgct ccttccccta     240
   ctctatgggc tggcacggcg cgccatttaa tggcgaagag aatcaacact ggcagctgca     300
   cgcgcacttt tatccgcctc tgctgcgctc cgccaccgta cgtaaattta tggttggtta     360
   tgaaatgctg gcagagaccc agcgagacct gaccgcagaa caggcagcag agcgtttgcg     420
   cgcagtcagc gatatccatt ttcgcgaatc cggagtgtaa gaaacgaaac tcccgcactg     480
   gcacccgatg gtcagccgta ccgactgtta actttgcgta acaacgcagg gatggtagtc     540
   acgctgatgg actggggtgc gactttactt tccgcccgta ttccgctttc cgatggcagc     600
   gtccgcgagg cgctgctcgg ctgtgccagc ccggaatgct atcaggatca ggccgcgttt     660
   ctgggggcct ctattggtcg ttatgccaac cgtatcgcca atagccgtta tacctttgac    720
   ggtgaaaccg tgacgctttc gccaagtcag ggcgttaacc agctgcacgg cgggccggaa     780
   gggttcgaca aacgtcgctg gcagattgtg aaccagaacg atcgtcaggt gctgtttgca     840
   agcttccc                                                              848
<210> 46
   <211> 2804
   <212> DNA
   <213> Artificial sequence
<220>
   <223> pKD3
<400> 46
   agattgcagc attacacgtc ttgagcgatt gtgtaggctg gagctgcttc gaagttccta      60
   tactttctag agaataggaa cttcggaata ggaacttcat ttaaatggcg cgccttacgc     120
   cccgccctgc cactcatcgc agtactgttg tattcattaa gcatctgccg acatggaagc     180
   catcacaaac ggcatgatga acctgaatcg ccagcggcat cagcaccttg tcgccttgcg     240
   tataatattt gcccatggtg aaaacggggg cgaagaagtt gtccatattg gccacgttta     300
   aatcaaaact ggtgaaactc acccagggat tggctgagac gaaaaacata ttctcaataa     360
   accctttagg gaaataggcc aggttttcac cgtaacacgc cacatcttgc gaatatatgt     420
   gtagaaactg ccggaaatcg tcgtggtatt cactccagag cgatgaaaac gtttcagttt     480
   gctcatggaa aacggtgtaa caagggtgaa cactatccca tatcaccagc tcaccgtctt     540
   tcattgccat acgtaattcc ggatgagcat tcatcaggcg ggcaagaatg tgaataaagg     600
   ccggataaaa cttgtgctta tttttcttta cggtctttaa aaaggccgta atatccagct     660
   gaacggtctg gttataggta cattgagcaa ctgactgaaa tgcctcaaaa tgttctttac     720
   gatgccattg ggatatatca acggtggtat atccagtgat ttttttctcc attttagctt     780
   ccttagctcc tgaaaatctc gacaactcaa aaaatacgcc cggtagtgat cttatttcat     840
   tatggtgaaa gttggaacct cttacgtgcc gatcaacgtc tcattttcgc caaaagttgg     900
   cccagggctt cccggtatca acagggacac caggatttat ttattctgcg aagtgatctt     960
   ccgtcacagg taggcgcgcc gaagttccta tactttctag agaataggaa cttcggaata    1020
   ggaactaagg aggatattca tatggaccat ggctaattcc catgtcagcc gttaagtgtt    1080
   cctgtgtcac tgaaaattgc tttgagaggc tctaagggct tctcagtgcg ttacatccct    1140
   ggcttgttgt ccacaaccgt taaaccttaa aagctttaaa agccttatat attctttttt    1200
   ttcttataaa acttaaaacc ttagaggcta tttaagttgc tgatttatat taattttatt    1260
   gttcaaacat gagagcttag tacgtgaaac atgagagctt agtacgttag ccatgagagc    1320
   ttagtacgtt agccatgagg gtttagttcg ttaaacatga gagcttagta cgttaaacat    1380
   gagagcttag tacgtgaaac atgagagctt agtacgtact atcaacaggt tgaactgcgg    1440
   atcttgcggc cgcaaaaatt aaaaatgaag ttttaaatca atctaaagta tatatgagta    1500
   aacttggtct gacagttacc aatgcttaat cagtgaggca cctatctcag cgatctgtct    1560
   atttcgttca tccatagttg cctgactccc cgtcgtgtag ataactacga tacgggaggg    1620
   cttaccatct ggccccagtg ctgcaatgat accgcgagac ccacgctcac cggctccaga    1680
   tttatcagca ataaaccagc cagccggaag ggccgagcgc agaagtggtc ctgcaacttt    1740
   atccgcctcc atccagtcta ttaattgttg ccgggaagct agagtaagta gttcgccagt    1800
   taatagtttg cgcaacgttg ttgccattgc tacaggcatc gtggtgtcac gctcgtcgtt    1860
   tggtatggct tcattcagct ccggttccca acgatcaagg cgagttacat gatcccccat    1920
   gttgtgcaaa aaagcggtta gctccttcgg tcctccgatc gttgtcagaa gtaagttggc    1980
   cgcagtgtta tcactcatgg ttatggcagc actgcataat tctcttactg tcatgccatc    2040
   cgtaagatgc ttttctgtga ctggtgagta ctcaaccaag tcattctgag aatagtgtat    2100
   gcggcgaccg agttgctctt gcccggcgtc aatacgggat aataccgcgc cacatagcag    2160
   aactttaaaa gtgctcatca ttggaaaacg ttcttcgggg cgaaaactct caaggatctt    2220
   accgctgttg agatccagtt cgatgtaacc cactcgtgca cccaactgat cttcagcatc    2280
   ttttactttc accagcgttt ctgggtgagc aaaaacagga aggcaaaatg ccgcaaaaaa    2340
   gggaataagg gcgacacgga aatgttgaat actcatactc ttcctttttc aatattattg    2400
   aagcatttat cagggttatt gtctcatgag cggatacata tttgaatgta tttagaaaaa    2460
   taaacaaata ggggttccgc gcacatttcc ccgaaaagtg ccacctgcat cgatggcccc    2520
   ccgatggtag tgtggggtct ccccatgcga gagtagggaa ctgccaggca tcaaataaaa    2580
   cgaaaggctc agtcgaaaga ctgggccttt cgttttatct gttgtttgtc ggtgaacgct    2640
   ctcctgagta ggacaaatcc gccgggagcg gatttgaacg ttgcgaagca acggcccgga    2700
   gggtggcggg caggacgccc gccataaact gccaggcatc aaattaagca gaaggccatc    2760
   ctgacggatg gcctttttgc gtggccagtg ccaagcttgc atgc                      2804
<210> 47
   <211> 2458
   <212> DNA
   <213> Artificial sequence
<220>
   <223> pRF113
<400> 47
   agctttaaaa gccttatata ttcttttttt tcttataaaa cttaaaacct tagaggctat     60
   ttaagttgct gatttatatt aattttattg ttcaaacatg agagcttagt acgtgaaaca    120
   tgagagctta gtacgttagc catgagagct tagtacgtta gccatgaggg tttagttcgt    180
   taaacatgag agcttagtac gttaaacatg agagcttagt acgtgaaaca tgagagctta    240
   gtacgtacta tcaacaggtt gaactgcgga tcttgcggcc gcaaaaatta aaaatgaagt    300
   tttaaatcaa tctaaagtat atatgagtaa acttggtctg acagttacca atgcttaatc    360
   agtgaggcac ctatctcagc gatctgtcta tttcgttcat ccatagttgc ctgactcccc    420
   gtcgtgtaga taactacgat acgggagggc ttaccatctg gccccagtgc tgcaatgata    480
   ccgcgagacc cacgctcacc ggctccagat ttatcagcaa taaaccagcc agccggaagg    540
   gccgagcgca gaagtggtcc tgcaacttta tccgcctcca tccagtctat taattgttgc    600
   cgggaagcta gagtaagtag ttcgccagtt aatagtttgc gcaacgttgt tgccattgct    660
   acaggcatcg tggtgtcacg ctcgtcgttt ggtatggctt cattcagctc cggttcccaa    720
   cgatcaaggc gagttacatg atcccccatg ttgtgcaaaa aagcggttag ctccttcggt    780
   cctccgatcg ttgtcagaag taagttggcc gcagtgttat cactcatggt tatggcagca    840
   ctgcataatt ctcttactgt catgccatcc gtaagatgct tttctgtgac tggtgagtac    900
   tcaaccaagt cattctgaga atagtgtatg cggcgaccga gttgctcttg cccggcgtca    960
   atacgggata ataccgcgcc acatagcaga actttaaaag tgctcatcat tggaaaacgt   1020
   tcttcggggc gaaaactctc aaggatctta ccgctgttga gatccagttc gatgtaaccc   1080
   actcgtgcac ccaactgatc ttcagcatct tttactttca ccagcgtttc tgggtgagca   1140
   aaaacaggaa ggcaaaatgc cgcaaaaaag ggaataaggg cgacacggaa atgttgaata   1200
   ctcatactct tcctttttca atattattga agcatttatc agggttattg tctcatgagc   1260
   ggatacatat ttgaatgtat ttagaaaaat aaacaaatag gggttccgcg cacatttccc   1320
   cgaaaagtgc cacctgcatc gatggccccc cgatggtagt gtggggtctc cccatgcgag   1380
   agtagggaac tgccaggcat caaataaaac gaaaggctca gtcgaaagac tgggcctttc   1440
   gttttatctg ttgtttgtcg gtgaacgctc tcctgagtag gacaaatccg ccgggagcgg   1500
   atttgaacgt tgcgaagcaa cggcccggag ggtggcgggc aggacgcccg ccataaactg   1560
   ccaggcatca aattaagcag aaggccatcc tgacggatgg cctttttgcg tggccagtgc   1620
   caagcttgga ttatgttcag cgcgagctgg cagacggtag ccgtaccgtt gtcgaaaccg   1680
   aacactggtt agccgtcgtg ccttactggg ctgcctggcc gttcgaaacg ctactgctgc   1740
   ccaaagccca cgttttacgg atcaccgatt tgaccgacgc ccagcgcagc gatctggcgc   1800
   tggcgttgaa aaagctgacc agtcgttatg acaacctctt ccagtgctcc ttcccctact   1860
   ctatgggctg gcacggcgcg ccatttaatg gcgaagagaa tcaacactgg cagctgcacg   1920
   cgcactttta tccgcctctg ctgcgctccg ccaccgtacg taaatttatg gttggttatg   1980
   aaatgctggc agagacccag cgagacctga ccgcagaaca ggcagcagag cgtttgcgcg   2040
   cagtcagcga tatccatttt cgcgaatccg gagtgtaaga aacgaaactc ccgcactggc   2100
   acccgatggt cagccgtacc gactgttaac tttgcgtaac aacgcaggga tggtagtcac   2160
   gctgatggac tggggtgcga ctttactttc cgcccgtatt ccgctttccg atggcagcgt   2220
   ccgcgaggcg ctgctcggct gtgccagccc ggaatgctat caggatcagg ccgcgtttct   2280
   gggggcctct attggtcgtt atgccaaccg tatcgccaat agccgttata cctttgacgg   2340
   tgaaaccgtg acgctttcgc caagtcaggg cgttaaccag ctgcacggcg ggccggaagg   2400
   gttcgacaaa cgtcgctggc agattgtgaa ccagaacgat cgtcaggtgc tgtttgca      2458
<210> 48
   <211> 1717
   <212> DNA
   <213> Escherichia coli
<220>
   <221> misc_feature
   <222> (1)..(1717)
   <223> galK locus
<400> 48
   ggcgaagaga atcaacactg gcagctgcac gcgcactttt atccgcctct gctgcgctcc      60
   gccaccgtac gtaaatttat ggttggttat gaaatgctgg cagagaccca gcgagacctg     120
   accgcagaac aggcagcaga gcgtttgcgc gcagtcagcg atatccattt tcgcgaatcc     180
   ggagtgtaag aaatgagtct gaaagaaaaa acacaatctc tgtttgccaa cgcatttggc     240
   taccctgcca ctcacaccat tcaggcgcct ggccgcgtga atttgattgg tgaacacacc     300
   gactacaacg acggtttcgt tctgccctgc gcgattgatt atcaaaccgt gatcagttgt     360
   gcaccacgcg atgaccgtaa agttcgcgtg atggcagccg attatgaaaa tcagctcgac     420
   gagttttccc tcgatgcgcc cattgtcgca catgaaaact atcaatgggc taactacgtt     480
   cgtggcgtgg tgaaacatct gcaactgcgt aacaacagct tcggcggcgt ggacatggtg     540
   atcagcggca atgtgccgca gggtgccggg ttaagttctt ccgcttcact ggaagtcgcg     600
   gtcggaaccg tattgcagca gctttatcat ctgccgctgg acggcgcaca aatcgcgctt     660
   aacggtcagg aagcagaaaa ccagtttgta ggctgtaact gcgggatcat ggatcagcta     720
   atttccgcgc tcggcaagaa agatcatgcc ttgctgatcg attgccgctc actggggacc     780
   aaagcagttt ccatgcccaa aggtgtggct gtcgtcatca tcaacagtaa cttcaaacgt     840
   accctggttg gcagcgaata caacacccgt cgtgaacagt gcgaaaccgg tgcgcgtttc     900
   ttccagcagc cagccctgcg tgatgtcacc attgaagagt tcaacgctgt tgcgcatgaa     960
   ctggacccga tcgtggcaaa acgcgtgcgt catatactga ctgaaaacgc ccgcaccgtt    1020
   gaagctgcca gcgcgctgga gcaaggcgac ctgaaacgta tgggcgagtt gatggcggag    1080
   tctcatgcct ctatgcgcga tgatttcgaa atcaccgtgc cgcaaattga cactctggta    1140
   gaaatcgtca aagctgtgat tggcgacaaa ggtggcgtac gcatgaccgg cggcggattt    1200
   ggcggctgta tcgtcgcgct gatcccggaa gagctggtgc ctgccgtaca gcaagctgtc    1260
   gctgaacaat atgaagcaaa aacaggtatt aaagagactt tttacgtttg taaaccatca    1320
   caaggagcag gacagtgctg aacgaaactc ccgcactggc acccgatggt cagccgtacc    1380
   gactgttaac tttgcgtaac aacgcaggga tggtagtcac gctgatggac tggggtgcga    1440
   ctttactttc cgcccgtatt ccgctttccg atggcagcgt ccgcgaggcg ctgctcggct    1500
   gtgccagccc ggaatgctat caggatcagg ccgcgtttct gggggcctct attggtcgtt   1560
   atgccaaccg tatcgccaat agccgttata cctttgacgg tgaaaccgtg acgctttcgc    1620
   caagtcaggg cgttaaccag ctgcacggcg ggccggaagg gttcgacaaa cgtcgctggc    1680
   agattgtgaa ccagaacgat cgtcaggtgc tgtttgc                             1717
<210> 49
   <211> 21
   <212> DNA
   <213> artificial sequence
<220>
   <223> GalK forward
<400> 49
   ggcgaagaga atcaacactg g 21
<210> 50
   <211> 21
   <212> DNA
   <213> Artificial sequence
<220>
   <223> GalK Reverse
<400> 50
   gcaaacagca cctgacgatc g 21
<210> 51
   <211> 1136
   <212> DNA
   <213> Escherichia coli
<220>
   <221> misc_feature
   <222> (1)..(1136)
   <223> galK deletion
<400> 51
   ggcgaagaga atcaacactg gcagctgcac gcgcactttt atccgcctct gctgcgctcc      60
   gccaccgtac gtaaatttat ggttggttat gaaatgctgg cagagaccca gcgagacctg     120
   accgcagaac aggcagcaga gcgtttgcgc gcagtcagcg atatccattt tcgcgaatcc     180
   ggagtgtaag aaacgaaact cccgcactgg cacccgatgg tcagccgtac cgactgttaa     240
   ctttgcgtaa caacgcaggg atggtagtca cgctgatgga ctggggtgcg actttacttt     300
   ccgcccgtat tccgctttcc gatggcagcg tccgcgaggc gctgctcggc tgtgccagcc     360
   cggaatgcta tcaggatcag gccgcgtttc tgggggcctc tattggtcgt tatgccaacc     420
   gtatcgccaa tagccgttat acctttgacg gtgaaaccgt gacgctttcg ccaagtcagg     480
   gcgttaacca gctgcacggc gggccggaag ggttcgacaa acgtcgctgg cagattgtga     540
   accagaacga tcgtcaggtg ctgtttgcgg cgaagagaat caacactggc agctgcacgc     600
   gcacttttat ccgcctctgc tgcgctccgc caccgtacgt aaatttatgg ttggttatga     660
   aatgctggca gagacccagc gagacctgac cgcagaacag gcagcagagc gtttgcgcgc     720
   agtcagcgat atccattttc gcgaatccgg agtgtaagaa acgaaactcc cgcactggca     780
   cccgatggtc agccgtaccg actgttaact ttgcgtaaca acgcagggat ggtagtcacg     840
   ctgatggact ggggtgcgac tttactttcc gcccgtattc cgctttccga tggcagcgtc     900
   cgcgaggcgc tgctcggctg tgccagcccg gaatgctatc aggatcaggc cgcgtttctg     960
   ggggcctcta ttggtcgtta tgccaaccgt atcgccaata gccgttatac ctttgacggt    1020
   gaaaccgtga cgctttcgcc aagtcagggc gttaaccagc tgcacggcgg gccggaaggg    1080
   ttcgacaaac gtcgctggca gattgtgaac cagaacgatc gtcaggtgct gtttgc        1136
<210> 52
   <211> 23
   <212> DNA
   <213> unknown
<220>
   <223> Example of a Cas9 target site:PAM sequence
<220>
   <221> misc_feature
   <222> (1)..(20)
   <223> n = A, C, T, or G
<220>
   <221> misc_feature
   <222> (21)..(21)
   <223> n = A, C, T, or G (indicated as an "X" in Specification)
<400> 52
   nnnnnnnnnn nnnnnnnnnn ngg 23